(19) Europäisches Patentamt
European Patent Office
Office européen des brevets

(11) EP 2 865 677 B1

(12) # EUROPEAN PATENT SPECIFICATION

(45) Date of publication and mention of the grant of the patent:
**23.11.2016 Bulletin 2016/47**

(51) Int Cl.:
***C07D 311/74*** *(2006.01)*

(21) Application number: **13189689.6**

(22) Date of filing: **22.10.2013**

(54) **Synthesis of chromane compounds and their derivatives by a copper-catalyzed conjugate addition reaction**

Synthese von Chromanverbindungen und ihren Derivaten durch kupferkatalysierte Konjugatadditionsreaktion

Synthèse de composés de type chromane et de ses dérivés par réaction d'addition d'un conjugué catalysé au cuivre

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**29.04.2015 Bulletin 2015/18**

(73) Proprietor: **DSM IP Assets B.V.**
**6411 TE Heerlen (NL)**

(72) Inventors:
- **Termath, Andreas Ole**
**50939 Köln (DE)**
- **Schmalz, Hans-Günter**
**50939 Köln (DE)**

(74) Representative: **Dux, Roland**
**DSM Nutritional Products Ltd**
**Patent Department**
**Wurmisweg 576**
**4303 Kaiseraugst (CH)**

(56) References cited:
**EP-A1- 2 522 647**

- **KAWASAKI M ET AL: "Asymmetric synthesis of 2-substituted 4-chromanones using enzyme-catalyzed reactions", JOURNAL OF MOLECULAR CATALYSIS. B, ENZYMATIC, ELSEVIER, AMSTERDAM, NL, vol. 54, no. 3-4, 1 August 2008 (2008-08-01), pages 93-102, XP023177149, ISSN: 1381-1177, DOI: 10.1016/J.MOLCATB.2007.12.022 [retrieved on 2008-01-04]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 865 677 B1

## Description

### Technical Field

The present invention relates to the field of the synthesis of chiral chromane compounds, as well as the derivatives and precursors thereof.

### Background of the invention

Chromane compounds represent an important class of chiral natural products and bioactive compounds.

An important class of chromane compounds are members of the vitamin E group, i.e. all tocol and tocotrienol derivatives exhibiting qualitatively the biological activity of α-tocopherol (Pure & Appl.Chem., Vol. 54, No. 8, pp. 1507-1510, 1982).

On the one hand the typical technical synthesis of vitamin E leads to mixtures of isomers. On the other hand higher bioactivity (biopotency) has been shown to occur in general by tocopherols and tocotrienols having the R-configuration at the chiral centre situated next to the ether atom in the ring of the molecule (indicated by * in the formulas used later on in the present document) (i.e. 2R-configuration), as compared to the corresponding isomers having S-configuration. Particularly active are the isomers of tocopherols having the natural configuration at all chiral centres, for example (R,R,R)-tocopherols, as has been disclosed for example by H. Weiser et al. in J. Nutr. 1996, 126(10), 2539-49. This leads to a strong desire for an efficient process for separating the isomers. Hence, the isomer separation not only of vitamin E, but also of their esters, particularly their acetates, as well as of their precursors is of prime interest.

To overcome separation problems, it has been tried to offer stereoselective syntheses allowing the preferential formation of the desired isomers only. However, these methods are very expensive, complex and/or exotic as compared to the traditional industrial synthesis leading to isomer mixtures.

Therefore, a large interest exists in providing stereospecific or at least highly stereoselective synthesis routes leading to the desired isomer.

The desired chirality at the chiral carbon centre in the 2-position of the chromane ring is particularly difficult is to achieve stereoselectively.

A synthetic pathway for chromanes is via their corresponding chromanones. It is known from Kabbe and Heitzer, Synthesis 1978; (12): 888-889 that α-tocopherol can be synthesized via α-tocotrienol from 4-oxo-α-tocotrienol which is accessible from 2-acetyl-3,5,6-trimethylhydroquinone and farnesylacetone in the presence of pyrrolidine. However, this procedure leads to an all-racemic mixture, meaning in particular a mixture of stereoisomers, in view of the configurations at the 2-position of the chromane and chromanone ring, respectively. Furthermore, Kabbe and Widdig, Angew. Chem. Int. Ed. Engl. 1982, vol 21, 247-256 discloses synthesis and reactions of 4-chromanones.

### Summary of the invention

Therefore, the problem to be solved by the present invention is to offer a method for the synthesis of 4-chromanones or chromanes, i.e. of compounds of formula (I-A) or (I-B), (XXI-A) or (XXI-B), or (V-A) or (V-B), respectively, in a stereoselective manner in view of the 2-position in the chromanone or chromane ring.

Surprisingly, it has been found the process according to claim 1 is able to solve this problem.

The invention solves the problems by offering new pathways yielding chiral 4-chromanones starting from chromenones using a copper (I) catalysed 1,4-addition in the presence of a specific chiral compound.

As this method requires new substances as starting materials, a further object of the invention is to offer suitable starting substances for the above mentioned method as well as a method of preparing said starting materials in high yields and in a very efficient, particularly cost-efficient, way. It has been surprisingly found that this problem can be solved by the substances and methods which are subject of further independent claims.

Further aspects of the invention are subject of further independent claims.

Particularly preferred embodiments are subject of dependent claims.

### Detailed description of the invention

In a first aspect the present invention relates to a method of preparing compound of formula (I-A) or (I-B)

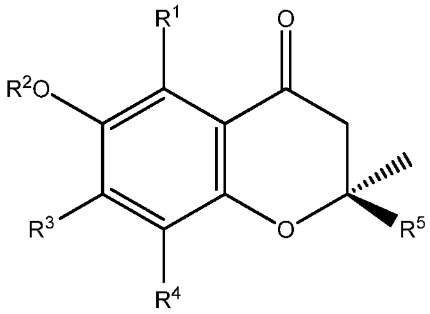

(I-A)

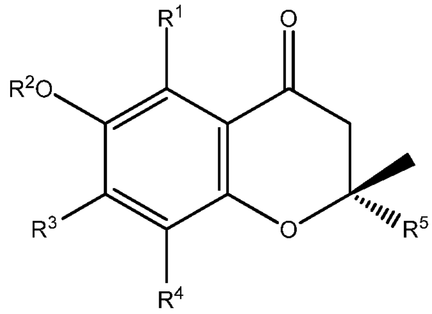

(I-B)

comprising the step of reacting compound of formula (XII)

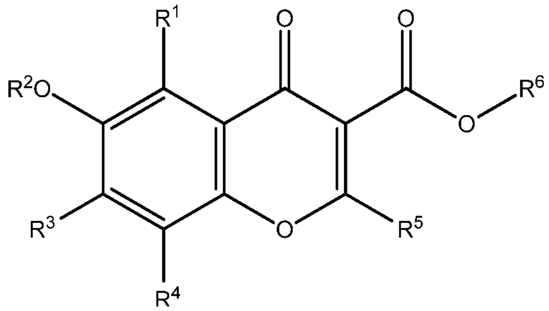

(XII)

with $Al(CH_3)_3$ in the presence of a Cu(I) salt and chiral compound of formula (II-A) or (II-B)

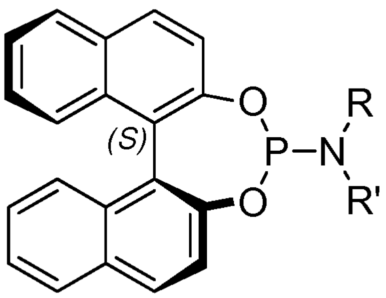

(II-A)

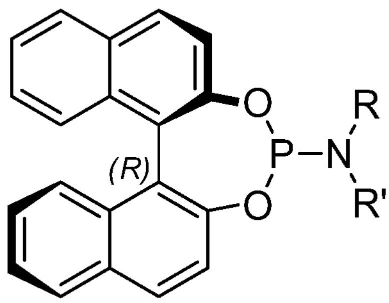

(II-B)

followed by ester hydrolysis and decarboxylation by heating to a temperature of 100-190 °C, particularly 150-190°C;

wherein $R^5$ represents either a linear or branched completely saturated $C_{6-25}$-alkyl group or a linear or branched $C_{6-25}$-alkyl group comprising at least one carbon-carbon double bond;
$R^1$, $R^3$ and $R^4$ are independently from each other hydrogen or methyl groups;
$R^2$ represents hydrogen or a phenol protecting group;
R' and R are independently from each other a hydrocarbyl group with 1 to 12 carbon atoms, which optionally is substituted by $OR^6$, or form together a $C_{4-6}$-alkylene group which optionally comprises a functional group which is selected from the group consisting of

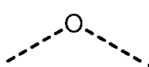 , 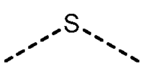 , 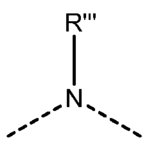 and 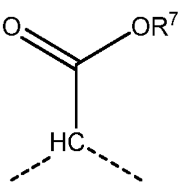

wherein R''' and $R^7$ are independently from each other a $C_{1-4}$-alkyl group; and $R^6$ is a methyl or ethyl group or *tert.*-butyl group;
with the proviso that for the preparation of compound (I-A) the compound (II-A) is used and that for the preparation of compound (I-B) the compound (II-B) is used.

The term "independently from each other" in this document means, in the context of substituents, moieties, or groups, that identically designated substituents, moieties, or groups can occur simultaneously with a different meaning in the same molecule.
A "$C_{x-y}$-alkyl", resp. "$C_{x-y}$-acyl" group, is an alkyl resp. an acyl group comprising x to y carbon atoms, i.e. for example an $C_{1-3}$-alkyl group, is an alkyl group comprising 1 to 3 carbon atoms. The alkyl resp. the acyl group can be linear or branched. For example-$CH(CH_3)$-$CH_2$-$CH_3$ is considered as a $C_4$-alkyl group.
A "$C_{x-y}$-alkylene" group is an alkylene group comprising x to y carbon atoms, i.e., for example $C_2$-$C_6$ alkylene group is an alkyl group comprising 2 to 6 carbon atoms. The alkylene group can be linear or branched. For example the group -$CH(CH_3)$-$CH_2$- is considered as a $C_3$-alkylene group.
The term "hydrogen" means in the present document H and not $H_2$.
In the present document any single dotted line represents the bond by which a substituent is bound to the rest of a molecule.
The chirality of an individual stereogenic centre or an axis of chirality is indicated by the label R or S according to the rules defined by R. S. Cahn, C. K. Ingold and V. Prelog. This R/S-concept and rules for the determination of the absolute configuration in stereochemistry is known to the person skilled in the art.
For simplification, the definition of substituents labeled, e.g. R, R', $R^1$ ,$R^2$, $R^3$, $R^4$, $R^5$ or $R^6$, in one formula also applies to other (different) formulas bearing the same labels , e.g. R, R', $R^1$ ,$R^2$, $R^3$, $R^4$, $R^5$ or $R^6$, in this document.
$R^1$, $R^3$ and $R^4$ are independently from each other hydrogen or methyl groups.
Preferred are the following combinations of $R^1$, $R^3$ and $R^4$:

$R^1 = R^3 = R^4 = CH_3$
or
$R^1 = R^4 = CH_3$, $R^3$=H
or
$R^1$ = H, $R^3 = R^4 = CH_3$
or
$R^1 = R^3$= H, $R^4 = CH_3$

$R^2$ represents either hydrogen or a phenol protecting group.
A phenol protecting group is a group which protects the phenolic group (OH attached directly to an aromatic ring) and can be deprotected easily, i.e. by state-of-the-art methods, to the phenolic group again.
The phenol protecting group forms with the rest of the molecule a chemical functionality which is particularly selected from the group consisting of ester, ether or acetal. The protecting group can be easily removed by standard methods known to the person skilled in the art such as described in P. G. Wuts, T. W. Greene, *Greene's Protective Groups in Organic Synthesis,* Wiley-Interscience, Hoboken, NJ, 2007, Chapter 3: Protection for phenols and catechols, the disclosure of which is incorporated herein by reference.
In case where the phenol protecting group forms an ether with the rest of the molecule, the substituent $R^2$ is in particular a linear or branched $C_{1-10}$- alkyl or cycloalkyl or aralkyl group. Preferably the substituent $R^2$ is a benzyl group or a substituted benzyl group, particularly preferred a benzyl group. A particularly preferred further substituent $R^2$ is a methyl group. The deprotecting of ether to yield the corresponding phenolic compound can be performed particularly by the method using $BF_3$ and $AlCl_3$ described in D. Woggon et al., Chembiochem 2011, 12, 118-124.
In case where the phenol protecting group forms an ester with the rest of the molecule, the ester is an ester of an organic or inorganic acid.
If the ester is an ester of an organic acid, the organic acid can be a monocarboxylic acid or a polycarboxylic acid, i.e. an acid having two or more COOH-groups. Polycarboxylic acids are preferably malonic acid, succinic acid, glutaric acid, adipic acid, maleic acid or fumaric acid.
Preferably the organic acid is a monocarboxylic acid.
Hence, the substituent $R^2$ is preferably an acyl group. The acyl group is particularly a $C_{1-7}$-acyl, preferably acetyl, trifluoroacetyl, propionyl or benzoyl group, or a substituted benzoyl group.
If the ester is an ester of an inorganic acid, the inorganic acid is preferably nitric acid or a polyprotic acid, i.e. an acid able to donate more than one proton per acid molecule, particularly selected from the group consisting of phosphoric acid, pyrophosphoric acid, phosphorous acid, sulfuric acid and sulfurous acid.
In case where the phenol protecting group forms an acetal with the rest of the molecule, the substituent $R^2$ is

preferably

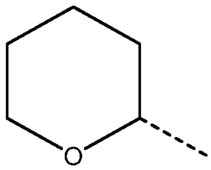 or 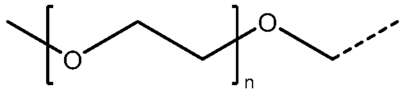

with n=0 or 1.

Hence, the acetals formed are preferably methoxymethyl ether (MOM-ether), β-methoxyethoxymethyl ether (MEM-ether) or tetrahydropyranyl ether (THP-ether). The protecting group can easily be removed by acid.

The protecting group is introduced by reaction of the corresponding molecule where $R^2$ = H with a protecting agent.

The protecting agents leading to the corresponding phenol protecting groups as well as the chemical processes and conditions for these reactions are known to the person skilled in the art. If, for example, the phenol protecting group forms an ester with the rest of the molecule, the suitable protecting agent is for example an acid, an anhydride or an acyl halide.

In the case where an ester is formed by the above reaction with the protecting agent, and that said ester is an ester of an organic polycarboxylic acid or an inorganic polyprotic acid, not all acid groups are necessarily esterified to qualify as protected in the sense of this document. Preferable esters of inorganic polyprotic acids are phosphates.

It is preferred that the protecting group $R^2$ is a benzoyl group or a $C_{1-4}$-acyl group, particularly acetyl or trifluoroacetyl group. The molecules in which $R^2$ represents an acyl group, particularly an acetyl group, can be easily prepared from the corresponding unprotected molecule by esterification. Conversely, the phenolic compound can be obtained from the corresponding ester by ester hydrolysis

It is important to realize that the introduction of the protective group by using a protecting agent can occur at different stages of manufacture of the methods described in the present document.

The residue $R^5$ represents either a linear or branched completely saturated $C_{6-25}$-alkyl group or a linear or branched $C_{6-25}$-alkyl group comprising at least one carbon-carbon double bond.

Preferably the group $R^5$ is of formula (III).

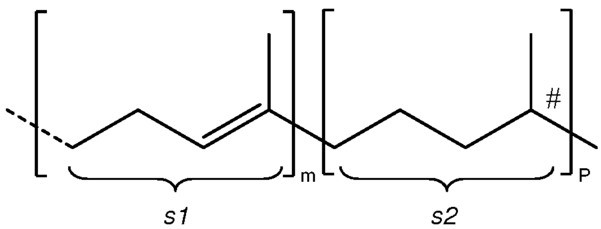

(III)

In formula (III) m and p stand independently from each other for a value of 0 to 5 provided that the sum of m and p is 1 to 5. Furthermore, the substructures in formula (III) represented by *s1* and *s2* can be in any sequence. The dotted line represents the bond by which the substituent of formula (III) is bound to the rest of the corresponding compound having $R^5$ as substituent. Furthermore, # represents a chiral centre, obviously except in case where said centre is linked to two methyl groups.

It is preferred that group $R^5$ is of formula (III-x).

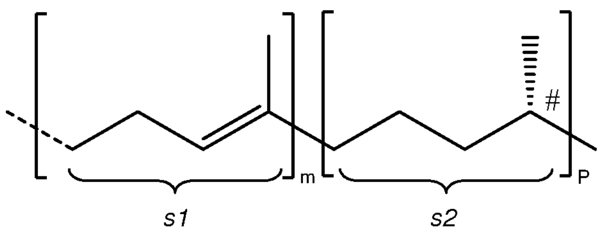

(III-x)

As mentioned above the substructures in formula (III) represented by *s1* and s2 can be in any sequence. It is, therefore, obvious that in cases where the terminal group consists of substructure s2, this terminal substructure has no

chiral centre.

The double carbon bond(s) in formula (III) and (III-x) is shown in the E-configuration, however, also the Z-configuration(s) thereof shall be meant by this formula. It is preferred that all double bonds in formula (III) and (III-x) have the E-configuration.

In one preferred embodiment m stands for 3 and p for 0.

In another preferred embodiment ρ stands for 3 and m for 0.

Therefore, $R^5$ is preferably of formula (III-A), particularly (III-ARR), or (III-B).

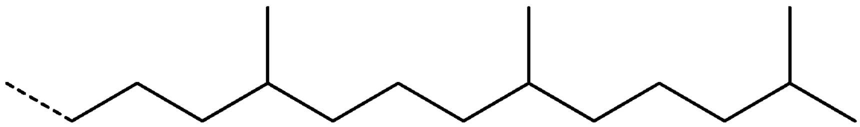

(III-A)

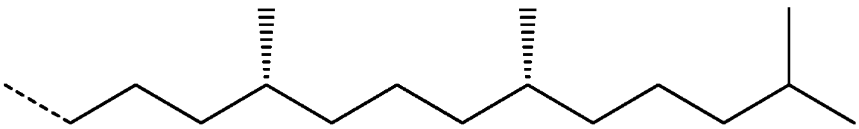

(III-ARR)

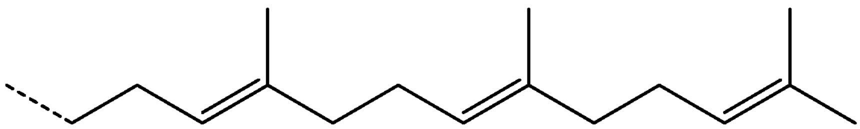

(III-B)

Compound of formula (XII) is reacted with $Al(CH_3)_3$. The person skilled in the art knows trimethylaluminium and its structure. As trimethylaluminium forms easily dimers, in the present document the term "trimethylaluminium" or "$Al(CH_3)_3$" not only strictly means the compound of formula $Al(CH_3)_3$, but also its dimers, i.e. $Al_2(CH_3)_6$. Trimethylaluminium is a commercial product, for example available from Sigma-Aldrich.

Said reaction is made in the presence of a Cu(I) salt.

It has been found that Cu(I) carboxylates or Cu(I) sulfonates are particularly suited for this method.

Preferred Cu(I) salts are Cu(I)-thiophene-2-carboxylate or Cu(I)-triflate.

Cu(I)-thiophene-2-carboxylate,

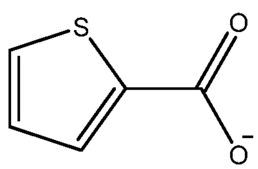

$Cu^+$, is a known compound and is often abbreviated by the scientific community as "CuTC".

Cu(I)-triflate (=Cu(I)-trifluoromethanesulfonate) is the copper (I) salt of triflic acid (=trifluoromethanesulfonic acid), and is known to the person skilled in the art to exist also as dimer, such as of copper(I) triflate $(CuOTf)_2$ or their solvate adducts such as $(CuOTf)_2$•benzene. Therefore, when using the term "Cu(I)-triflate" in this document, also its dimer is meant.

Compound of formula (XII) is reacted in the presence of a chiral compound of formula (II-A) or (II-B)

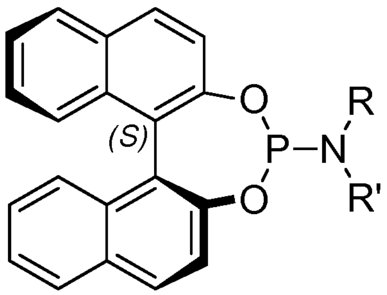

(II-A)

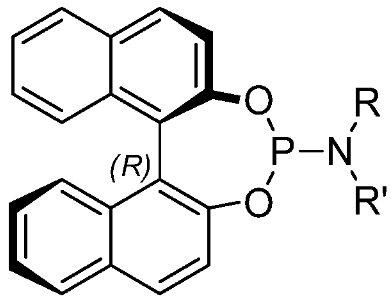

(II-B)

R' and R are independently from each other a hydrocarbyl group with 1 to 12 carbon atoms, which optionally is substituted by $OR^6$, or form together a $C_{4-6}$-alkylene group which comprises a functional group which is selected from the group consisting of

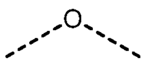 , 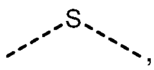 , 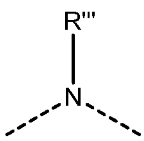 and 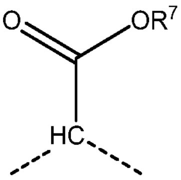

wherein R''' and $R^7$ are independently from each other a $C_{1-4}$-alkyl group.

So in one embodiment the substituents R' and R are individual hydrocarbyl substituents, which optionally is substituted by $OR^6$.

Particularly R' and/or R are individually $C_{1-6}$-alkyl groups or aryl or aralkyl or alkoxyaralkyl groups. Preferably R' and/or R are methyl or benzyl or 1-phenylethyl or 4-methoxybenzyl or 1-naphthylmethyl or 2-naphthylmethyl groups.

In a preferred embodiment R = R', particularly that R = R' = methyl group.

In a very preferred embodiment R represents a methyl group and R' represents a benzyl group.

In another embodiment the substituents R' and R form together a $C_{4-6}$-alkylene group which optionally comprises a functional group which is selected from the group consisting of

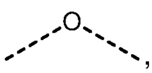 , 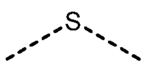 , 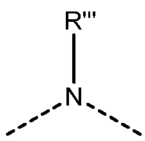 and 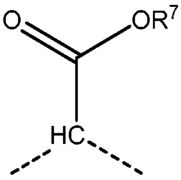

wherein R''' and $R^7$ are independently from each other a $C_{1-4}$-alkyl group.

Said alkylene group forms with the nitrogen atom to which R and R' are attached, therefore, a five to eight membered ring. Preferably said alkylene group is selected from the group consisting of

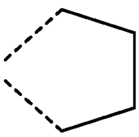 , 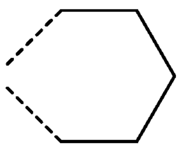 , 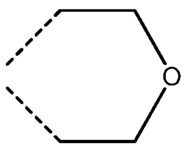 , 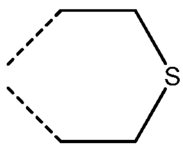 , 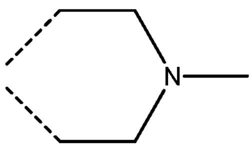 ,

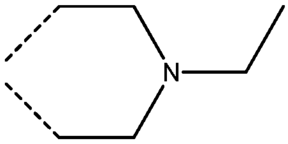, 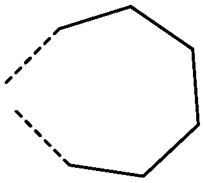, 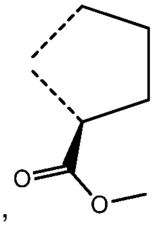 and 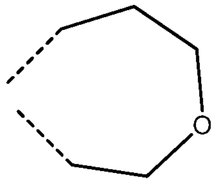

Preferably said alkylene group is an $C_{4-6}$-alkylene group or an $C_{4-6}$-alkylene group comprising one ether oxygen, and preferably is selected from the group consisting of

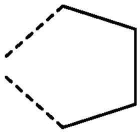, 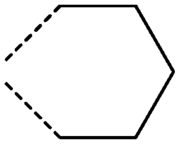 and 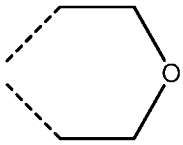

Chiral compounds of formula (II-A) and formula (II-B) are commercially available or can be obtained in high yield from (S)- or (R)- ([1,1'-binaphthalene]-2,2'-diol (=(S)- or (R)- 1,1'-bi-2-naphthol = (S)- or (R)-BINOL) which is commercially available for example from Sigma-Aldrich, and $PCl_3$ followed by addition of the corresponding amine HNRR' in the presence of triethylamine in THF as described for example in J. Org. Chem. 2005, 70, 943-951.

Compound of formula (II-A) and (II-B) are chiral compounds. Compound of formula (II-A) is the enantiomer of compound of formula (II-B).

It has been found now that for preparing the specific stereoisomer of compound (I-A) the compound (II-A) needs to be used, whereas for preparing the specific stereoisomer of compound (I-B) the compound (II-B) needs to be used. Therefore, depending on the chirality of the chiral compound (i.e. (II-A) or (II-B)) used in the reaction, the desired chirality in the compound (I-A) or (I-B) is formed at a very high preference. Hence, for obtaining the desired product, i.e. either compound (I-A) or (I-B), having the desired chirality (R or S) at the stereogenic centre at the 2-position of the ring (i.e. next to the oxygen atom), exclusively or at least at high preference, the corresponding enantiomer of chiral compound (i.e. (II-A) or (II-B) respectively) needs to be used.

It has been observed that other chiral compounds, particularly other phosphorus comprising compounds, than compound of formula (II-A) or (II-B) do not lead to the desired stereoselective addition.

Particularly, it has been found that in the presence of other chiral phosphoramidites, such as phosphoramidites derived from 3,3'-disubstituted BINOLs, or phosphites or phosphines the desired reaction does not proceed.

Particularly, it has been found that the corresponding chiral compounds having substituents directly attached to the naphthyl ring (of the BINOL) also do not show the desired action. Therefore, the structure of compounds of formula (II-A) or (II-B) is a key element of the invention.

Compounds of formula (II-A) or (II-B can be produced from the corresponding BINOL which is converted to the chlorophospite by means of PCl3 and reaction with the corresponding secondary amine HNRR' in the presence of triethylamine ($Et_3N$) as shown for compound of formula (II-A).

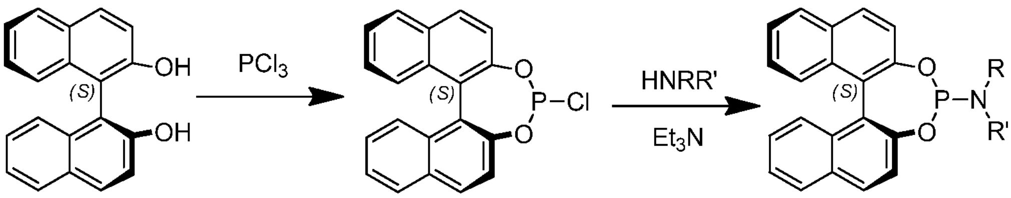


It has been observed that the molar ratio of $Al(CH_3)_3$ to compound of formula (XII) is preferably between 1.1 : 1 and 10:1, more preferred between 1.5:1 and 5:1.

Furthermore, it is preferred that the amount of Cu(I) salt, on basis of the copper content, is 1 mol-% to 10 mol-%, more preferred 2 mol-% - 8 mol-%, relative to the amount of compound of formula (XII).

Furthermore, it is preferred that the amount chiral compound of formula (II-A) or (II-B) is 1 mol-% to 20 mol-%, more preferred 5 mol-% - 15 mol-%, relative to the amount of compound of formula (XII).

It is further preferred that the molar ratio of Cu(I) salt to chiral compound of formula (II-A) or (II-B) is between 1:2 - 2:1, particularly between 1:1.1 and 1:1.9.

It is preferred that the reaction is performed in an inert solvent, particularly in an ether, particularly tetrahydrofuran and diethyl ether.

Reaction of compound of formula (XII) with $Al(CH_3)_3$ in the presence of a Cu(I) salt and chiral compound of formula (II-A) or (II-B) furnishes an intermediate of the following structure

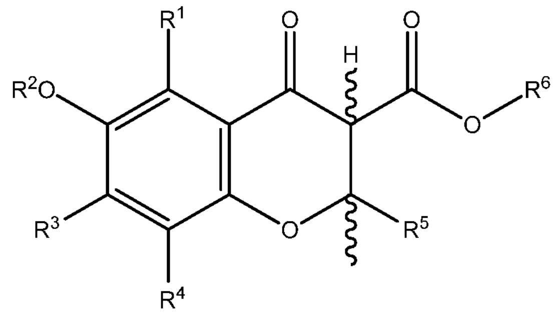

In a further step of the method this intermediate is hydrolysed and decarboxylated by heating to a temperature of 100-190 °C, particularly of 150-190°C.

It has been observed that the decarboxylation is preferably carried out by heating to a temperature of 100-190 °C in the presence of water and dimethyl sulfoxide and LiCl.

Details of the decarboxylation method are disclosed in P. Krapcho et al., J. Org. Chem. 1978, 43, 138-147, the whole disclosure of which is incorporated herein by reference.

This reaction then yields the desired product, i.e. compound of formula (I-A) or (I-B).

It has been observed that the reaction is highly stereoselective. This is expressed in this document by the isomeric excess (*"ie"*) being determined by the absolute value of the difference of amounts of the isomers ([2R]) having R configuration at stereogenic carbon centre in position 2 and the isomers ([2S]) having S configuration at stereogenic carbon centre in position 2, divided by their sum: and is expressed in %:

$$ie = abs\left(\frac{[2R]-[2S]}{[2R]+[2S]}\right)$$

We have been able to show that using the current invention isomeric excess of 50 % or more, particularly of more than 75%, preferably of more than 92%, corresponding to a ratio of [2R]/[2S], or of [2S]/[2R], of more than 24:1, can be easily achieved.

Compound of formula (I-A) or of formula (I-B), respectively, can be used for the preparation of compound of formula (IV-A) or of formula (IV-B), respectively and for the preparation of compound of formula (V-A) or of formula (V-B) respectively. Hence, the method of preparation of these compounds represent further aspects of the present invention.

Hence, the invention also relates to a method of preparing compound of formula (IV-A) or (IV-B).

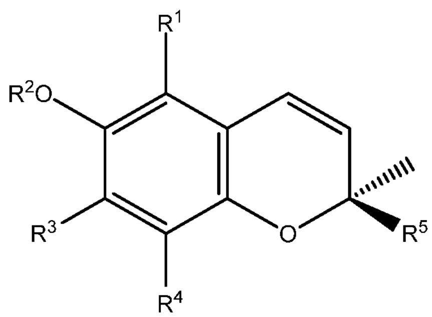

(IV-A)

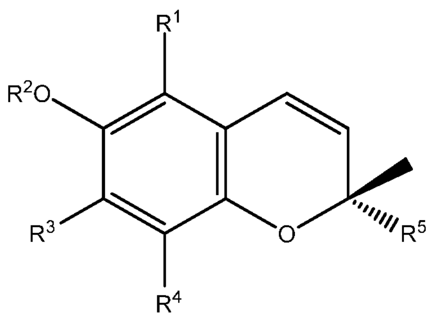

(IV-B)

This method comprises the steps

a) preparing compound of formula (I-A) or (I-B) as described above in great detail;
b) reduction of compound of formula (I-A) or (I-B) by a hydride, particularly by sodium borohydride;
c) treatment by acid

with the proviso that when compound of formula (I-A) is used in step a) the compound of formula (IV-A) is formed and when compound of formula (I-B) is used in step a) the compound of formula (IV-B) is formed.

The reduction of compound of formula (I-A) or (I-B) by a hydride, particularly by sodium borohydride and the subsequent treatment by acid is particularly done as described in Kabbe and Heitzer, Synthesis 1978, 888-889 or in Bradley et al., J. Chem. Res., Miniprint 1993, 210, the whole disclosure of which is incorporated herein by reference.

The preferred compounds of formula (IV-A) or (IV-B), respectively, are (2R)- or (2S)-3,4-dehydro-tocopherols and (2R)- or (2S)-3,4-dehydro-tocotrienols and (R)-2-methyl-2-(4-methylpent-3-en-1-yl)-2H-chromen-6-ol or (S)-2-methyl-2-(4-methylpent-3-en-1-yl)-2H-chromen-6-ol, particularly (2R)-3,4-dehydro-α-tocopherol, (2S)-3,4-dehydro-α-tocopherol, (2R)-3,4-dehydro-β-tocopherol, (2S)-3,4-dehydro-β-tocopherol, (2R)-3,4-dehydro-γ-tocopherol, (2S)-3,4-dehydro-γ-tocopherol, (2R)-3,4-dehydro-δ-tocopherol, (2S)-3,4-dehydro-δ-tocopherol; (2S)-3,4-dehydro-α-tocotrienol, (2R)-3,4-dehydro-β-tocotrienol, (2S)-3,4-dehydro-β-tocotrienol, (2R)-3,4-dehydro-γ-tocotrienol, (2S)-3,4-dehydro-γ-tocotrienol, (2R)-3,4-dehydro-δ-tocotrienol, (2S)-3,4-dehydro-δ-tocotrienol; preferably (2R)-3,4-dehydro-α-tocotrienol and (2R,4'R,8'R)-3,4-dehydro-α-tocopherol.

2-Methyl-2-(4-methylpent-3-en-1-yl)-2H-chromen-6-ol which is also known as cordiachromene is a particularly important compound which can be obtained by the above method. Cordiachromene has shown interesting properties such as antibacterial or anti-inflammatory activity.

In further aspect, the invention also relates to a method of preparing compound of formula (V-A) or (V-B).

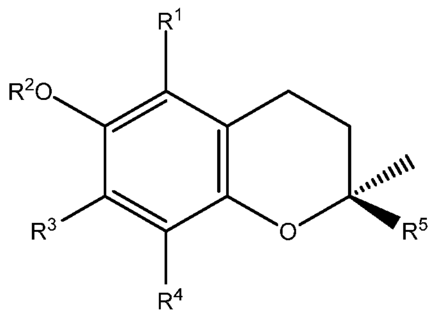
(V-A)

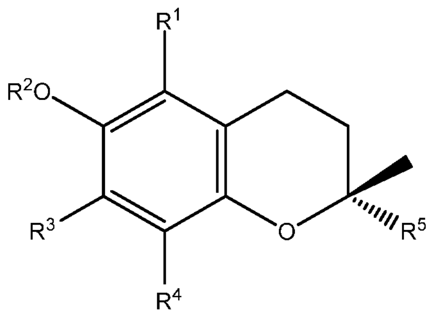
(V-B)

This method comprises the steps

a) preparing compound of formula (I-A) or (I-B) as described above in great detail;
b') hydrogenation of compound of formula (I-A) or (I-B), particularly by means of molecular hydrogen in the presence of a Pd/C catalyst or by metallic zinc in the presence of an acid;

with the proviso that when compound of formula (I-A) is used in step a) the compound of formula (V-A) is formed and when compound of formula (I-B) is used in step a) the compound of formula (V-B) is formed

The hydrogenation of compound of formula (I-A) or (I-B) is particularly done as disclosed for in US 6,096,907 the whole disclosure of which is incorporated herein by reference.

An alternative method of preparing compound of formula (V-A) or (V-B) is by reduction of compound of formula (IV-A) or (IV-B). This can be done for example by the method as described in Schudel et al., Helv. Chim. Acta, 46, 2517-2526 (1963), the entire disclosure of which is incorporated herein by reference, in particularly by using sodium / ethanol.

The preferred compounds of formula (V-A) or of formula (V-B), respectively, are α-tocopherol, β-tocopherol, γ-tocopherol, δ-tocopherol, α-tocotrienol, β-tocotrienol, γ-tocotrienol, δ- tocotrienol, particularly (2R, 4'R, 8'R)-α-tocopherol.

It has been observed that starting from compound of formula (I-A) or (I-B), respectively, the reactions involved to yield compounds of formula (IV-A) or (IV-B), respectively, or (V-A) or (V-B), respectively, are carried out without racemization of the chirality at stereogenic centre at the 2 position in the ring. This has the benefit of obtaining compound of formula (I-A) or (I-B), respectively, at very high isomeric excess *(ie)* also for the subsequent products in the reaction chain.

It is evident that despite the compounds (I-A) or (I-B), (IV-A) or (IV-B), (V-A) or (V-B) are obtained in a very high isomeric excess (*ie*) they can be further purified by known methods, such as by chromatography on chiral phase, particularly as disclosed by WO 2012/152779 A1, the entire disclosure of which is incorporated herein by reference.

As the compound of formula (XII) is one of the key starting materials used in the method of preparing compound of formula (I-A) or (I-B), this substance, its precursors as well as the method of preparing them represent further aspects of the present invention.

Therefore, compound of formula (XII) is a further aspect of the present invention

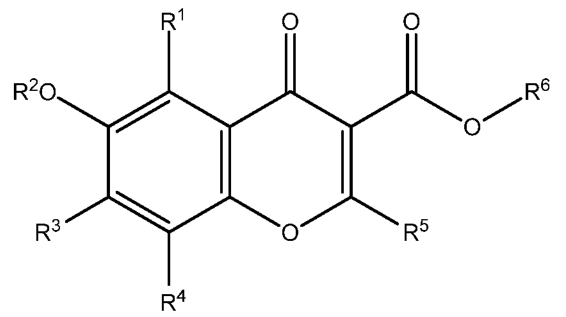

(XII).

The substituents $R^1$, $R^2$, $R^3$, $R^4$, $R^5$ and $R^6$ as well as their preferred embodiments have been discussed already before in great detail.

Compound of formula (XII) can be produced by a method comprising an intramolecular reaction of formula (XI) in the presence of methanol binding agent in methanesulfonic acid.

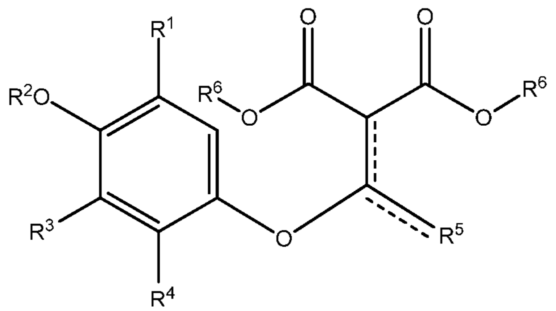

(XI)

$R^6$ represents a methyl or ethyl group or *tert.*-butyl group and the dotted lines denote the two alternative positions of one carbon-carbon double bond. This means that the formula (XI) represents the two formulae (XI-i) and (XI-ii). It is preferred that both $R^6$ stand for the same substituent.

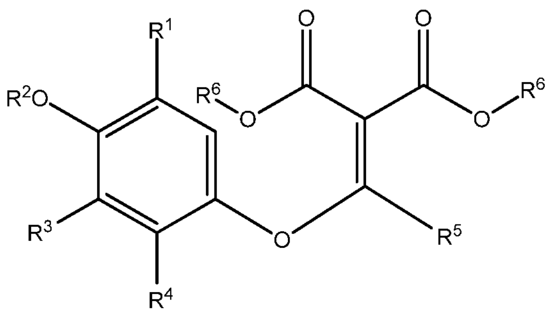

(XI-i)

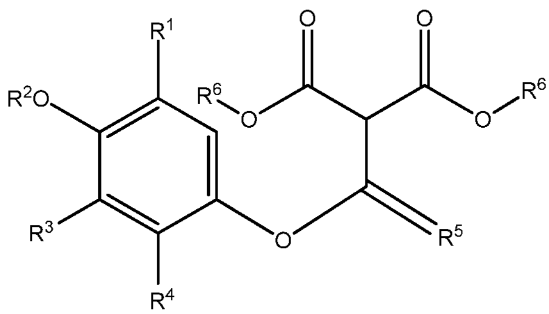

(XI-ii)

Strictly seen, the representation of the compound of formula (XI-ii) is formally not correct, as the substituent $R^5$ has been defined before as being an alkyl, but is used for the sake of simplicity nevertheless. For clarification it is mentioned here that in this case "alkyl", in the context of $R^5$, means that the alkyl group as defined before can also be bound by a double bond to the rest of the molecule.

Therefore, particularly the compounds of formula (XI-ii1), (XI-ii2), (XI-ii3) are to be considered to fall under the scope of compound of formula (XI).

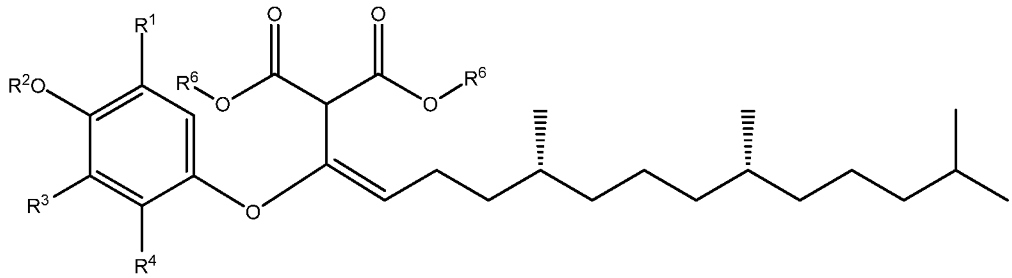

(XI-ii1)

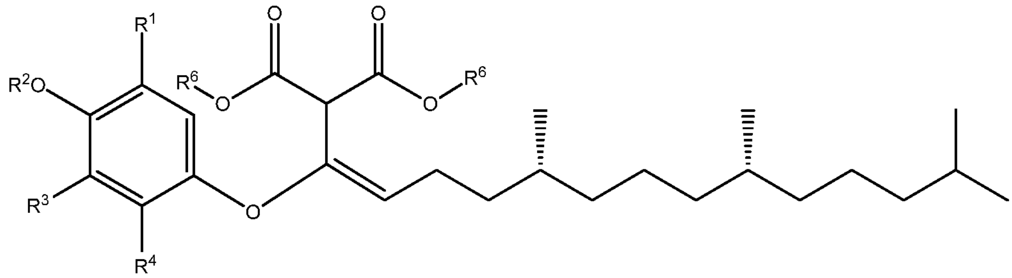

(XI-ii2)

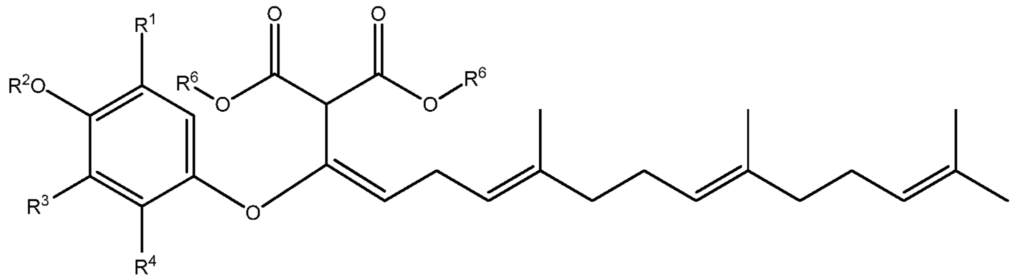

(XI-ii3)

However, for the preparation of formula (XII) mentioned before it does not matter if compound (XI-i) or (XI-ii) or a mixture of (XI-i) and (XI-ii) is used, as they all yield the same product, i.e. compound of formula (XII).

The methanol binding agent is a material which binds methanol either physically or chemically.

Methanol can be bound physically. This is for example the case in molecular sieves with sufficiently large pores (such as type 4 Å molecular sieve where methanol is adsorbed on its surface within the pores.

Methanol can also be bound chemically. In this case methanol reacts chemically with the methanol binding agent. It is preferred that the methanol binding agent is a solid. Particularly preferred as methanol binding agent is phosphorus pentoxide. Particularly useful for the above is Eaton's reagent.

The reaction temperature for this reaction is preferably between 40 and 70 °C, particularly between 50 and 60°C. The reaction preferably takes place during 10 minutes and 6 hours, preferably between 0.5 and 3 hours.

The starting material for this reaction, i.e. compound of formula (XI) and its method of preparation represent further aspects of the present invention.

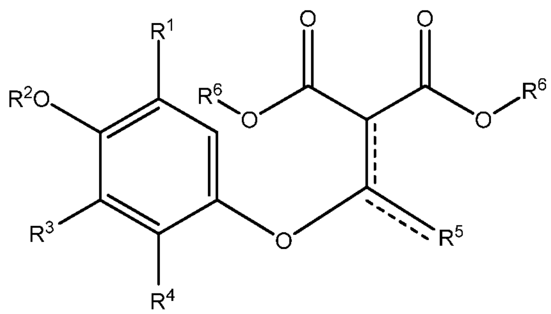

(XI)

The substituents $R^1$, $R^2$, $R^3$, $R^4$, $R^5$ and $R^6$ as well as their preferred embodiments have been discussed already before in great detail.

Compound of formula (XI) can be prepared by a method comprising the steps of

- reacting compound of formula (X) with sulfonyl halide or anhydride of an organic sulfonic acid and in the presence of a base, particularly in the presence of a tertiary amine; to form an enol sulfonate intermediate;
- reacting said enol sulfonate intermediate with compound of formula (XI-a) in the presence of a base, particularly in the presence of a tertiary amine.

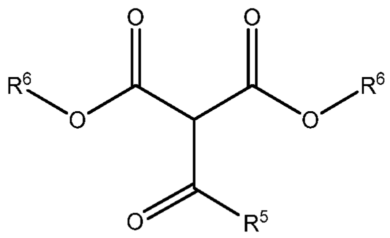

(X)

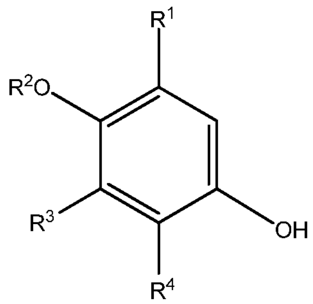

(XI-a)

This reaction leads to two different products as mentioned above, i.e. compound of formula (XI-i) and (XI-ii). Typically, approximately, an equimolar mixture of (XI-i) and (XI-ii) is obtained.

Compound of formula (X) is in an equilibrium with its enol structure of formula (X').

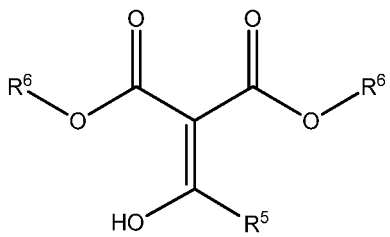

(X')

For sake of simplicity in the present document when compound of formula (X) is given also its enol form, i.e. formula (X') is meant.

Alkane- or arene sulfonyl halides are particular suitable sulfonyl halides.

Particularly sulfonyl chlorides have shown to be useful as sulfonyl halides. Preferred sulfonyl chloride is tosyl chloride.

Particularly the anhydride of triflic acid (= trifluoromethanesulfonic anhydride = triflic anhydride) is particularly useful as anhydride of an organic sulfonic acid.

Triethylamine has shown to be a particular suitable tertiary amine.

The above reaction is performed stepwise. In a first step compound of formula (X) is reacted in the presence of a base with sulfonyl halide or anhydride of organic sulfonic acid to form an enol sulfonate intermediate of the following formula

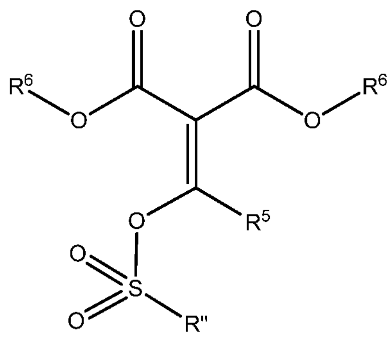

in which R" stands for the organic substituent of the sulfonyl chloride or anhydride of an organic sulfonic acid used, such as trifuoromethyl or p-methylphenyl (=tolyl) in the case of triflic anhydride or tosyl chloride.

The reaction is preferably done in an inert solvent, particularly in dichloromethane, at temperatures between -10 °C and +30 °C, particularly between 0°C and 25°C.

In a second step, the above intermediate is reacted with the phenolic compound of formula (XI-a) in the presence of a base, particularly a tertiary amine, which typically takes place at a temperature between 10 °C and 50 °C, particularly between 20°C and 30°C.

The molar ratio of compound of formula (XI-a) to compound of formula (X) is typically between 2:1 and 2:1, preferably 0.9:1-1.5:1.

The molar ratio of compound of the base to compound of formula (X) in the first step is typically between 1:1 and 2:1, preferably 1.1:1 - 1.5:1.

The molar ratio of compound of sulfonyl halide or anhydride of an organic sulfonic acid to compound of formula (X) is typically between 1:1 and 2:1, preferably 1.1:1 - 1.5:1.

The starting material for this reaction, i.e. compound of formula (X) and its method of preparation represent further aspects of the present invention.

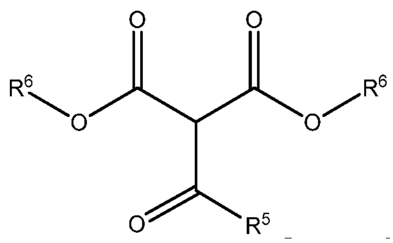

(X)

The substituents $R^5$ and $R^6$ as well as their preferred embodiments have been discussed already before in great detail.

Compound of formula (X) can be prepared by two different routes.

In the first route compound of formula (X) is prepared according to a method comprising the step of reacting compound of formula (X-a) with compound of formula (X-b) in the presence of alkali metal hydride in an ether, particularly in tetrahydrofuran.

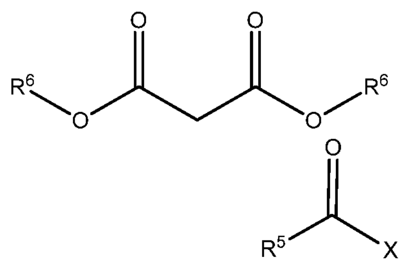

(X-a)

(X-b)

X in compound of formula (X-b) stands for Cl or Br or

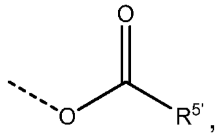

preferably Cl; wherein X = Cl or Br; wherein $R^{5'}$ represents either a linear or branched completely saturated $C_{1-25}$-alkyl group or a linear or branched $C_{2-25}$-alkyl group comprising at least one carbon-carbon double bond; particularly $R^{5'}$.

Suitable alkali metal hydrides are known to the person skilled in the art. Particularly useful is sodium hydride.

The reaction is preferably done at temperatures between - 10 °C and 30 °C, particularly between 0°C and 25°C.

It has been observed that a molar ratio of the alkali metal hydride to compound of formula (X-a) is preferably 1.5 to 3, particularly between 1.8 and 2.2.

The molar ratio of compound of formula (X-b) to compound of formula (X-a) is typically between 1:2 - 2:1, particularly between 0.8 :1 and 1.2:1, preferably in the range of 1:1 to 1.1:1.

In the second route compound of formula (X) is prepared according to a method comprising the steps of

i) reacting compound of formula (X-a') with compound of formula (X-b')

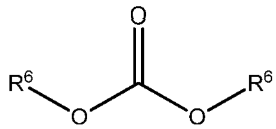

(X-a')

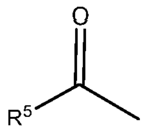

(X-b')

in the presence of alkali metal hydride in an ether, particularly in tetrahydrofuran;
ii) reacting with compound of formula (X-c')

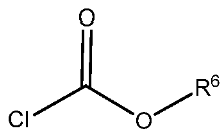

(X-c')

in the presence of a base, particularly pyridine, and a metal salt.

Suitable alkali metal hydride are known to the person skilled in the art. Particularly useful is sodium hydride or potassium hydride. It can be advantageous to use a mixture of different metal hydrides, particularly a mixture of sodium hydride and potassium hydride.

Suitable metal salt are particularly metal salts of groups 2 and 13 of the periodic table as well as the transition metals. Preferably the metal salt is an Mg salt, particularly $MgCl_2$.

It has been further observed that compound of formula (XXI-A) or (XXI-B) can be prepared by a method comprising the step of reacting compound of formula (XXII) with $Al(CH_3)_3$ in the presence of a Cu(I) salt and chiral compound of formula (II-A) or (II-B) with the proviso that for the preparation of compound (XXI-A) the compound (II-A) is used and that for the preparation of compound (XXI-B) the compound (II-B) is used.

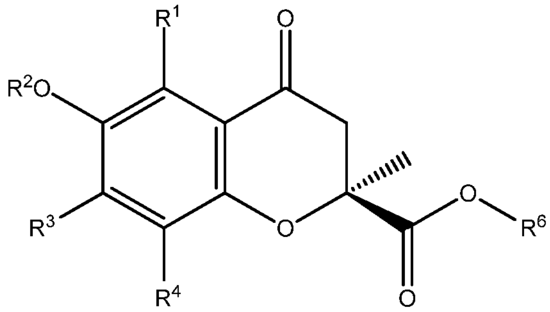

(XXI-A)

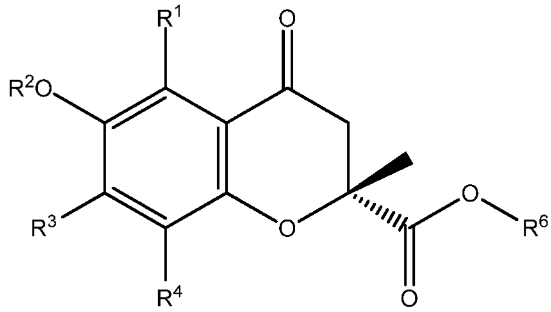

(XXI-B)

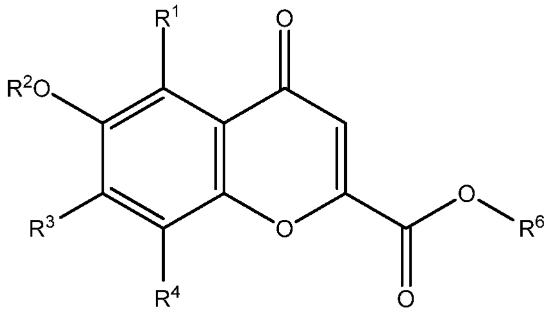

(XXII)

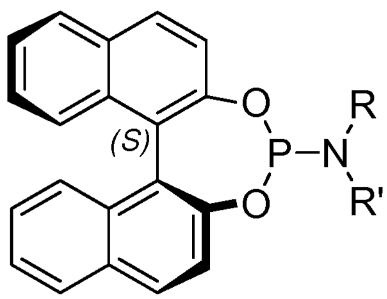

(II-A)

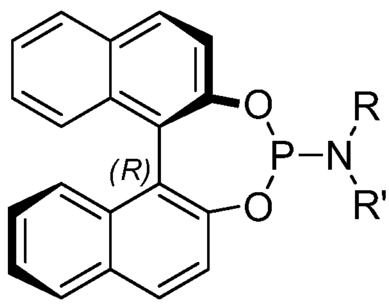

(II-B)

The substituents $R^1$, $R^2$, $R^3$, $R^4$ and $R^6$ as well as their preferred embodiments have been discussed already before in great detail.

Compound of formula (XXII) or (XXI-A) or (XXI-B), respectively, are structurally very similar to compounds of formula (XII) or (I-A) or (I-B), respectively.

They are very similar, particularly in the fact that they are both chromenones, and both carry a $COOR^6$ group, which is either attached at C2 or C3 of the chromenone. In both cases a methyl group is stereoselectively introduced at C2 of the chromenone moiety, resulting in chiral compounds of formula (XXI-A) or (XXI-B), respectively, as well as (I-A) or (I-B), respectively.

Details, conditions and preferred embodiments, of the method for preparing compound of formula (I-A) or (I-B) have been mentioned before in great detail and apply to the method of preparing compound of formula (XXI-A) or (XXI-B) as well.

Hence, for obtaining the desired product, i.e. either compound (XXI-A) or (XXI-B), having the desired chirality (R or S) at the stereogenic centre at the 2-position of the ring (i.e. next to the oxygen atom), exclusively or at least at high preference, the corresponding enantiomer of chiral compound (i.e. (II-A) or (II-B) respectively) needs to be taken.

The compound of formula (XXII) can be prepared by a method comprising the steps of reacting compound of formula (XX) with compound of formula (XXI-a)

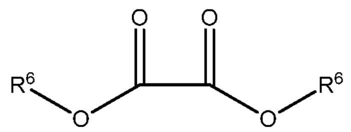

(XX)

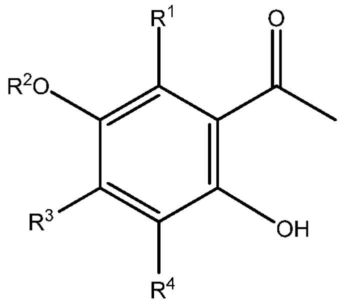

(XXI-a)

in the presence of alkali metal ethanolate or methanolate followed by treatment with an acid.

The acid is particularly a strong mineral acid, preferably $H_2SO_4$.

The reaction is preferably done in an inert solvent, particularly in a hydrocarbon, preferably toluene, at temperatures of 80- 130°C, preferably at reflux temperature of toluene.

The molar ratio of compound of formula (XX) to compound of formula (XXI-a) is typically between 1:1 and 10:1, preferably 3:1 - 6:1.

The molar ratio of the alkali metal ethanolate or methanolate to compound of formula (XXI-a) is typically between 1:1 and 20:1, preferably 8:1 - 15:1.

The molar ratio of compound of mineral acid to compound of formula (XXI-a) is typically between 1:1 and 1:20, preferably 1:5 1- 1:15.

Particularly the compound of formula (XXII) is selected from the group consisting of

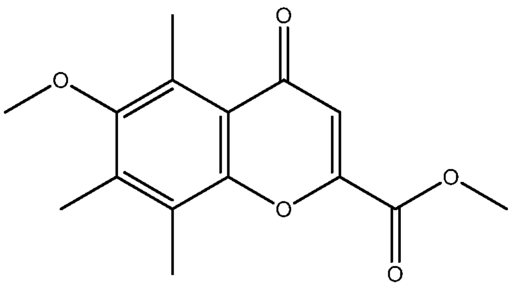

and

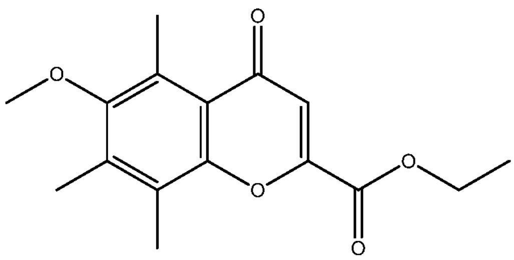

Compounds of formula (XXI-a) could be prepared by a method comprising the steps of - acylating compound of formula (XI-a) to yield compound of formula (XXX)

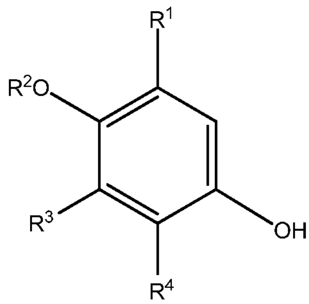

(XI-a)

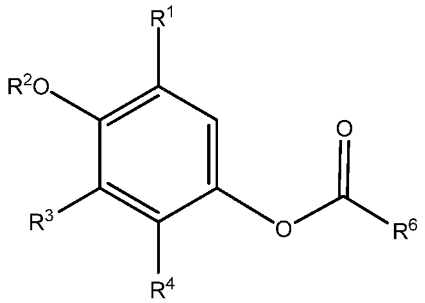

(XXX)

followed by

- Fries-rearrangement of compound of formula (XXX) in the presence of a Lewis acid.

The acylation of compound of formula (XI-a) is performed by the reaction with a corresponding acylating agent, particularly with acyl halide, preferably with acetyl chloride or pivaloyl chloride, or with an anhydride, particularly acetic anhydride.

The Fries-rearrangement is performed preferably in the presence of $TiCl_4$ as Lewis acid. Furthermore, $BF_3$ or $BF_3$-diethyl ether adduct or $BF_3$-acetic acid adducts can be used.

It is preferred that molar ratio of Lewis acid to the amount of compound of formula (XI-a) used is at least 1.5 particularly between 1.6 and 3.0.

The Fries-rearrangement is preferably carried out in an inert solvent, preferably in dichloromethane at a temperature between 30 and 50°C, preferably at reflux temperature of dichloromethane.

Particularly the compound of formula (XXX) is selected from the group consisting of

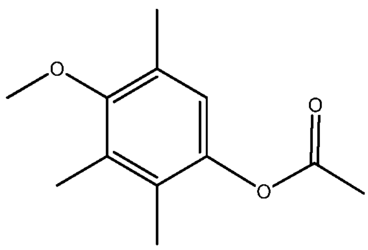

and

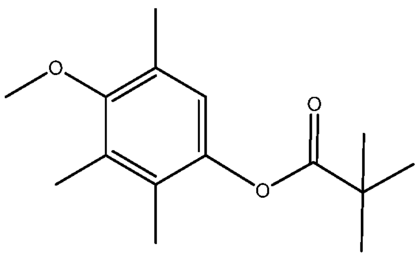

Compounds of formula (XXI-A) or (XXI-B), respectively, can be reduced under retention of the configuration to compounds of formula (XXV-A) or (XXV-B) respectively, as already discussed in detail for the reduction of compounds of formula (I-A) or (I-B) to formula (V-A) or (V-B).I

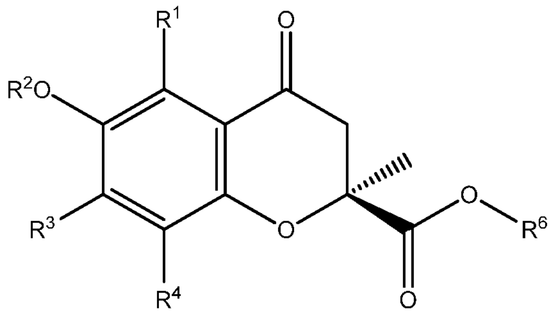

(XXI-A)

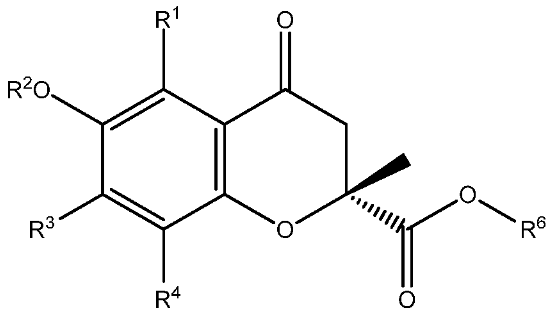

(XXI-B)

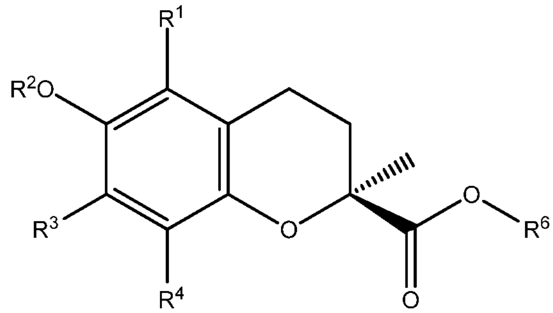

(XXV-A)

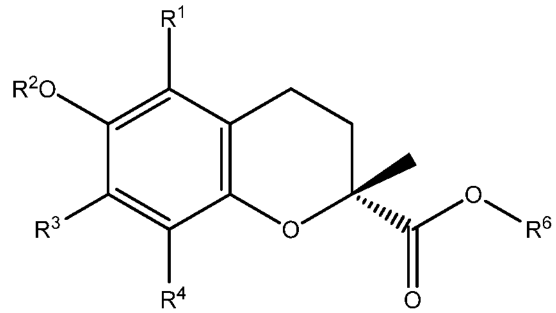

(XXV-B)

Compounds of formula (XXV-A) or (XXV-B) can then further be reduced to compounds of formula (XXVI-A) or (XXVI-B) or of formula (XXVII-A) or (XXVII-B) which in turn can be chemically transformed by known methods to compound of formula (V-A) or (V-B).

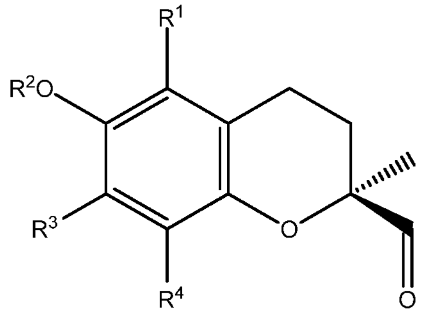

(XXVI-A)

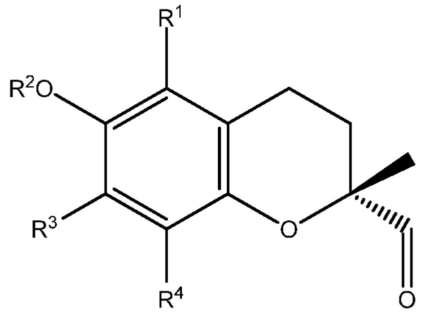

(XXIV-B)

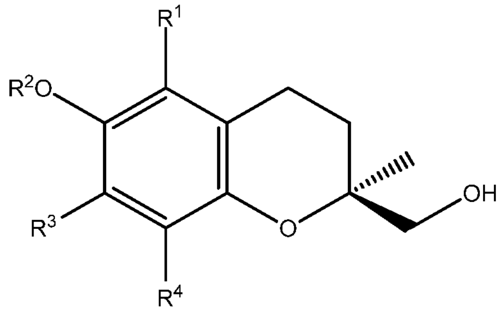

(XXVII-A)

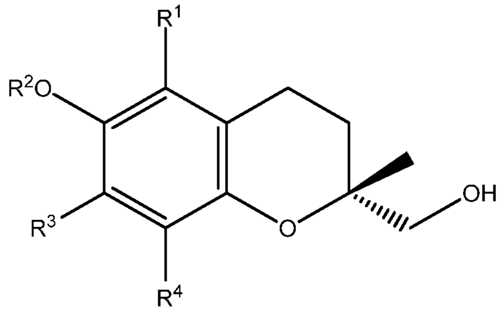

(XXVII-B)

Figures

In the following paragraphs an overview of the different reactions of the present inventions are given in a schematic way. This, however, is not to be understood as limiting the invention to the embodiments described here in the figures.

Figures 1 and 2 give an overview of the reactions involved in the present invention.

The central aspect of the present invention is the method of preparing compound of formula (I-A) or (I-B) comprising the step of reacting compound of formula (XII) with $Al(CH_3)_3$ in the presence of a Cu(I) salt and chiral compound of formula (II-A) or (II-B) followed by ester hydrolysis and decarboxylation by heating to a temperature of 100-190 °C with the proviso that for the preparation of compound (I-A) the compound (II-A) is used and that for the preparation of compound (I-B) the compound (II-B) is used. This reaction is schematically represented in figure 1 by the reaction marked by ⑤ and is highlighted in a frame. Compound of formula (I-A) can be used for preparing compound of formula (IV-A) by a reaction marked as ⑥, which in turn can be transformed into compound of formula (V-A) by a reaction marked as ⑦. An alternative route yielding compound of formula (V-A) is the direct conversion of compound of formula (I-A) to compound of formula (V-A) by a reaction marked as ⑧.

For sake of simplicity in the representation of figure 1 only compound of formula (I-A) and, subsequently, also only formulae (IV-A) and (V-A) are shown. For obtaining this compound, exclusively or at least at very high yield, chiral compound of formula (II-A) is used in this reaction marked by ⑤. If chiral compound of formula (II-B) would be used chiral compound of formula (I-B) would be obtained, exclusively or at least in very high yield. In that case the compounds of formula (IV-B) and (V-B) would be obtained accordingly by the subsequent reactions marked as ⑥, ⑦ and ⑧.

The invention relates also to new substances and methods in preparing compound (XII), one of the key starting materials of the key reaction marked as ⑤.

Figure 1 shows the compound of formula (XII) as well as its preparation by an intramolecular reaction of compound of formula (XI) in the presence of methanol binding agent in methanesulfonic acid, which is depicted schematically by the reaction marked as ④.

Figure 1 shows the compound of formula (XI) as well as its preparation by reacting compound of formula (X) with sulfonyl halide or anhydride of an organic sulfonic acid and in the presence of a base to form an enol sulfonate intermediate and reacting said enol sulfonate intermediate with compound of formula (XI-a) in the presence of a base, schematically depicted by the reaction marked as ③.

Figure 1 shows the compound of formula (X) as well as its two preparation pathways.

In the first variant, compound of formula (X) is prepared by the reaction of formula (X-a) with compound of formula (X-b) in the presence of alkali metal hydride in an ether, which is depicted schematically by the reaction marked as ①.

In the second variant, compound of formula (X) is prepared by the reaction of compound of formula (X-a') with compound of formula (X-b') in the presence of alkali metal hydride in an ether and subsequently reacting with compound of formula (X-c') in the presence of a base, which is depicted schematically by the reaction marked as ②

A further central aspect of the present invention is the method of preparing compound of formula (XXI-A) or (XXI-B) comprising the step of reacting compound of formula (XXII) with $Al(CH_3)_3$ in the presence of a Cu(I) salt and chiral compound of formula (II-A) or (II-B) followed by ester hydrolysis and decarboxylation by heating to a temperature of 100-190 °C with the proviso that for the preparation of compound (XXI-A) the compound (II-A) is used and that for the preparation of compound (XXI-B) the compound (II-B) is used. This reaction is schematically represented in figure 2 by reaction marked as ❹ and is highlighted in a frame. Compound of formula (XXI-A) can be used for preparing compound of formula (XXV-A) by a reaction marked as ❺, which in turn can be reacted to either compound of formula (XXVI-A) by a reaction marked as❻ , or to compound of formula (XXVII-A) by a reaction marked as ❼. Compounds of formula (XXVI-A) and of formula(XXVII-A) can in turn yield compound of formula (V-A) by means of subsequent reactions marked by ❽ and ❾.

For sake of simplicity, in this representation of figure 2 only compound of formula (XXI-A) and, subsequently, also only formulae (XXV-A) and (XXVI-A) and (XXVII-A) and (V-A) are shown. For obtaining this compound, exclusively or at least at very high yield, chiral compound of formula (II-A) is used in this reaction marked as ❹. If chiral compound of formula (II-B) would be used chiral compound of formula (XXI-B) would be formed, exclusively or at least in very high yield. In that case, the compounds of formulae (XXV-B) and (XXVI-B) and (XXVII-B) and (V-B) would be accordingly obtained by the subsequent reactions marked as ❺, ❻, ❼, ❽ and ❾.

The invention relates also to new substances and methods in preparing compound (XXII), one of the key starting materials of the key reaction marked as ❹.

Figure 2 shows the compound of formula (XXII) as well as its preparation by the steps of reacting compound of formula (XX) with compound of formula (XXI-a) in the presence of alkali metal ethanolate or methanolate followed by treatment with an acid, which is depicted schematically by the reaction marked as ❸.

Figure 2 shows the compound of formula (XXI-a) as well as its preparation by reaction of acylating compound of formula (XI-a), marked as ❶, to yield compound of formula (XXX) followed by Fries-rearrangement of compound of formula (XXX) in the presence of a Lewis acid, marked as ❷.

## Examples

The present invention is further illustrated by the following experiments.

Preparation of (7R,11R)-methyl 7,11,15-trimethyl-3-oxohexadecanoate (②)

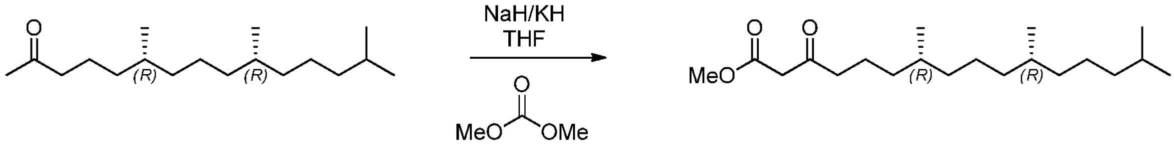


NaH (8.94 g, 223.5 mmol, 60% in paraffin oil, 3.0 eq.) and KH (1.08 g, 13.72 mmol, 50% in paraffin oil, 0.18 eq.) was washed with n-hexane (3 x) and residual solvent was removed in vacuo. The solid residue was suspended in THF (90 mL) followed by addition of dimethyl carbonate (18.8 mL, 223.5 mmol, 3.0 eq.). The resulting suspension was heated to reflux and then 1 mL of a solution of (6*R*,10*R*)-trimethylpentadecan-2-one (24.1 mL, 20.0 g, 74.5 mmol, 1.0 eq.) (synthesized according to example **19** in WO 2012/171969 A1) in THF (tetrahydrofuran) (60 mL) was added via dropping funnel. After 15 min the remaining solution of (6*R*, 10*R*)-trimethylpentadecan-2-one was added dropwise. Subsequently, the reaction was heated to reflux for 4 h. After cooling by an ice-bath, the reaction was quenched by addition of 1 N HCl resulting in evolution of gas. The aqueous phase was extracted with *tert*.-butyl methyl ether (TBME) and the organic phase was washed with water and brine. After drying over $MgSO_4$ the organic phase was concentrated in vacuo, furnishing 23.17 g (70.9 mmol, 95% yield) of (7*R*,11 R)-methyl 7,11,15-trimethyl-3-oxohexadecanoate as pale yellow oil.
**$R_f$** ($SiO_2$ cyclohexane/EtOAc 5:1) = 0.58
**FT-IR** (ATR) [$cm^{-1}$] = 2947 (s), 2929 (s), 2856 (s), 1748 (s), 1715 (s), 1652 (m), 1629 (m), 1456 (s), 1407 (m), 1375 (m), 1361 (m), 1318 (m), 1234 (s), 1151 (s), 1074 (w), 1020 (w), 830 (w), 799 (w), 737 (w).
**$^1$H NMR** (300 MHz, $CDCl_3$): δ 3.73 (s, 3H), 3.45 (s, 2H), 2.52 (t, *J* = 7.4 Hz, 2H), 1.71-1.47 (m, 2H), 1.45-0.98 (m, 17H), 0.87-0.84 (m, 12H) ppm.

Additionally, a second set of low intensity signals was observed, which could be assigned to the enol tautomer of the product: (300 MHz, $CDCl_3$): δ 12.03 (s) and 4.99 (s). **$^{13}$C-NMR** (75 MHz, $CDCl_3$): δ 203.0, 167.8, 52.5, 49.2, 43.5, 39.5, 37.5, 37.4, 37.3, 36.4, 32.9, 32.8, 28.1, 24.9, 24.6, 22.9, 22.8, 21.2, 19.9, 19.7 ppm.
**$[\alpha]_D^{20}$** = -3.3, $[\alpha]_{546}^{20}$ = -4.2.

<u>Preparation of dimethyl 2-((5R,9R)-5,9,13-trimethyltetradecanoyl)malonate from (7R, 11R)-methyl 7,11,15-trimethyl-3-oxohexadecanoate</u> (②)

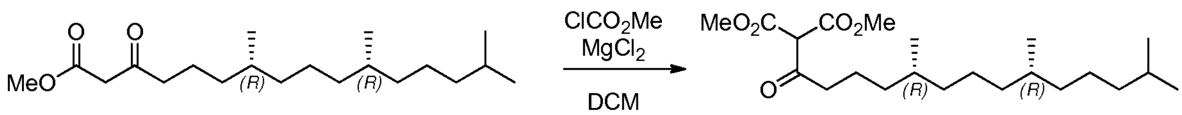


$MgCl_2$ (12.93 g, 61.3 mmol, 1.93 eq.) was suspended in $CH_2Cl_2$ (350 mL) and treated with a solution of (7*R*,11R)-methyl 7,11,15-trimethyl-3-oxohexadecanoate (23.03 g, 70.5 mmol, 1.0 eq.) in $CH_2Cl_2$ (50 mL). The resulting suspension was cooled to 0 °C and pyridine (21.9 mL, 271 mmol, 3.8 eq.) was added. The mixture was stirred for 20 min at 0 °C. Methyl chloroformate (10.5 mL, 135.5 mmol, 1.93 eq.) was added dropwise and the reaction was stirred for 20 min at 0 °C followed by 20 min at room temperature. After 40 h the reaction was cooled to 0 °C and additional methyl chloroformate (10.5 mL, 135.5 mmol, 1.93 eq.) was added. The reaction was allowed to stir for 1 d at room temperature. The reaction was cooled to 0 °C and quenched with 2N HCl. The organic phase was separated and the aqueous phase was extracted with *tert*.-butyl methyl ether (TBME). The combined organic phases were washed with brine and subsequently dried over $MgSO_4$. Filtration and concentration in vacuo furnished dimethyl 2-((5R,9R)-5,9,13-trimethyltetradecanoyl)malonate (17.2 g, 45.4 mmol, 97% yield) as red-brown oil (pure according to NMR).
**$R_f$** ($SiO_2$, cyclohexane/EtOAc 5:1) = 0.26
**FT-IR** (ATR) [$cm^{-1}$] = 2950 (s), 2923 (s), 2865 (s), 1760 (m), 1723 (s), 1650 (s), 1596 (s), 1442 (s), 1372 (s), 1240 (s), 1192 (s), 1150 (m), 1085 (s), 1038 (s), 953 (m), 918 (m), 858 (br, m), 802 (m), 775 (m).
**$^1$H NMR** (500 MHz, $CDCl_3$): δ 13.42 (s) and 4.50 (s) add up to 1H, 3.80-3.77 (m, 6H), 2.59 (td, J = 13.3, 6.7 Hz) and 2.46-2.37 (m) add up to 2H, 1.75-1.56 (m, 2H), 1.55-1.48 (m, 1H), 1.41-1.03 (m, 16H), 0.87-0.83 (m, 12H) ppm.
**$^{13}$C NMR** (125 MHz, CDCl3): δ 198.6, 183.7, 171.5, 166.4, 164.9, 99.0, 64.8, 53.0, 52.1, 52.0, 42.3, 39.3, 37.3, 37.2, 32.1, 36.5, 36.1, 34.2, 32.7, 32.6, 32.4, 27.9, 24.7, 24.3, 24.2, 22.6, 22.5, 20.9, 19.7, 19.4 ppm.
**HRMS (ESI)** calcd. for $C_{22}H_{44}O_5Na$ $(M+Na)^+$ 407.2767955, found 407.27646
**$[\alpha]_{365}^{20}$ =** -1.8, $[\alpha]_{405}^{20}$ = -1.2, $[\alpha]_{546}^{20}$ = -0.7

Preparation of dimethyl 2-((5R,9R)-5,9,13-trimethyltetradecanoyl)malonate from (5R,9R)-5,9,13-trimethyltetradecanoic acid ①

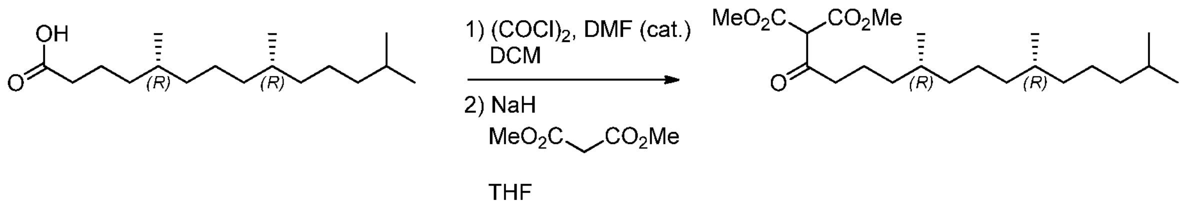


(5*R*,9R)-5,9,13-Trimethyltetradecanoic acid (10.0 g, 37.0 mmol, 1.0 eq.) was dissolved in $CH_2Cl_2$ (150 mL). Two drops of DMF (dimethylformamide) were added followed by dropwise addition of oxalyl chloride (3.8 mL, 44.3 mmol, 1.2 eq.) at 0 °C. The reaction was allowed to warm to room temperature and was stirred overnight. Excess reagent and solvent were removed in vacuo, furnishing (5*R*,9*R*)-5,9,13-trimethyltetradecanoyl chloride as yellow oily residue, which was used without further purification in the next step.

Dimethyl carbonate (4.65 mL, 40.7 mmol, 1.1 eq.) was added slowly to a suspension of NaH (3.25 g, 81.4 mmol, 2.0 eq.) in THF (300 mL) at 0 °C and subsequently stirred for an additional 30 min at 0 °C. The (5*R*,9*R*)-5,9,13-trimethyltetradecanoyl chloride obtained above was dissolved in THF (100 mL) and added dropwise to the reaction mixture at 0 °C. The reaction was stirred for 5 h at room temperature and subsequently quenched with 2N HCl at 0 °C. The reaction mixture was extracted with *tert*.-butyl methyl ether (TBME) and the combined organic phases were washed with brine, then dried over $MgSO_4$, filtered and concentrated in vacuo. The residue was purified by column chromatography (silica gel, cyclohexane/EtOAc 40:1 furnishing dimethyl 2-((5R,9R)-5,9,13-trimethyltetradecanoyl)malonate (13.11 g, 34.1 mmol, 92% yield) as pale yellow oil.

The analytical data is in accordance with the data shown above.

Preparation of dimethyl 2-((5R,9R)-1-(4-methoxy-2,3,5-trimethylphenoxy)-5,9,13-trimethyltetradecylidene)malonate and dimethyl 2-((5R,9R)-1-(4-methoxy-2,3,5-trimethylphenoxy)-5,9,13-trimethyltetradec-1-en-1-yl)malonate (③)

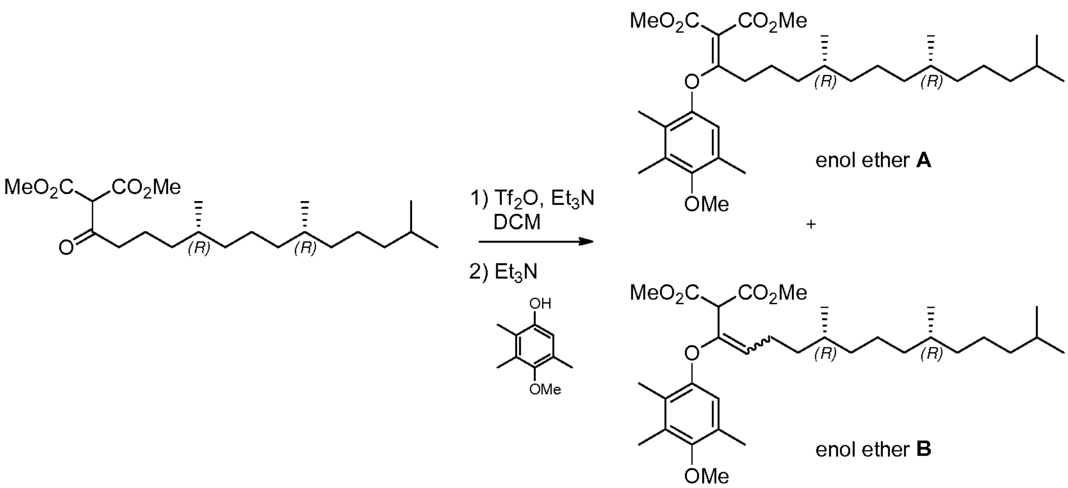


Dimethyl 2-((5R,9R)-5,9,13-trimethyltetradecanoyl)malonate (10.0 g, 26.00 mmol, 1.0 eq.) was dissolved in $CH_2Cl_2$ (80 mL) and treated with $Et_3N$ (triethylamine) (4.32 mL, 31.2 mmol, 1.2 eq.). $Tf_2O$ (triflic anhydride)(4.95 mL, 31.2 mmol, 1.2 eq.) was added slowly at 0 °C, the bright brown solution was allowed to warm to room temperature and stirred for 3 h. The reaction was cooled back to 0 °C and additional $Et_3N$ (4.32 mL, 31.2 mmol, eq.) was added, followed by portion-wise addition of 4-methoxy-2,3,5-trimethylphenol (4.32 g, 26.0 mmol, 1.0 eq.). The dark brown solution was then stirred for 5 d at room temperature. The mixture was diluted with $CH_2Cl_2$ and subsequently washed with 1 N HCl (2 x), $NaHCO_3$ solution (5%) and brine. The organic phase was dried over $MgSO_4$ and concentrated in vacuo. The residue was purified by column chromatography (siliga gel, cyclohexane/EtOAc 15:1 furnishing a mixture of dimethyl

2-((5*R*,9*R*)-1-(4-methoxy-2,3,5-trimethylphenoxy)-5,9,13-trimethyltetradecylidene)malonate and dimethyl 2-((5*R*,9*R*)-1-(4-methoxy-2,3,5-trimethylphenoxy)-5,9,13-trimethyltetradec-1-en-1-yl)malonate (11.04 g, 80%, 1:1 by NMR) as pale yellow oil.
**R$_f$** ($SiO_2$, cyclohexane/EtOAc 5:1) = 0.47
**FT-IR** (ATR) [$cm^{-1}$] = 2948 (s), 2922 (s), 2865 (s), 1738 (s), 1718 (s), 1669 (w), 1628 (m), 1576 (w), 1474 (m), 1459 (m), 1432 (m), 1400 (m), 1376 (m), 1364 (m), 1307 (s), 1254 (s), 1227 (s),1202 (ss), 1147 (s), 1087 (ss), 1059 (s), 1028 (s), 1004 (s), 946 (w), 908 (m), 871 (m), 825 (w), 796 (w), 755 (m), 731 (s).
**$^1$H NMR** (300 MHz, $CDCl_3$): δ 6.69 (s, enol ether **A)** and 6.63 (s, enol ether **B)** add up to 1 H, 4.61 (s, 0.5H, enol ether **B);** 4.41 (t, $^3J$= 7.7 Hz, 0.5H, enol ether **B),** 3.82/3.75/3.72 (s each, add up to 6H), 3.665/3.660 (s each, 3H), 2.61 (t, $^3J$= 7.9 Hz, 1 H, enol ether **A),** 2.23/2.20/2.10/2.05 (s each, add up to 9H), 1.98-1.88 (m, 1H, enol ether **B),** 1.59-0.97 (m, 18H), 0.88-0.74 (m, 12H) ppm.
**$^{13}$C NMR** (75 MHz, $CDCl_3$): δ 173.2, 167.3/166.2/164.6, 153.8/153.4, 147.7, 147.3, 147.2, 131.0/130.5/128.71/128.67/128.3/127.2, 120.8/119.8, 110.2, 106.8, 59.8, 52.6, 52.6/52.1/51.6, 39.2/37.3/37.1/36.5, 32.7/32.1, 28.9, 27.8, 24.8/24.7/24.3/24.3/24.1, 22.6/22.5/19.6/19.4/19.3, 15.8/12.5/12.4/12.0 ppm.
**HRMS** calcd. for $C_{32}H_{52}O_6Na$ $(M+Na)^+$ 555.3656105, found 555.36476.
$[\alpha]_D^{20}$ = -2.1, $[\alpha]_{546}^{20}$ = -2.4, $[\alpha]_{405}^{20}$ = -6.1

Preparation of methyl 6-methoxy-5,7,8-trimethyl-4-oxo-2-((4R,8R)-4,8,12-trimethyltridecyl)-4H-chromene-3-carboxylate (④)

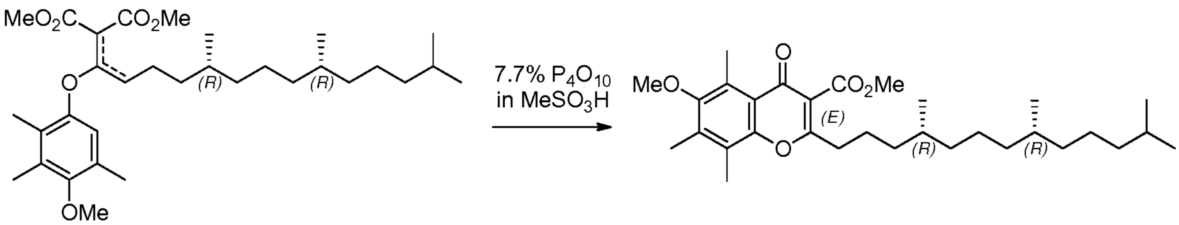


A mixture of dimethyl 2-((5*R*,9*R*)-1-(4-methoxy-2,3,5-trimethylphenoxy)-5,9,13-trimethyltetradecylidene)¬malonate and dimethyl 2-((5*R*,9*R*)-1-(4-methoxy-2,3,5-trimethylphenoxy)-5,9,13-trimethyltetradec-1-en-1-yl)malonate (1.00 g, 1.88 mmol, 1.0 eq.) was dissolved in Eaton's reagent (3 mL of 7.7% $P_4O_{10}$ in $MeSO_3H$ and the bright brown solution was stirred at 55 °C for 1 h. The reaction mixture was poured into ice-water and the flask was rinsed with EtOAc (ethyl acetate)and ice-water. The aqueous phase was extracted with EtOAc (3 x) and the combined organic phases were washed with $NaHCO_3$ (5%) and brine. The organic phase was dried over $MgSO_4$ filtered and concentrated in vacuo. The residue was purified by column chromatography (silica gel, cyclohexane/EtOAc 15:1), furnishing methyl 6-methoxy-5,7,8-trimethyl-4-oxo-2-((4*R*,8*R*)-4,8,12-trimethyltridecyl)-4*H*-chromene-3-carboxylate (626 mg, 67% yield) as bright yellow oil.
**R$_f$** ($SiO_2$, cyclohexane/EtOAc 5:1) = 0.34
**FT-IR** (ATR) [$cm^{-1}$] = 2948 (s), 2923 (s), 2865 (s), 1732 (s), 1642 (ss), 1567 (w), 1458 (s), 1433 (m), 1384 (s), 1325 (m), 1272 (m), 1192 (m), 1159 (m), 1095 (s), 1054 (m), 1037 (m), 1000 (m), 963 (w), 878 (w), 808 (w), 792 (w), 735 (w).
**$^1$H NMR** (300 MHz, $CDCl_3$): δ 3.92 (s, 3H), 3.67 (s, 3H), 2.76 (s, 3H), 2.70 (td, *J* = 7.5, 1.4 Hz, 2H), 2.35 (s, 6H), 1.85-1.56 (m, 2H), 1.56-1.06 (m, 17H), 0.88-0.82 (m, 12H) ppm. **$^{13}$C NMR** (75 MHz, $CDCl_3$): δ 177.2, 167.2, 166.2, 153.9, 151.7, 137.1, 129.5, 123.9, 120.2, 118.2, 60.3, 52.5, 39.3/37.4/37.3/37.2/36.3, 32.9, 32.8/32.5/28.0, 24.8/24.7/24.4, 22.7/22.6/19.7/19.5, 13.8/13.5/12.0 ppm.
**HRMS** calcd. for $C_{31}H_{49}O_5$ $(M+H)^+$ 501.3574511, found 501.35753; calcd. for $C_{31}H_{48}O_5Na$ $(M+Na)^+$ 523.3393957, found 523.33899.
$[\alpha]_{589}^{20}$ = -1.3, $[\alpha]_{546}^{20}$ = -1.9, $[\alpha]_{405}^{20}$ = -4.4

Preparation of (2R)-methyl 6-methoxy-2,5,7,8-tetramethyl-4-oxo-2-((4R,8R)-4,8,12-trimethyltridecyl)chroman-3-carboxylate (⑤)

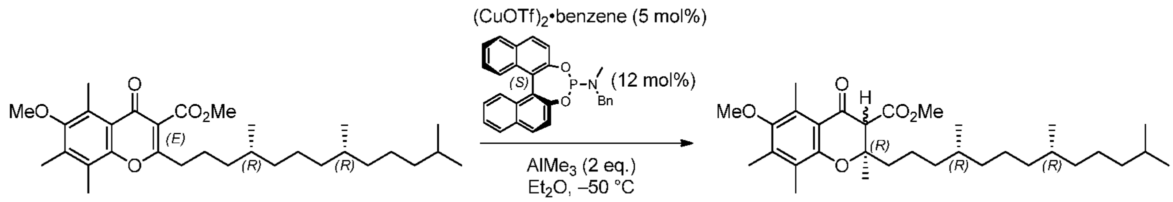


$(CuOTf)_2$·benzene (50.6 mg, 100 μmol, 5 mol%) and ($S_a$)-*N*-benzyl-*N*-methyldinaphtho[2,1-*d*:1',2'-*f*][1,3,2]dioxaphosphepin-4-amine (109.6 mg, 240 μmol, 12 mol%) were dissolved in $Et_2O$ (16 mL) under argon and stirred at room temperature for 1 h. The suspension was cooled to -30 °C and treated dropwise with $AlMe_3$ (4.0 mL of a 1 M solution in heptane, 4.0 mmol, 2.0 eq.). The yellow solution was stirred for 15 min at this temperature and then cooled to -50 °C. Subsequently, a solution of 6-methoxy-5,7,8-trimethyl-4-oxo-2-((4*R*,8*R*)-4,8,12-trimethyltridecyl)-4*H*-chromene-3-carboxylate (1.0 g, 2.0 mmol, 1.0 eq.) in $Et_2O$ (8 mL) was added dropwise. The reaction was stirred at -50 °C for 18 h and then quenched with 2 N HCl. The reaction mixture was extracted with EtOAc (2 x) and the combined organic phases were washed with aq. $NaHCO_3$ solution (5%) and brine. The organic phase was dried over $MgSO_4$ filtered and concentrated in vacuo. The residue was purified by column chromatography (silica gel, cyclohexane/EtOAc 30:1 furnishing (2R)-methyl 6-methoxy-2,5,7,8-tetramethyl-4-oxo-2-((4*R*,8*R*)-4,8,12-trimethyltridecyl)-chroman-3-carboxylate (948 mg, 92% yield) as yellow oil. The diastereoselectivity could not be determined at this point. Therefore the product was decarboxylated in the next step, at which point the diastereoselectivity was determined.
**$R_f$** ($SiO_2$, cyclohexane/EtOAc 5:1) = 0.58
**FT-IR** (ATR) [$cm^{-1}$] = 2948 (s), 2923 (s), 2865 (m), 2357 (w), 1747 (s), 1681 (s), 1588 (m), 1558 (w), 1538 (w), 1520 (w), 1505 (w), 1453 (ss), 1434 (s), 1391 (s), 1377 (s), 1339 (s), 1321 (m), 1286 (s), 1270 (s), 1203 (m), 1172 (m), 1130 (m), 1091 (s), 1044 (m), 1027 (m), 1004 (m), 926 (w), 907 (w), 879 (w), 804 (w), 773 (w), 759 (w), 734 (w), 667 (w).
**$^1$H NMR** (500 MHz, $CDCl_3$): δ 3.83 (s, 0.65H), 3.78 (s, 2H), 3.75 (s, 1H), 3.73 (s, 0.35H), 3.63 and 3.62 (s each, add up to 3H), 2.54 (s, 3H), 2.25 (s, 3H), 2.13 and 2.12 (s each, add up to 3H), 1.90-1.80 (m, 0.7H), 1.77-1.64 (m, 1.3H), 1.56-0.98 (m, 22H), 0.87-0.81 (m, 12H) ppm. Additionally, a second set of low intensity signals was observed, which could be assigned to the enol tautomer of the product: (500 MHz, $CDCl_3$): δ 13.45 (s), 3.81 (s), 2.51 (s), 2.21 (s), 2.09 (s).
**$^{13}$C NMR** (125 MHz, $CDCl_3$): δ 190.2, 189.9, 168.4, 168.3, 154.3, 154.1, 150.82, 150.75, 139.0, 138.9, 130.1, 130.0, 124.2, 124.1, 116.8, 116.6, 81.2, 81.1, 63.8, 62.1, 60.35, 60.33, 52.1, 39.9, 39.3/37.4/37.3/37.2/36.9/24.8/24.3/20.5/20.4, 34.5, 32.8/32.5, 27.9, 23.6, 22.7/22.6/19.71/19.70/19.6/19.5, 20.1, 13.80/13.7/13.63/13.60, 11.95/11.89 ppm.
**HRMS** calcd. for $C_{32}H_{52}O_5Na$ 539.3707, found 539.3705

Preparation of (R)-6-methoxy-2,5,7,8-tetramethyl-2-((4R,8R)-4,8,12-trimethyltridecyl)chroman-4-one(⑤)

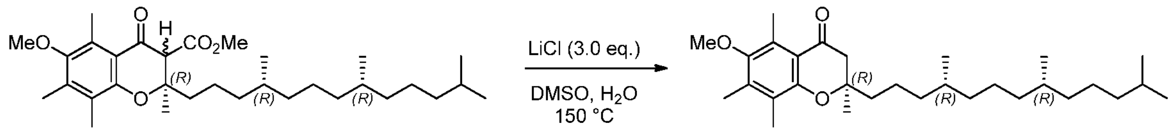


(2R)-Methyl 6-methoxy-2,5,7,8-tetramethyl-4-oxo-2-((4*R*,8*R*)-4,8,12-trimethyltridecyl)chroman-3-carboxylate (500 mg, 0.967 mmol, 1.0 eq.) was dissolved in DMSO (5 mL). LiCl (123 mg, 2.90 mmol, 3.0 eq.) and water (0.6 mL, 33 mmol, 35 eq.) were added. The reaction was heated to 150 °C for 5 h, then cooled to room temperature and treated with 0.1 N HCl and EtOAc. The organic phase was separated, the aqueous phase was extracted with EtOAc (2 x) and the combined organic phases were washed with brine. The organic phase was dried over $MgSO_4$ filtered and concentrated in vacuo. The residue was purified by column chromatography (silica gel, cyclohexane/$Et_2O$ 50:1), furnishing (*R*)-6-methoxy-2,5,7,8-tetramethyl-2-((4*R*,8*R*)-4,8,12-trimethyltridecyl)chroman-4-one (391 mg, 90% yield) as yellow oil.
The ratio of epimers at C2 was determined by HPLC analysis (chiral phase Daicel Chiralcel® OD-H, n-hexane/EtOH 99.85:0.15, flow 1.0 mL/min): (*2R,4'R,8'R*) : (*2S,4'R,8'R*) = 96.5 : 3.5, corresponding to a *ie* of 93%.
**$R_f$** ($SiO_2$, cyclohexane/EtOAc 5:1) = 0.68
**$^1$H NMR** (300 MHz, $CDCl_3$): δ 3.62 (s, 3H), 2.65 ($m_c$, 2H), 2.55 (s, 3H), 2.25 (s, 3H), 2.12 (s, 3H), 1.78-0.94 (m, 24H), 0.87-0.82 (m, 12H) ppm.
**$^{13}$C NMR** (75 MHz, $CDCl_3$): δ 194.8, 154.9, 150.4, 138.2, 129.5, 124.1, 117.1, 79.7, 60.4, 49.2, 39.8, 39.7, 39.3, 37.4, 37.3, 37.1, 32.8, 32.6, 28.0, 24.8, 24.4, 23.7, 22.7, 22.6, 19.7, 19.6, 13.9, 13.6, 12.0 ppm.
**HRMS** calcd. for $C_{30}H_{50}O_3$ 481.3652, found 481.3652

$[\alpha]_D^{20}$ = -4.3

Preparation of (2R,4'R,8'R)-α-tocopheryl methyl ether (⑧)

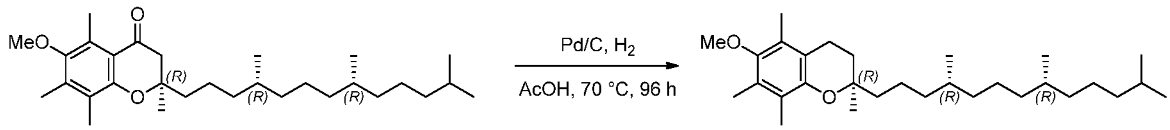


(*R*)-6-Methoxy-2,5,7,8-tetramethyl-2-((4*R*,8*R*)-4,8,12-trimethyltridecyl)chroman-4-one (160 mg, 0.349 mmol, 1.0 eq.) was dissolved in AcOH (acetic acid) (5 mL). The resulting solution was degassed several times. Pd/C (40 mg, 0.038 mmol, 10 wt%, 10.8 mol%) was added and the reaction was stirred at 70 °C for 96 h under an atmosphere of hydrogen (1 bar). The reaction was allowed to cool to room temperature and poured into a mixture of water and *tert.*-butyl methyl ether (TBME). The organic phase was washed with aq. $NaHCO_3$ (5%) and brine. Subsequently, the organic phase was filtered over Celite, dried over $MgSO_4$, filtered and concentrated in vacuo, furnishing (2*R*,4'*R*,8'*R*)-*α*-tocopheryl methyl ether (150 mg, 0.337 mmol, 97% yield) as pale yellow oil.
**$R_f$** ($SiO_2$, cyclohexane/EtOAc 5:1) = 0.66.
**$^1$H NMR** (300 MHz, $CDCl_3$): δ 3.62 (s, 3H), 2.57 (t, $^3J$= 6.8 Hz, 2H), 2.18 (s, 3H), 2.14 (s, 3H), 2.08 (s, 3H), 1.86-1.70 (m, 2H), 1.60-1.00 (m, 21 H), 1.23 (s, 3H), 0.88-0.83 (m, 12H) ppm. **$^{13}$C NMR** (75 MHz, $CDCl_3$): δ 149.4, 147.8, 127.7, 125.7, 122.9, 117.5, 74.8, 60.4, 40.1, 39.4, 37.5, 37.3, 32.8, 32.7, 31.3, 28.0, 24.8, 24.4, 23.9, 22.7, 22.6, 21.0, 20.6, 19.7, 12.5, 11.8, 11.7. The analytical data is in accordance with published data. Ref: J. Chapelat, A. Buss, A. Chougnet, W.-D. Woggon, Org. Lett. 2008, 10, 5123-5126.

Variation of chiral compound of formula (II-A or (11-B)

According to the procedure for the preparation of (R)-6-methoxy-2,5,7,8-tetramethyl-2-((4R,8R)-4,8,12-trimethyltridecyl)chroman-4-one respectively (2R)-methyl 6-methoxy-2,5,7,8-tetramethyl-4-oxo-2-((4R,8R)-4,8,12-trimethyltridecyl)chroman-3-carboxylate (⑤) as shown above other chiral compounds comprising a phosphorus atom have been used accordingly. Slightly different conditions were used in these cases: CuTC (10 mol%), ligand (20 mol%) in tetrahydrofuran. The isomeric excess (*ie*) has been measured and compiled in table 1.

Table 1. The influence of chiral compound comprising a phosphorus atom in the reaction for the preparation of (R)-6-methoxy-2,5,7,8-tetramethyl-2-((4R,8R)-4,8,12-trimethyltridecyl)chroman-4-one. * Conditions as described above in detail for the preparation of (2R)-methyl 6-methoxy-2,5,7,8-tetramethyl-4-oxo-2-((4R,8R)-4,8,12-trimethyltridecyl)-chroman-3-carboxylate.

| Example | *1* | *2* |
|---|---|---|
| Chiral compound | 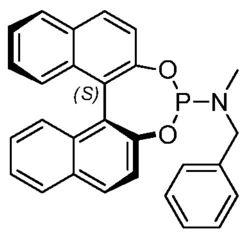 | 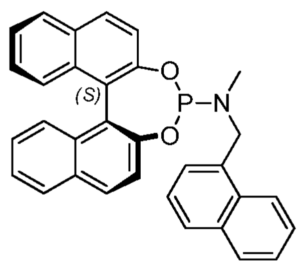 |
| *ie* (%) | 61 resp. 93* | 32 |
| | | |

(continued)

| Example | *3* | *4* |
|---|---|---|
| | 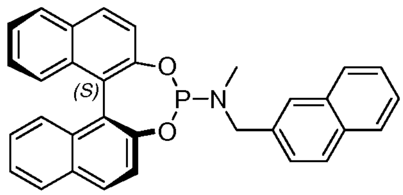 | 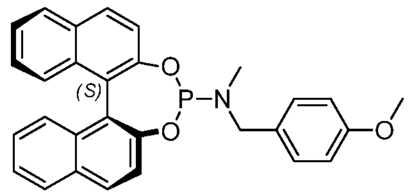 |
| *ie* (%) | 50 | 47 |

Preparation of (2*R*)-methyl 6-methoxy-2,5,7,8-tetramethyl-4-oxo-2-pentadecylchroman-3-carboxylate (⑤)

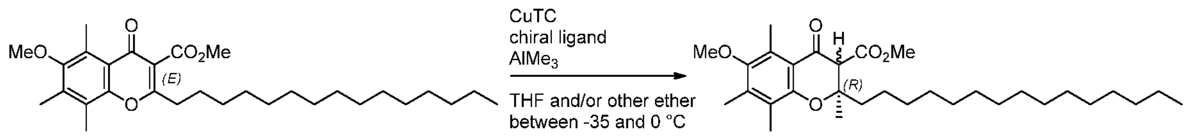


In a typical experiment, CuTC (10 mol%) and chiral ligand according to table 2 (20 mol%) were dissolved in THF (2 mL) and the reaction was stirred for 2 h at room temperature. After cooling to -30 °C, $AlMe_3$ (1 N in heptane, 2.0 eq.) was added slowly and the reaction was stirred for 15 min. A solution of methyl 6-methoxy-5,7,8-trimethyl-4-oxo-2-pentadecyl-4*H*-chromene-3-carboxylate (0.205 mmol, 1.0 eq.) in THF (2 mL) was added slowly and the reaction was stirred at this temperature for at least 18 h. The reaction was quenched with 1 N HCl, extracted with EtOAc and concentrated. The crude reaction product was used in the next step without purification.

The enantioselectivity could not be determined at this point. Therefore the product was converted to the chromanone in the next step, at which point the enantioselectivity was determined.

A pure sample was obtained after purification by column chromatography (silica gel, cyclohexane/EtOAc 20:1).
**$R_f$** ($SiO_2$ cyclohexane/EtOAc 5:1) = 0.60
**Mp.** 77.5 °C
**FT-IR** (ATR) [$cm^{-1}$] = 2920 (s), 2849 (s), 1746 (s), 1680 (s), 1588 (m), 1453 (s), 1391 (s), 1377 (m), 1340 (s), 1320 (m), 1286 (s), 1270 (s), 1204 (m), 1172 (m), 1129 (m), 1090 (s), 1043 (m), 1003 (m), 930 (w), 907 (w), 878 (w), 817 (w), 773 (w), 758 (w), 721 (w), 647 (w). **$^1$H NMR** (500 MHz, $CDCl_3$): δ 3.82 (s, 0.6H), 3.77 (s, ~2H), 3.75 (s, ~1H), 3.72 (s, 0.4H), 3.63 and 3.62 (s each, add up to 3H), 2.53 (s, 3H), 2.25 (s, 3H), 2.13 and 2.12 (s each, add up to 3H), 1.88-1.81 (m, 0.5H), 1.80-1.67 (m, 1.5H), 1.55-1.45 (br m, 2H), 1.47 (s, ~2H), 1.43 (s, ~1 H), 1.31-1.22 (m, 24H), 0.88 (t, $^3J$= 7.0 Hz, 3H) ppm.

Additionally, a second set of low intensity signals was observed, which could be assigned to the enol tautomer of the product: (500 MHz, $CDCl_3$): δ 13.45 (s), 3.81 (s), 2.51 (s), 2.24 (s), 2.08 (s) ppm.
**$^{13}$C NMR** (125 MHz, $CDCl_3$): δ 191.8, 191.2, 168.4, 168.3, 154.3, 154.1, 150.8, 150.7, 139.0, 138.9, 130.1, 130.0, 124.2, 124.1, 116.8, 116.6, 81.2, 81.0, 63.8, 62.1, 60.33, 60.32, 52.1, 39.9, 34.2, 31.9/29.66/29.65/29.63/29.62/29.59/29.58/29.47/29.42/29.40/29.3/23.2/23.1 /23.0/22.7, 23.5, 20.1, 14.1, 13.8/13.7/13.61/13.58, 11.94/11.89 ppm.
**HR-MS (ESI)** calcd. for $C_{31}H_{50}O_5$ 525.3350, found 525.3550

Preparation of (R)-6-methoxy-2,5,7,8-tetramethyl-2-pentadecylchroman-4-one (⑤)

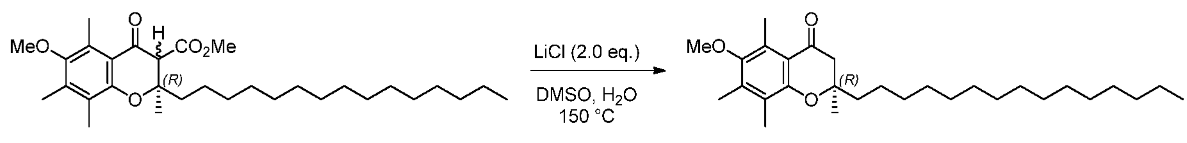


(2R)-Methyl 6-methoxy-2,5,7,8-tetramethyl-4-oxo-2-pentadecylchroman-3-carboxylate (50 mg, 0.1 mmol, 1.0

eq.) was suspended in DMSO (1 mL) in a GC vial, LiCl (9 mg, 0.2 mmol, 2.0 eq.) and a drop of water were added. The reaction was heated to 150 °C for 5 h, then cooled to room temperature and treated with 0.1 N HCl. The reaction was extracted with EtOAc (2 x), the combined organic phases were washed with brine and dried over $MgSO_4$. After filtration and concentration in vacuo, (*R*)-6-methoxy-2,5,7,8-tetramethyl-2-pentadecylchroman-4-one (44 mg, >98% yield) was obtained as a pale yellow solid.

**$R_f$** ($SiO_2$, cyclohexane/EtOAc 5:1) = 0.68

**Mp.** 60.3 °C

**FT-IR** (ATR) [$cm^{-1}$] = 2920 (s), 285 (s), 1736 (w), 1680 (s), 1587 (m), 1453 (s), 1390 (m), 1377 (m), 1289 (s), 1267 (m), 1166 (w), 1090 (s), 1048 (m), 1006 (m), 904 (w), 877 (w), 789 (w), 722 (w), 690 (w).

**$^1$H NMR** (500 MHz, $CDCl_3$): δ 3.60 (s, 3H), 2.63 ($m_c$, 2H), 2.53 (s, 3H), 2.22 (s, 3H), 2.10 (s, 3H), 1.72 (ddd, *J* = 13.8, 11.8, 4.8 Hz, 1H), 1.72 (ddd, *J* = 13.8, 11.8, 4.8 Hz, 1H), 1.43-1.34 (m, 2H), 1.33 (s, 3H), 1.31-1.23 (m, 24H), 0.86 (t, $^3J$= 7.0 Hz, 3H) ppm.

**$^{13}$C NMR** (125 MHz, $CDCl_3$): δ 194.8, 154.9, 150.4, 138.2, 129.5, 124.1, 117.1, 79.7, 60.3, 49.2, 39.5/31.9/29.69/29.67/29.65/29.63/29.53/29.50/29.4/23.5/22.7, 23.7, 14.1, 13.9, 13.6, 12.0 ppm.

**HRMS** calcd. for $C_{29}H_{48}O_3$ 467.3496, found 467.3495

**HPLC** analysis on a chiral stationary phase: Daicel Chiralpak AD-RH, MeOH, flow 0.4 mL/min, $t_r$(1)= 11.0 min, $t_r$(2) = 13.2 min.

Table 2. The influence of chiral compound comprising a phosphorus atom in the reaction for the preparation of (R)-6-methoxy-2,5,7,8-tetramethyl-2-pentadecylchroman-4-one. Ph=phenyl

| Example | ***Ref.1*** | ***Ref.2*** |
|---|---|---|
| Chiral compound | 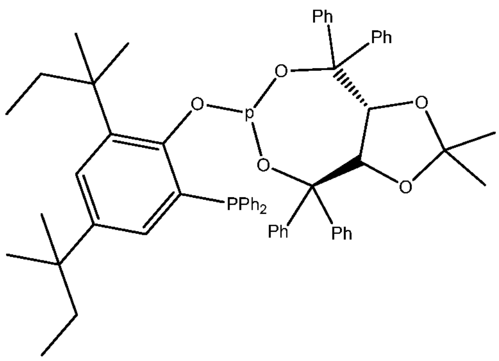 | 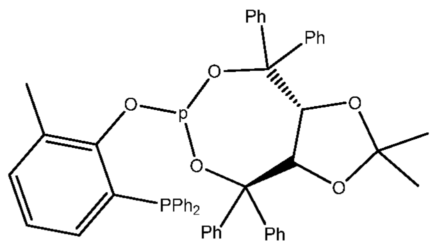 |
| *ie* (%) | 4 | <2 |
| | | |
| Example | ***Ref.3*** | ***Ref.4*** |
| Chiral compound | 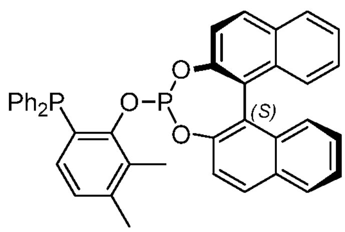 | 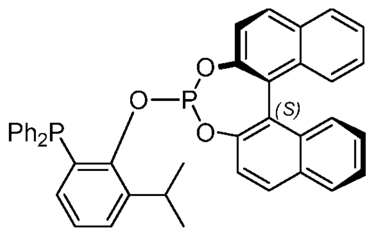 |
| *ie* (%) | no reaction | no reaction |
| | | |

(continued)

| Example | ***Ref.5*** | ***Ref.6*** |
|---|---|---|
| Chiral compound | 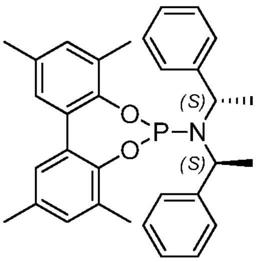 | 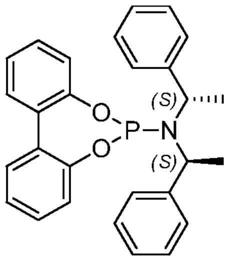 |
| *ie* (%) | <2 | no reaction |

According to the procedure shown above for the preparation of (R)-6-methoxy-2,5,7,8-tetramethyl-2-pentadecylchroman-4-one (⑤) other chiral compounds comprising a phosphorus atom have been used. The isomeric excess (*ie*) has been measured where a reaction has been observed to occur and compiled in table 2.

Table 2. The influence of chiral compound comprising a phosphorus atom in the reaction for the preparation of (R)-6-methoxy-2,5,7,8-tetramethyl-2-pentadecylchroman-4-one. Ph=phenyl

| Example | ***Ref.7*** | ***Ref.8*** |
|---|---|---|
| Chiral compound | 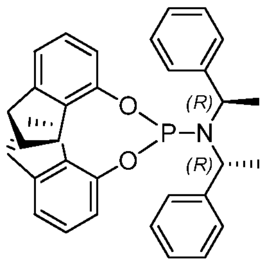 | 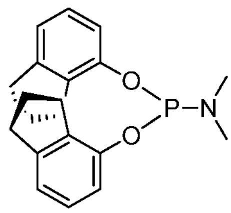 |
| *ie* (%) | no reaction | no reaction |
| | | |
| Example | ***Ref.9*** | ***Ref.10*** |
| Chiral compound | 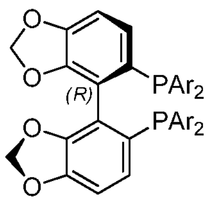 Ar = 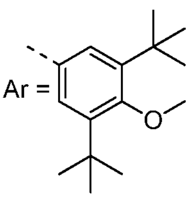 | 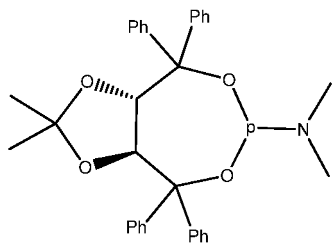 |
| *ie* (%) | <2 | no reaction |
| | | |
| Example | ***5*** | ***6*** |
| Chiral compound | 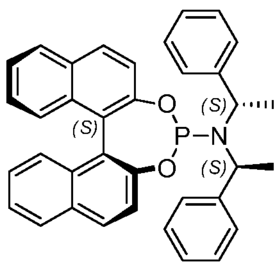 | 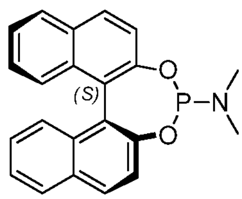 |

(continued)

| Example | ***5*** | ***6*** |
|---|---|---|
| *ie* (%) | 12 | 40 |
| Example | ***7*** | ***8*** |
| Chiral compound | 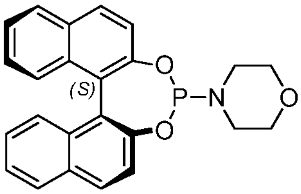 | 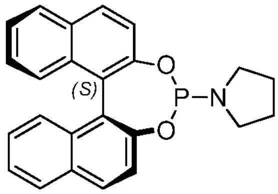 |
| *ie* (%) | 50 | 20 |
| | | |
| Example | ***9*** | ***10*** |
| | 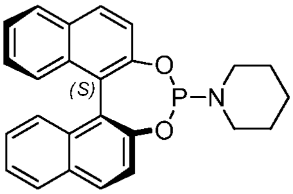 | 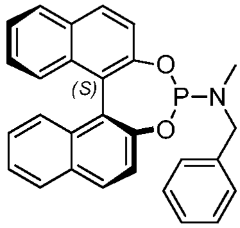 |
| *ie* (%) | 40 | 95 |
| | | |
| Example | ***11*** | ***12*** |
| | 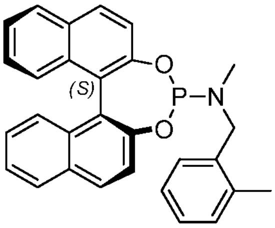 | 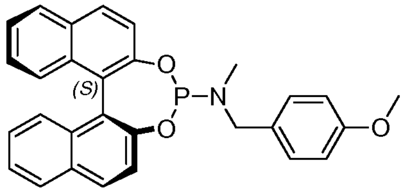 |
| *ie* (%) | 79 | 72 |
| | | |
| Example | ***13*** | ***14*** |
| | 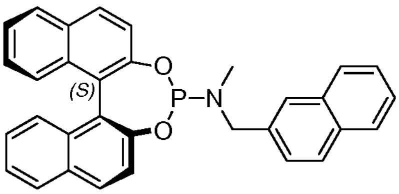 | 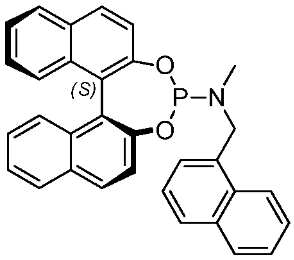 |
| *ie* (%) | 86 | 70 |

Preparation of 4-methoxy-2,3,5-trimethvlphenyl acetate (I)

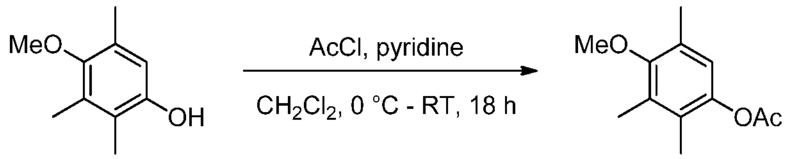


4-Methoxy-2,3,5-trimethylphenol (156.2 g, 0.847 mol, 90%, 1.0 eq.) was dissolved in $CH_2Cl_2$ (1.0 L) and pyridine (151.7 mL, 1.88 mol, 2.0 eq.) was added. The solution was cooled to 0 °C and acetyl chloride (87.0 mL, 1.22 mol, 1.3 eq.) was added dropwise. After stirring at rt over night the mixture was diluted with $CH_2Cl_2$, washed twice with 2N HCl, sat. $NaHCO_3$ and brine. After drying over $MgSO_4$ the solvent was removed under reduced pressure and the residue was purified by column chromatography (cyclohexane/EtOAc 10:1) to yield 174.0 g (0.836 mol, 99%) of 4-methoxy-2,3,5-trimethylphenyl acetate as a pale yellow oil.
**$R_f$** ($SiO_2$, cyclohexane/EtOAc 5:1) = 0.39

FT-IR (ATR) [$cm^{-1}$] = 2993 (w), 2935 (w), 2825 (w), 1754 (s), 1475 (m), 1454 (m), 1402 (m), 1366 (m), 1233 (m), 1198 (ss), 1094 (m), 1072 (s), 1007 (s), 899 (m), 855 (w), 816 (w), 754 (w), 724 (w).

**$^1$H NMR** (300 MHz, $CDCl_3$): δ 6.67 (s, 1H), 3.66 (s, 3H), 2.29 (s, 3H), 2.23 (s, 3H), 2.19 (s, 3H), 2.01 (s, 3H) ppm.

**$^{13}$C NMR** (125 MHz, $CDCl_3$): δ 169.7, 154.5, 144.8, 130.9, 128.9, 127.2, 121.1, 59.9, 20.8, 15.9, 12.74, 12.70 ppm.

**MS** (EI): m/z (%) = 208 ([$M^+$], 30), 166 (100), 151 (100), 123 (10), 107 (12), 91 (15).
**HRMS** calcd. for $C_{12}H_{16}O_3$ 208.1099, found 208.110.

Preparation of 1-(2-Hydroxy-5-methoxy-3,4,6-trimethylphenyl)ethanone (❷)
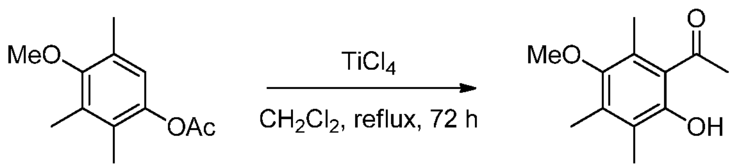


4-Methoxy-2,3,5-trimethylphenyl acetate (5.0 g, 24.0 mmol, 1.0 eq.) was dissolved in $CH_2Cl_2$ (25 mL) and $TiCl_4$ (5.28 mL, 48.0 mmol, 2.0 eq.) was gradually added at 0 °C. After refluxing for 72 h (the reaction can only be monitored by GC-MS) the whole mixture was poured onto ice-cold 2N HCl and thoroughly extracted three times with $CH_2Cl_2$. The combined organic extracts were washed with 5% $NaHCO_3$ and brine before drying over $MgSO_4$. The mixture was filtered over silica gel eluting with cyclohexane/EtOAc 10:1. Subsequently, the solvent was removed under reduced pressure to yield 4.21 g (20.22 mmol, 84%) of 1-(2-hydroxy-5-methoxy-3,4,6-trimethylphenyl)ethanone as pale yellow oil.
**$R_f$** ($SiO_2$ cyclohexane/EtOAc 5:1) = 0.39
**FT-IR** (ATR) [$cm^{-1}$] = 2984 (w), 2935 (m), 2829 (w), 1693 (w), 1613 (w), 1449 (m), 1416 (m), 1391 (s), 1355 (s), 1296 (ss), 1253 (s), 1227 (m), 1193 (m), 1165 (m), 1087 (s); 1046 (m), 1002 (s), 990 (s), 951 (m), 851 (m), 799 (m), 698 (w), 677 (m).
**$^1$H NMR** (300 MHz, $CDCl_3$): δ 12.28 (s, 1H), 3.60 (s, 3H), 2.60 (s, 3H), 2.44 (s, 3H), 2.22 (s, 3H), 2.12 (s, 3H) ppm.
**$^{13}$C NMR** (125 MHz, $CDCl_3$) : δ 205.9, 156.5, 149.4, 138.3, 127.5, 124.2, 119.7, 60.2, 33.0, 15.7, 13.5, 11.6 ppm.
**MS** (EI): m/z (%) = 208 ([$M^+$], 50), 193 (100), 179 (15), 147 (10), 135 (7), 119 (10), 91 (15), 77 (10), 67 (10).
**HRMS** calcd. for $C_{12}H_{16}O_3$ 208.1099, found 208.110

Preparation of ethyl 6-methoxy-5,7,8-trimethyl-4-oxo-4H-chromene-2-carboxylate (❸)

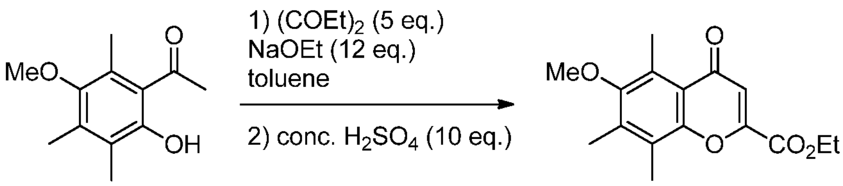

1-(2-Hydroxy-5-methoxy-3,4,6-trimethylphenyl)ethanone (3.12 g, 15 mmol, 1.0 eq.) and diethyl oxalate (10.15 mL, 75 mmol, 5.0 eq.) were dissolved in toluene (200 mL). A solution of NaOEt (12.25 g, 180 mmol, 12.0 eq.) in EtOH (80 mL) was added slowly via dropping funnel at room temperature. The mixture was stirred for 4 h at room temperature, after which full conversion was reached. Subsequently, conc. $H_2SO_4$ (8 mL, 150 mmol, 10.0 eq.) was added slowly and the reaction was heated to 80 °C for 3 h. After cooling to room temperature, water (approx. 150 mL) was added until the precipitate just dissolved completely. The aqueous phase was extracted with EtOAc (2 x), the combined organic phases were washed with brine, dried over $MgSO_4$ filtered and concentrated in vacuo. The residue was purified by column chromatography (silica gel, cyclohexane/EtOAc 5:1), furnishing ethyl 6-methoxy-5,7,8-trimethyl-4-oxo-4*H*-chromene-2-carboxylate (4.22 g, 97% yield) as yellow solid.
$\mathbf{R_f}$ ($SiO_2$, cyclohexane/EtOAc 5:1) = 0.68
**Mp.** 109 °C
**FT-IR** (ATR) [$cm^{-1}$] = 2982 (m), 2936 (m), 2840 (m), 1734 (s), 1645 (ss), 1630 (s), 1589 (m), 1457 (m), 1383 (m), 1322 (m), 1285 (s), 1239 (s), 1177 (s), 1091 (s), 1005 (s), 985 (s), 965 (m), 867 (m), 806 (m), 784 (m), 769 (m), 733 (w), 720 (w), 684 (w), 659 (w).
**$^1$H NMR** (300 MHz, $CDCl_3$): δ 6.96 (s, 1H), 4.45 (q, $^3J$= 7.1 Hz, 2H), 3.69 (s, 3H), 2.75 (s, 3H), 2.42 (s, 3H), 2.36 (s, 3H), 1.44 (t, $^3J$= 7.1 Hz, 3H) ppm.
**$^{13}$C NMR** (75 MHz, $CDCl_3$): δ 181.1, 160.7, 154.1, 152.0, 150.3, 138.1, 129.0, 125.1, 121.5, 114.9, 62.6, 60.3, 14.0, 13.8, 13.5, 12.1 ppm.
**HRMS (ESI)** calcd. for $C_{16}H_{18}O_5Na$ $(M+Na)^+$ 313.1046, found 313.1047

Preparation of (*S*)-ethyl 6-methoxy-2,5,7,8-tetramethyl-4-oxochroman-2-carboxylate (❹)
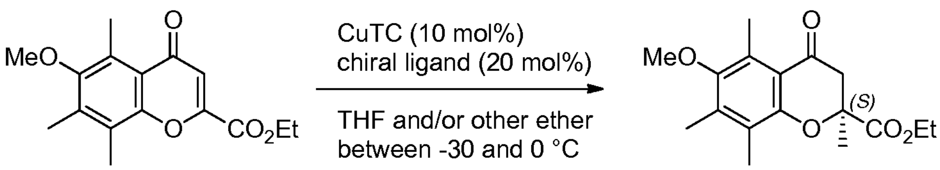


In a typical example, CuTC (10 mol%) and chiral compound (20 mol%) according to table 3 were dissolved in 2 mL THF (and/or other ethers) and stirred under argon at room temperature for 30 min. The mixture was cooled to a temperature between 0 °C and -30 °C, and $AlMe_3$ (0.52 mL of a 1 M solution in heptane, 0.52 mmol, 3.0 eq.) was slowly added dropwise. After 5 min, a solution of ethyl 6-methoxy-5,7,8-trimethyl-4-oxo-4*H*-chromene-2-carboxylate (0.172 mmol, 1.0 eq.) in THF (and/or other ethers) was slowly added dropwise. After stirring at this temperature for at least 24 h, the reaction was quenched with 1 N HCl and extracted with TBME (2 x). The combined organic phases were washed with brine, dried over $MgSO_4$ filtered and concentrated in vacuo. The enantiomer ratios were determined by analysis of the crude product by HPLC on a chiral stationary phase. The isomeric excess (*ie*) are reported in table 3. A purified sample was obtained after purification by column chromatography (silica gel, cyclohexane/EtOAc 6:1) as yellow oil.
$\mathbf{R_f}$ ($SiO_2$, cyclohexane/EtOAc 2:1) = 0.63
**FT-IR** (ATR) [$cm^{-1}$] = 2980 (w), 2920 (w), 2915 (w), 2847 (w), 1735 (s), 1681 (s), 1589 (m), 1453 (s), 1390 (m), 1370 (m), 1289 (s), 1263 (m), 1209 (m), 1188 (m), 1170 (m), 1123 (m), 1090 (s), 1036 (m), 1009 (m), 950 (w), 914 (w), 863 (w), 796 (s), 763 (m).
**$^1$H NMR** (300 MHz, $CDCl_3$): δ 4.11 (q, $^3J$= 7.1 Hz, 2H), 3.61 (s, 3H), 2.99 ($m_c$, 2H), 2.52 (s, 3H), 2.26 (s, 3H), 2.22 (s, 3H), 1.69 (s, 3H), 1.11 (t, $^3J$= 7.1 Hz, 3H) ppm.
**$^{13}$C NMR** (75 MHz, $CDCl_3$): δ 192.1, 172.1, 155.5, 151.3, 138.7, 130.1, 124.5, 117.5, 80.4, 61.6, 60.3, 47.1, 24.9, 13.94, 13.87, 13.6, 12.0 ppm.
**MS (EI DIP*/MS):** m/z (%) = 306 ([$M^+$], 47), 233 (100), 192 (40), 177 (47), 149 (27).
**HRMS (EI)** calcd. for $C_{17}H_{22}O_5$ 306.1467, found 306.148
*DIP = Direct Insertion Probe

Table 3. Variation of chiral compound in the preparation of (S)-ethyl 6-methoxy-2,5,7,8-tetramethyl-4-oxochroman-2-carboxylate.

| | ***15*** | ***16*** | ***17*** |
|---|---|---|---|
| Chiral compound | 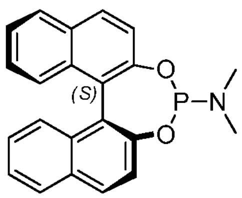 | 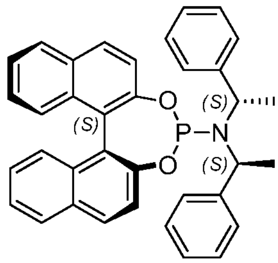 | 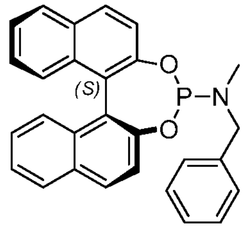 |
| *ie* (%) | 9 | 14 | 6 |

**Claims**

**1.** Method of preparing compound of formula (I-A) or (I-B)

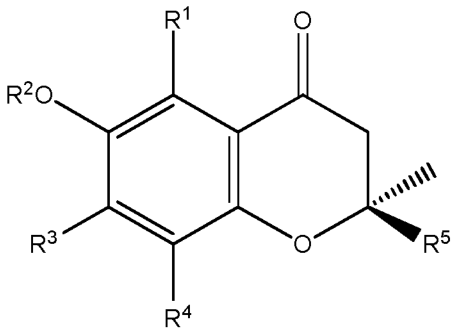

(I-A)

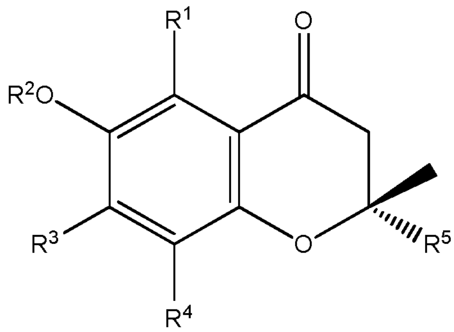

(I-B)

comprising the step of reacting compound of formula (XII)

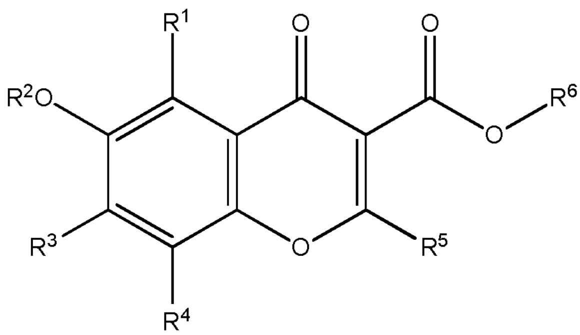

(XII)

with $Al(CH_3)_3$ in the presence of a Cu(I) salt and chiral compound of formula (II-A) or (II-B)

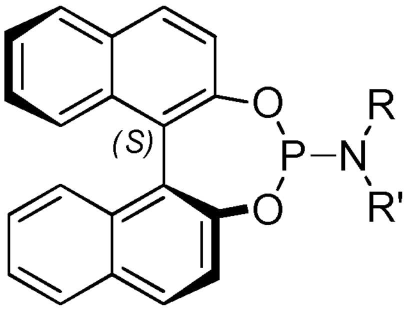

(II-A)

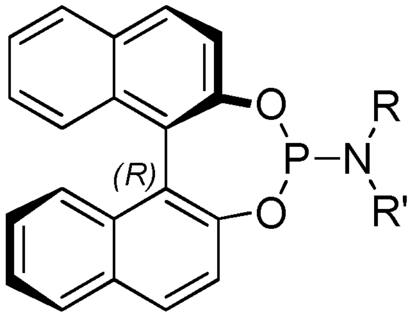

(II-B)

followed by a ester hydrolysis and decarboxylation by heating to a temperature of 100-190 °C, particularly 150-190°C;

wherein $R^5$ represents either a linear or branched completely saturated $C_{6-25}$-alkyl group or a linear or branched $C_{6-25}$-alkyl group comprising at least one carbon-carbon double bond;
$R^1$, $R^3$ and $R^4$ are independently from each other hydrogen or methyl groups;
$R^2$ represents hydrogen or a phenol protecting group;
R' and R are independently from each other a hydrocarbyl group with 1 to 12 carbon atoms, which optionally is substituted by $OR^6$, or form together a $C_{4-6}$-alkylene group which optionally comprises a functional group which is selected from the group consisting of

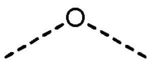, 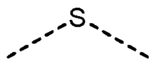, 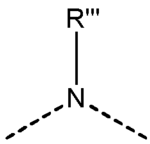 and 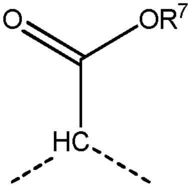

wherein R''' and $R^7$ are independently from each other a $C_{1-4}$-alkyl group;
and $R^6$ is a methyl or ethyl group or *tert.*-butyl group;

with the proviso that for the preparation of compound (I-A) the compound (II-A) is used and that for the preparation of compound (I-B) the compound (II-B) is used.

2. Method according to claim 1 **characterized in that** the Cu(I) salt is a Cu(I) carboxylate or Cu(I) sulfonate, particularly Cu(I)-thiophene-2-carboxylate or Cu(I)-triflate.

3. Method according to claim 1 or 2 **characterized in that** R' and/or R is a $C_{1-6}$-alkyl group or an aryl or aralkyl group, particularly a benzyl group or a methyl group or a 1-phenylethyl group or a 4-methoxybenzyl or a 1-naphthylmethyl or 2-naphthylmethyl group.

4. Method according to anyone of the preceding claims 1-3 **characterized in that** R = R', particularly that R = R' = methyl group.

5. Method according to anyone of the preceding claims 1-4 **characterized in that** $R^5$ is of formula (III)

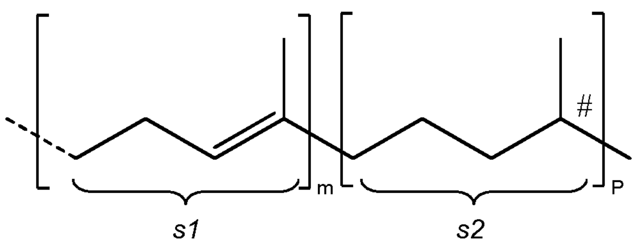

(III)

wherein m and p stand independently from each other for a value of 0 to 5 provided that the sum of m and p is 1 to 5, and where the substructures in formula (III) represented by *s1* and *s2* can be in any sequence;
and the dotted line represents the bond by which the substituent of formula (III) is bound to the rest of formula (I);
and wherein # represents a chiral centre, obviously except in case where said centre is linked to two methyl groups.

**6.** Method of preparing compound of formula (IV-A) or (IV-B)

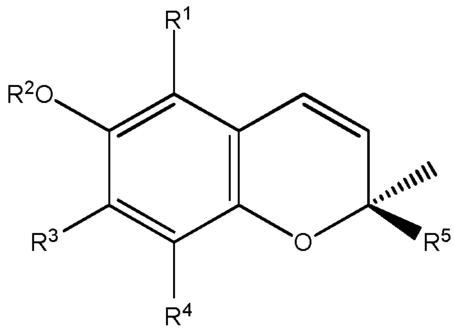

(IV-A)

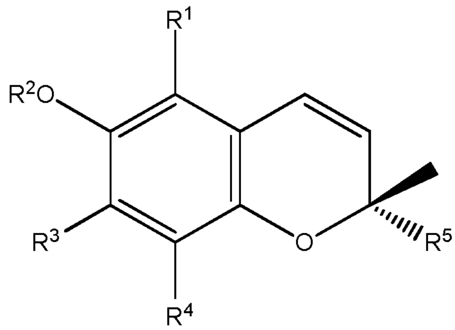

(IV-B)

comprising the steps

a) preparing compound of formula (I-A) or (I-B) according to a method according to anyone of the preceding claims 1 to 5;
b) reduction of compound of formula (I-A) or (I-B) by a hydride, particularly by sodium borohydride;
c) treatment by acid

with the proviso that when compound of formula (I-A) is used in step a) the compound of formula (IV-A) is formed and when compound of formula (I-B) is used in step a) the compound of formula (IV-B) is formed.

**7.** Method of preparing compound of formula (V-A) or (V-B)

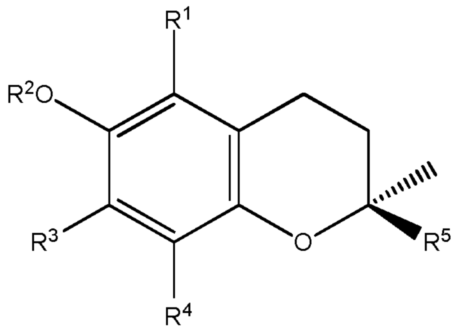

(V-A)

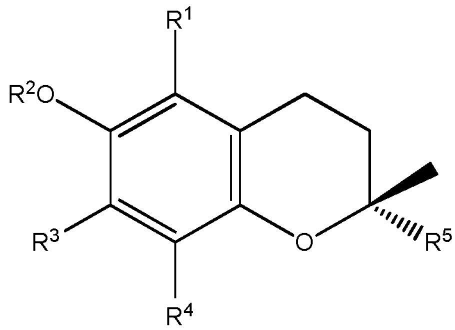

(V-B)

comprising the steps

a) preparing compound of formula (I-A) or (I-B) according to a method according to anyone of the preceding claims 1 to 5;
b') hydrogenation of compound of formula (I-A) or (I-B), particularly by means of molecular hydrogen in the presence of a Pd/C catalyst or by metallic zinc in the presence of an acid;

with the proviso that when compound of formula (I-A) is used in step a) the compound of formula (V-A) is formed and when compound of formula (I-B) is used in step a) the compound of formula (V-B) is formed

**8.** Compound of formula (X)

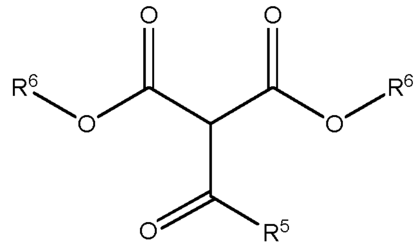

(X)

wherein $R^5$ represents either a linear or branched completely saturated $C_{6-25}$-alkyl group or a linear or branched $C_{6-25}$-alkyl group comprising at least one carbon-carbon double bond;
and $R^6$ is a methyl or ethyl or *tert*.-butyl group.

**9.** Compound of formula (XI)

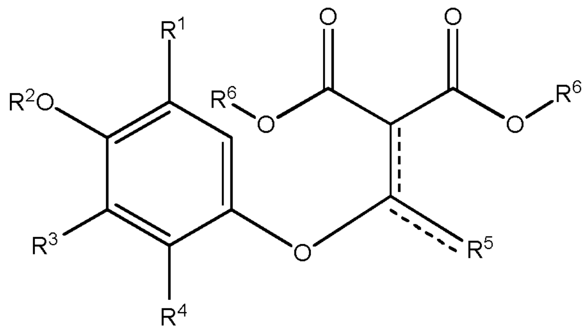

(XI)

wherein $R^5$ represents either a linear or branched completely saturated $C_{6-25}$-alkyl group or a linear or branched $C_{6-25}$-alkyl group comprising at least one carbon-carbon double bond;
$R^1$, $R^3$ and $R^4$ are independently from each other hydrogen or methyl groups;
$R^2$ represents hydrogen or a phenol protecting group;
and $R^6$ is a methyl or ethyl or *tert*.-butyl group;
and the dotted lines denotes the two alternative positions of one carbon-carbon double bond.

**10.** Compound of formula (XII)

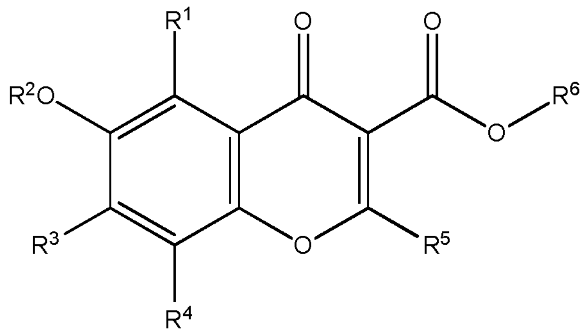

(XII)

wherein $R^5$ represents either a linear or branched completely saturated $C_{6-25}$-alkyl group or a linear or branched $C_{6-25}$-alkyl group comprising at least one carbon-carbon double bond;
wherein $R^1$, $R^3$ and $R^4$ are independently from each other hydrogen or methyl groups;
$R^2$ and $R^{2'}$ represents hydrogen or a phenol protecting group;
and $R^6$ is a methyl or ethyl or *tert*.-butyl group.

**11.** Compound of formula (X) or (XI) or (XII) according to claim 8 or 9 or 10 **characterized in that** $R^5$ is of formula (III)

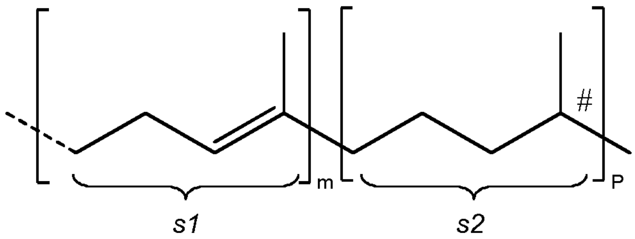

(III)

wherein m and p stand independently from each other for a value of 0 to 5 provided that the sum of m and p is 1 to 5, and where the substructures in formula (III) represented by *s1* and *s2* can be in any sequence;
and the dotted line represents the bond by which the substituent of formula (III) is bound to the rest of formula (X) or (XI) or (XII);
and wherein # represents a chiral centre, obviously except in case where said centre is linked to two methyl groups.

**12.** Method of preparing compound of formula (X)

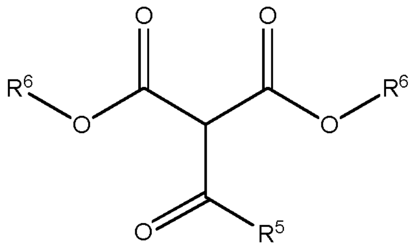

(X)

wherein $R^5$ represents either a linear or branched completely saturated $C_{6-25}$-alkyl group or a linear or branched $C_{6-25}$-alkyl group comprising at least one carbon-carbon double bond;
and $R^6$ is a methyl or ethyl or *tert*.-butyl group;
comprising the step of reacting compound of formula (X-a) with compound of formula (X-b)

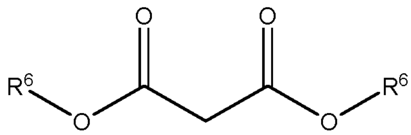

(X-a)

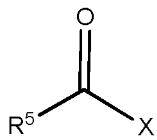

(X-b)

wherein X = Cl or Br or

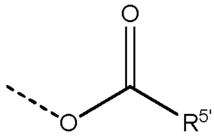
;

wherein $R^{5'}$ represents either a linear or branched completely saturated $C_{1-25}$-alkyl group or a linear or branched $C_{2-25}$-alkyl group comprising at least one carbon-carbon double bond; particularly $R^{5'}$;
in the presence of alkali metal hydride in an ether, particularly in tetrahydrofuran.

**13.** Method of preparing compound of formula (X)

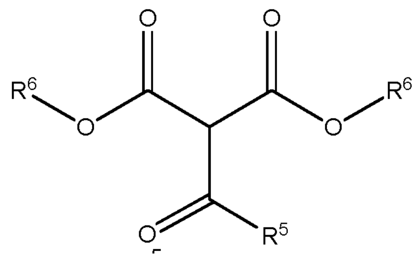 (X)

wherein $R^5$ represents either a linear or branched completely saturated $C_{6-25}$-alkyl group or a linear or branched $C_{6-25}$-alkyl group comprising at least one carbon-carbon double bond;
and $R^6$ is a methyl or ethyl or *tert*.-butyl group;

comprising the steps of

i) reacting compound of formula (X-a') with compound of formula (X-b')

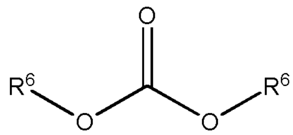 (X-a')

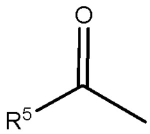 (X-b')

in the presence of alkali metal hydride in an ether, particularly in tetrahydrofuran;
ii) reacting with compound of formula (X-c')

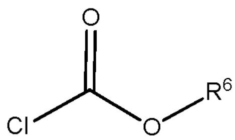 (X-c')

in the presence of a base, particularly pyridine, and a metal salt.

**14.** Method of preparing compound of formula (XI)

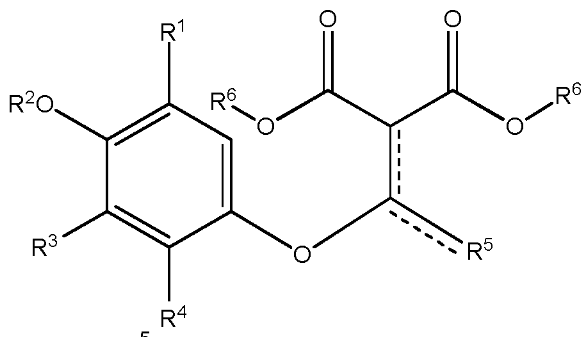 (XI)

wherein $R^5$ represents either a linear or branched completely saturated $C_{6-25}$-alkyl group or a linear or branched $C_{6-25}$-alkyl group comprising at least one carbon-carbon double bond;
$R^1$, $R^3$ and $R^4$ are independently from each other hydrogen or methyl groups;

$R^2$ represents hydrogen or a phenol protecting group;
and $R^6$ is a methyl or ethyl or *tert*.-butyl group;
and the dotted lines denotes the two alternative positions of one carbon-carbon double bond;

comprising the steps of

- reacting compound of formula (X) with sulfonyl halide or anhydride of an organic sulfonic acid and in the presence of a base, particularly in the presence of a tertiary amine; to form an enol sulfonate intermediate;
- reacting said enol sulfonate intermediate with compound of formula (XI-a) in the presence of a base, particularly in the presence of a tertiary amine

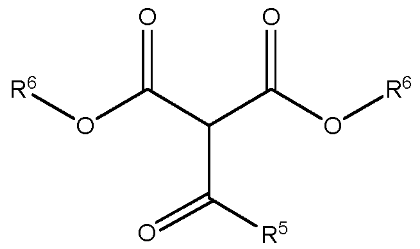

(X)

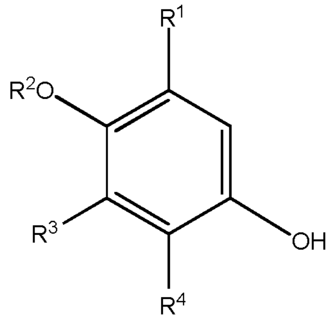

(XI-a).

**15.** Method of preparing compound of formula (XII)

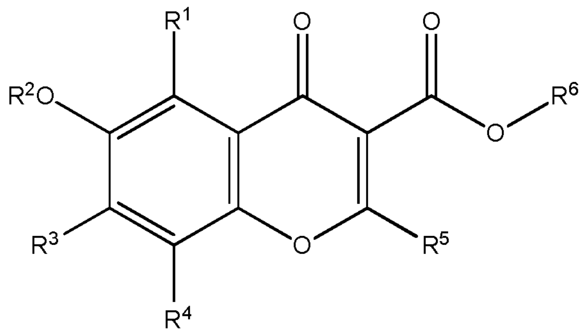

(XII)

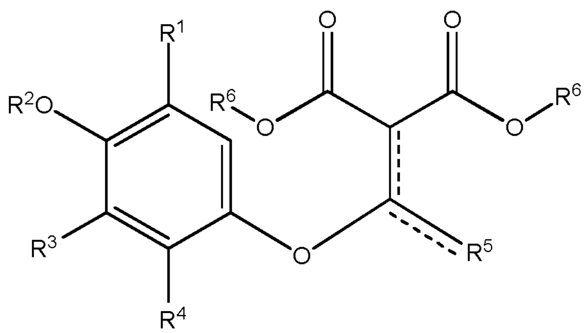

(XI).

wherein $R^5$ represents either a linear or branched completely saturated $C_{6-25}$-alkyl group or a linear or branched $C_{6-25}$-alkyl group comprising at least one carbon-carbon double bond;
$R^1$, $R^3$ and $R^4$ are independently from each other hydrogen or methyl groups;
$R^2$ represents hydrogen or a phenol protecting group;
and $R^6$ is a methyl or ethyl or *tert*.-butyl group;
and the dotted lines denotes the two alternative positions of one carbon-carbon double bond;
comprising an intramolecular reaction of formula (XI) in the presence of methanol binding agent in methanesulfonic acid.

**16.** Method of preparing compound of formula (XXI-A) or (XXI-B)

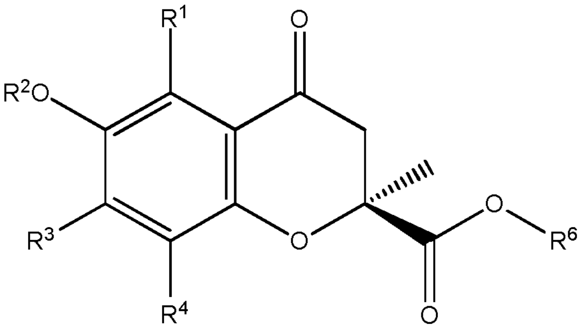
(XXI-A)

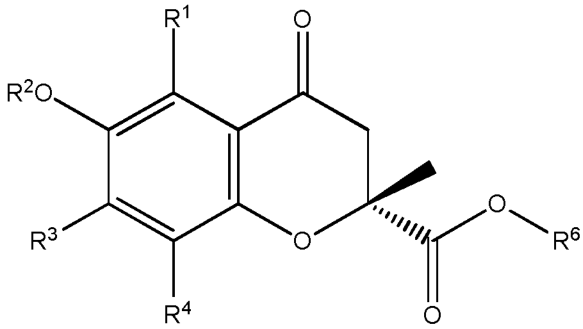
(XXI-B)

comprising the step of reacting compound of formula (XXII)

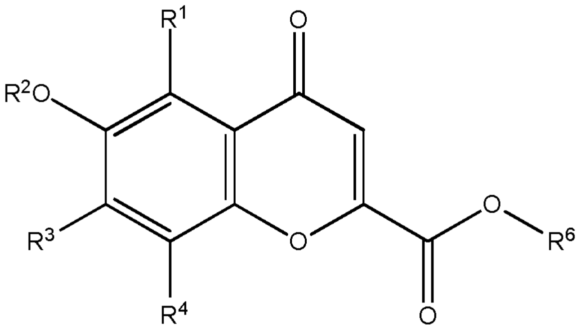
(XXII)

with $Al(CH_3)_3$ in the presence of a Cu(I) salt and chiral compound of formula (II-A) or (II-B)

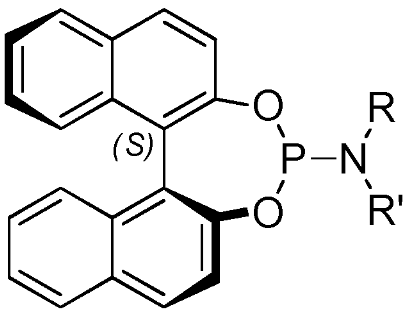
(II-A)

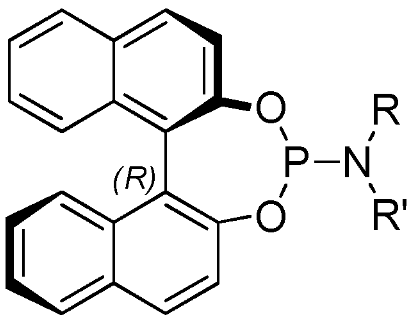
(II-B)

$R^1$, $R^3$ and $R^4$ are independently from each other hydrogen or methyl groups;
$R^2$ represents hydrogen or a phenol protecting group;
R' and R are independently from each other a hydrocarbyl group with 1 to 12 carbon atoms, which optionally is substituted by $OR^6$, or form together a $C_{4-6}$-alkylene group which optionally comprises a functional group

which is selected from the group consisting of

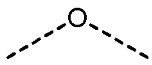 , 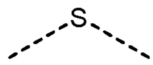 , 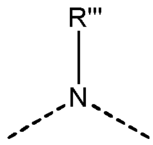 and 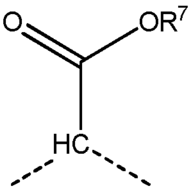

wherein R''' and $R^7$ are independently from each other a $C_{1-4}$-alkyl group;
and $R^6$ is a methyl or ethyl group or *tert*.-butyl group;

with the proviso that for the preparation of compound (XXI-A) the compound (II-A) is used and that for the preparation of compound (XXI-B) the compound (II-B) is used.

## Patentansprüche

**1.** Verfahren zur Herstellung einer Verbindung der Formel (I-A) oder (I-B)

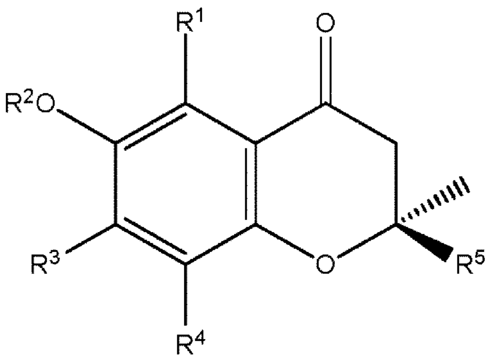

(I-A)

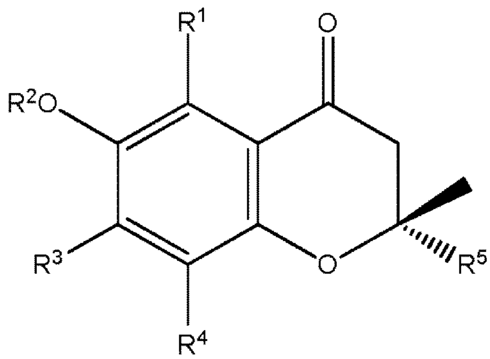

(I-B)

umfassend den Schritt des Umsetzens einer Verbindung der Formel (XII)

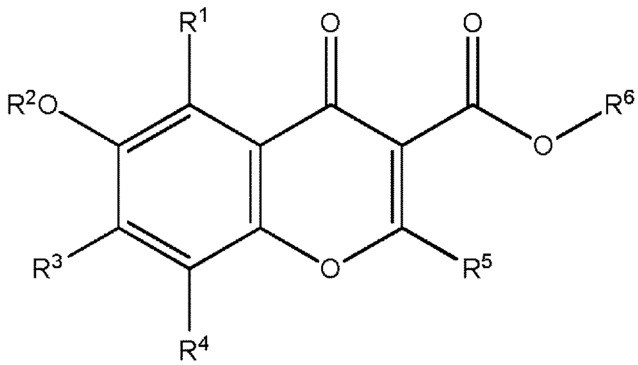

(XII)

mit $Al(CH_3)_3$ in Gegenwart eines Cu(I)-Salzes und einer chiralen Verbindung der Formel (II-A) oder (II-B)

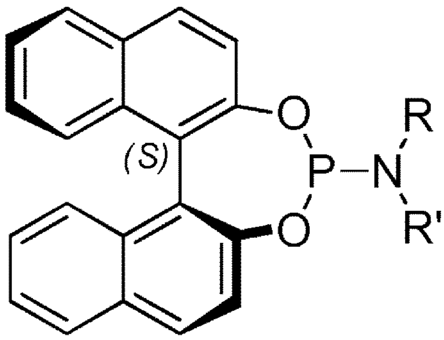 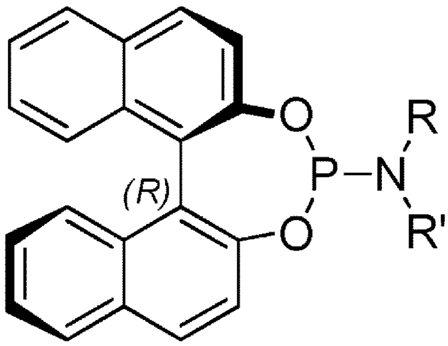

(II-A) (II-B)

mit nachfolgender Esterhydrolyse und Decarboxylierung durch Erhitzen auf eine Temperatur von 100-190°C, insbesondere 150-190°C;

wobei $R^5$ entweder für eine lineare oder verzweigte vollständig gesättigte $C_{6-25}$-Alkylgruppe oder für eine lineare oder verzweigte $C_{6-25}$-Alkylgruppe mit mindestens einer Kohlenstoff-Kohlenstoff-Doppelbindung steht;
$R^1$, $R^3$ und $R^4$ unabhängig voneinander für Wasserstoff oder Methylgruppen stehen;
$R^2$ für Wasserstoff oder eine Phenol-Schutzgruppe steht;
R' und R unabhängig voneinander für eine Hydrocarbylgruppe mit 1 bis 12 Kohlenstoffatomen, die gegebenenfalls durch $OR^6$ substituiert ist, stehen oder zusammen eine $C_{4-6}$-Alkylengruppe, die gegebenenfalls eine funktionelle Gruppe, die aus der Gruppe bestehend aus

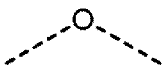, 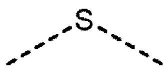, 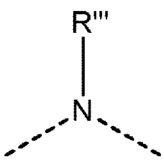 und 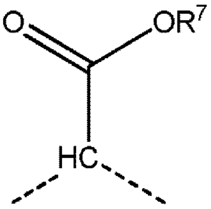

ausgewählt ist, bilden, wobei R"' und $R^7$ unabhängig voneinander für eine $C_{1-4}$-Alkylgruppe stehen und $R^6$ für eine Methyl- oder Ethylgruppe oder *tert.*-Butylgruppe steht; mit der Maßgabe, dass für die Herstellung von Verbindung (I-A) die Verbindung (II-A) verwendet wird und dass für die Herstellung von Verbindung (I-B) die Verbindung (II-B) verwendet wird.

2. Verfahren nach Anspruch 1, **dadurch gekennzeichnet, dass** es sich bei dem Cu(I)-Salz um ein Cu(I)-Carboxylat oder Cu(I)-Sulfonat, insbesondere Cu(I)-Thiophen-2-carboxylat oder Cu(I)-Triflat, handelt.

3. Verfahren nach Anspruch 1 oder 2, **dadurch gekennzeichnet, dass** R' und/oder R für eine $C_{1-6}$-Alkylgruppe oder eine Aryl- oder Aralkylgruppe, insbesondere eine Benzylgruppe oder eine Methylgruppe oder eine 1-Phenylethylgruppe oder eine 4-Methoxybenzyl- oder eine 1-Naphthylmethyl- oder 2-Naphthylmethylgruppe steht.

4. Verfahren nach einem der vorhergehenden Ansprüche 1-3, **dadurch gekennzeichnet, dass** R = R', insbesondere dass R = R' = Methylgruppe.

5. Verfahren nach einem der vorhergehenden Ansprüche 1-4, **dadurch gekennzeichnet, dass** $R^5$ die Formel (III)

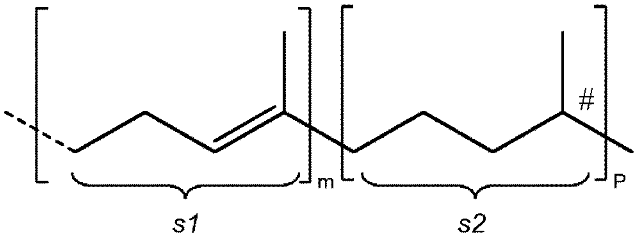

(III)

aufweist, wobei m und p unabhängig voneinander für einen Wert von 0 bis 5 stehen, mit der Maßgabe, dass die Summe von m und p gleich 1 bis 5 ist,
und wobei die durch *s1* und *s2* dargestellten Unterstrukturen in Formel (III) in beliebiger Reihenfolge vorliegen können;
und die gestrichelte Linie für die Bindung steht, über die der Substituent der Formel (III) an den Rest der Formel (I) gebunden ist; und wobei # für ein Chiralitätszentrum steht, offensichtlich mit Ausnahme des Falls, in dem das Zentrum mit zwei Methylgruppen verknüpft ist.

**6.** Verfahren zur Herstellung einer Verbindung der Formel (IV-A) oder (IV-B)

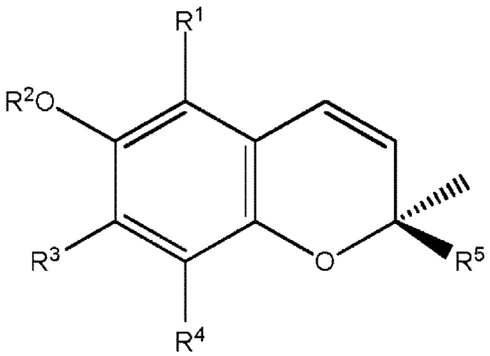

(IV-A)

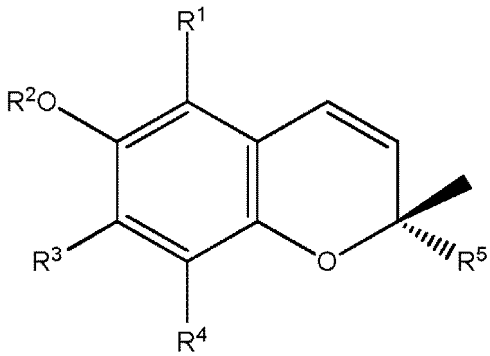

(IV-B)

das folgende Schritte umfasst:

a) Herstellen einer Verbindung der Formel (I-A) oder (I-B) gemäß einem Verfahren nach einem der vorhergehenden Ansprüche 1 bis 5;
b) Reduzieren der Verbindung der Formel (I-A) oder (I-B) mit einem Hydrid, insbesondere mit Natriumborhydrid;
c) Behandeln mit Säure;

mit der Maßgabe, dass dann, wenn in Schritt a) eine Verbindung der Formel (I-A) verwendet wird, die Verbindung der Formel (IV-A) gebildet wird, und dann, wenn in Schritt a) eine Verbindung der Formel (I-B) verwendet wird, die Verbindung der Formel (IV-B) gebildet wird.

**7.** Verfahren zur Herstellung einer Verbindung der Formel (V-A) oder (V-B)

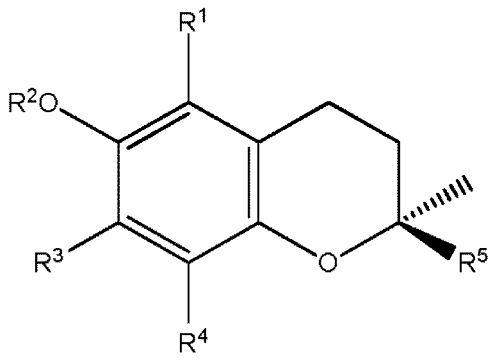

(V-A)

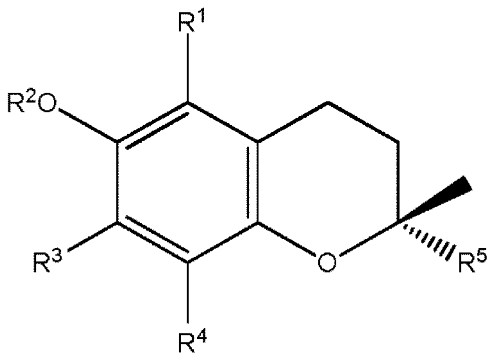

(V-B)

das folgende Schritte umfasst:

a) Herstellen einer Verbindung der Formel (I-A) oder (I-B) gemäß einem Verfahren nach einem der vorhergehenden Ansprüche 1 bis 5;
b') Hydrieren der Verbindung der Formel (I-A) oder (I-B), insbesondere mit molekularem Wasserstoff in Gegenwart eines Pd/C-Katalysators oder mit metallischem Zink in Gegenwart einer Säure;

mit der Maßgabe, dass dann, wenn in Schritt a) eine Verbindung der Formel (I-A) verwendet wird, die Verbindung der Formel (V-A) gebildet wird, und dann, wenn in Schritt a) eine Verbindung der Formel (I-B) verwendet wird, die Verbindung der Formel (V-B) gebildet wird.

**8.** Verbindung der Formel (X)

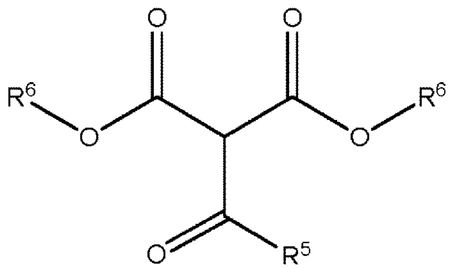

(X)

wobei $R^5$ entweder für eine lineare oder verzweigte vollständig gesättigte $C_{6-25}$-Alkylgruppe oder für eine lineare oder verzweigte $C_{6-25}$-Alkylgruppe mit mindestens einer Kohlenstoff-Kohlenstoff-Doppelbindung steht; und $R^6$ für eine Methyl- oder Ethyl- oder *tert.*-Butylgruppe steht.

**9.** Verbindung der Formel (XI)

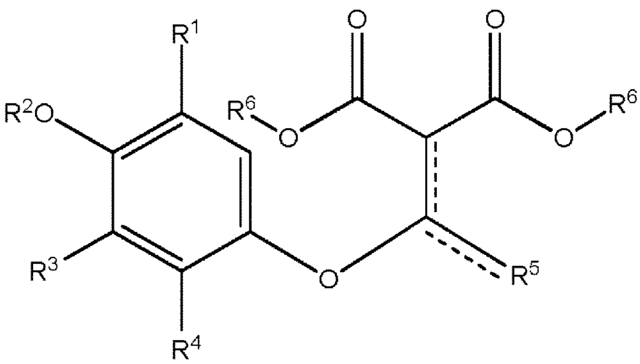

(XI)

wobei $R^5$ entweder für eine lineare oder verzweigte vollständig gesättigte $C_{6-25}$-Alkylgruppe oder für eine lineare oder verzweigte $C_{6-25}$-Alkylgruppe mit mindestens einer Kohlenstoff-Kohlenstoff-Doppelbindung steht;

$R^1$, $R^3$ und $R^4$ unabhängig voneinander für Wasserstoff oder Methylgruppen stehen;
$R^2$ für Wasserstoff oder eine Phenol-Schutzgruppe steht;
und $R^6$ für eine Methyl- oder Ethyl- oder *tert.*-Butylgruppe steht;
und die gestrichelten Linien die beiden alternativen Positionen einer Kohlenstoff-Kohlenstoff-Doppelbindung anzeigen.

**10.** Verbindung der Formel (XII)

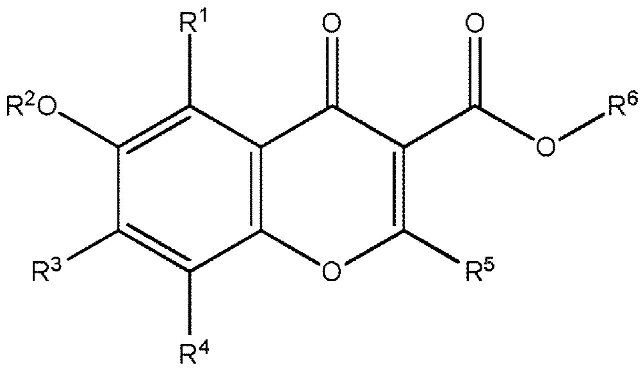

(XII)

wobei $R^5$ entweder für eine lineare oder verzweigte vollständig gesättigte $C_{6-25}$-Alkylgruppe oder für eine lineare oder verzweigte $C_{6-25}$-Alkylgruppe mit mindestens einer Kohlenstoff-Kohlenstoff-Doppelbindung steht;
$R^1$, $R^3$ und $R^4$ unabhängig voneinander für Wasserstoff oder Methylgruppen stehen;
$R^2$ und $R^{2'}$ für Wasserstoff oder eine Phenol-Schutzgruppe stehen;
und $R^6$ für eine Methyl- oder Ethyl- oder *tert.*-Butylgruppe steht.

**11.** Verbindung der Formel (X) oder (XI) oder (XII) gemäß Anspruch 8 bzw. 9 bzw. 10, **dadurch gekennzeichnet, dass** $R^5$ die Formel (III)

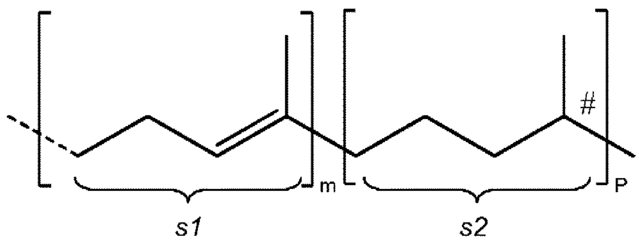

(III)

aufweist, wobei m und p unabhängig voneinander für einen Wert von 0 bis 5 stehen, mit der Maßgabe, dass die Summe von m und p gleich 1 bis 5 ist,
und wobei die durch *s1* und *s2* dargestellten Unterstrukturen in Formel (III) in beliebiger Reihenfolge vorliegen können;
und die gestrichelte Linie für die Bindung steht, über die der Substituent der Formel (III) an den Rest der Formel (X) bzw. (XI) bzw. (XII) gebunden ist;
und wobei # für ein Chiralitätszentrum steht, offensichtlich mit Ausnahme des Falls, in dem das Zentrum mit zwei Methylgruppen verknüpft ist.

**12.** Verfahren zur Herstellung einer Verbindung der Formel (X)

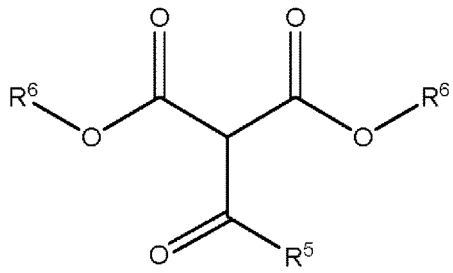

(X)

wobei $R^5$ entweder für eine lineare oder verzweigte vollständig gesättigte $C_{6-25}$-Alkylgruppe oder für eine lineare oder verzweigte $C_{6-25}$-Alkylgruppe mit mindestens einer Kohlenstoff-Kohlenstoff-Doppelbindung steht;
und $R^6$ für eine Methyl- oder Ethyl- oder *tert.*-Butylgruppe steht;
umfassend den Schritt des Umsetzens einer Verbindung der Formel (X-a) mit einer Verbindung der Formel (X-b)

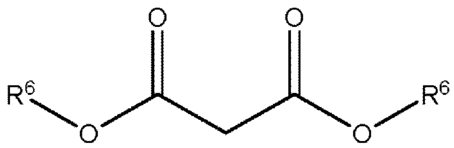

(X-a)

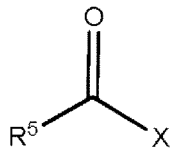

(X-b)

wobei X = Cl oder Br oder

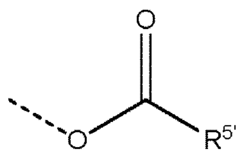

;

wobei $R^{5'}$ entweder für eine lineare oder verzweigte vollständig gesättigte $C_{1-25}$-Alkylgruppe oder für eine lineare oder verzweigte $C_{2-25}$-Alkylgruppe mit mindestens einer Kohlenstoff-Kohlenstoff-Doppelbindung steht; insbesondere $R^{5'}$;
in Gegenwart von Alkalimetallhydrid in einem Ether, insbesondere in Tetrahydrofuran.

**13.** Verfahren zur Herstellung einer Verbindung der Formel (X)

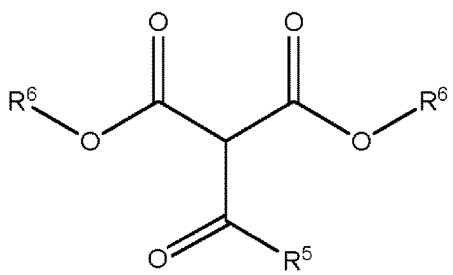

(X)

wobei $R^5$ entweder für eine lineare oder verzweigte vollständig gesättigte $C_{6-25}$-Alkylgruppe oder für eine lineare oder verzweigte $C_{6-25}$-Alkylgruppe mit mindestens einer Kohlenstoff-Kohlenstoff-Doppelbindung steht;
und $R^6$ für eine Methyl- oder Ethyl- oder *tert.*-Butylgruppe steht;

das folgende Schritte umfasst:

i) Umsetzen einer Verbindung der Formel (X-a') mit einer Verbindung der Formel (X-b')

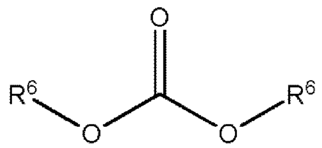

(X-a')

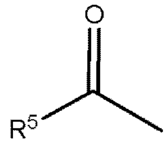

(X-b')

in Gegenwart von Alkalimetallhydrid in einem Ether, insbesondere in Tetrahydrofuran;
ii) Umsetzen mit einer Verbindung der Formel (X-c')

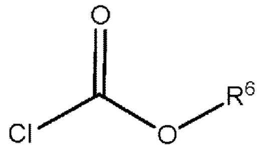

(X-c')

in Gegenwart einer Base, insbesondere Pyridin, und eines Metallsalzes.

**14.** Verfahren zur Herstellung einer Verbindung der Formel (XI)

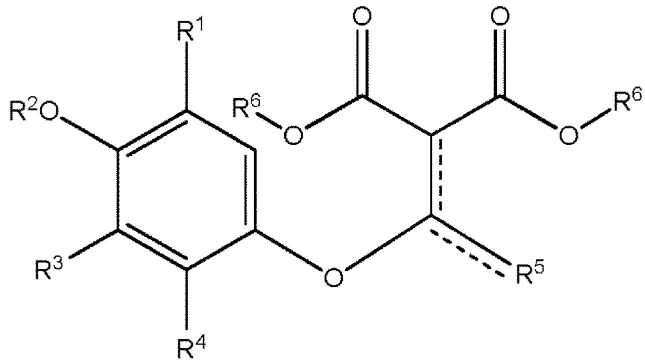

(XI)

wobei $R^5$ entweder für eine lineare oder verzweigte vollständig gesättigte $C_{6-25}$-Alkylgruppe oder für eine lineare oder verzweigte $C_{6-25}$-Alkylgruppe mit mindestens einer Kohlenstoff-Kohlenstoff-Doppelbindung steht;
$R^1$, $R^3$ und $R^4$ unabhängig voneinander für Wasserstoff oder Methylgruppen stehen;
$R^2$ für Wasserstoff oder eine Phenol-Schutzgruppe steht;
und $R^6$ für eine Methyl- oder Ethyl- oder *tert.*-Butylgruppe steht;
und die gestrichelten Linien die beiden alternativen Positionen einer Kohlenstoff-Kohlenstoff-Doppelbindung anzeigen;

das folgende Schritte umfasst:

- Umsetzen einer Verbindung der Formel (X) mit einem Sulfonylhalogenid oder Anhydrid einer organischen Sulfonsäure in Gegenwart einer Base, insbesondere in Gegenwart eines tertiären Amins; zu einem Enolsulfonat-Zwischenprodukt;
- Umsetzen des Enolsulfonat-Zwischenprodukts mit einer Verbindung der Formel (XI-a) in Gegenwart einer Base, insbesondere in Gegenwart eines tertiären Amins

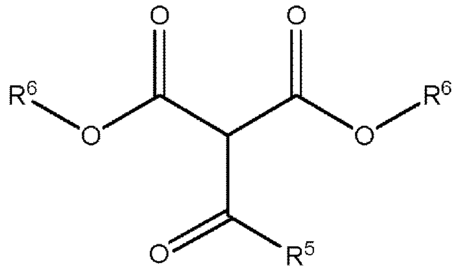

(X)

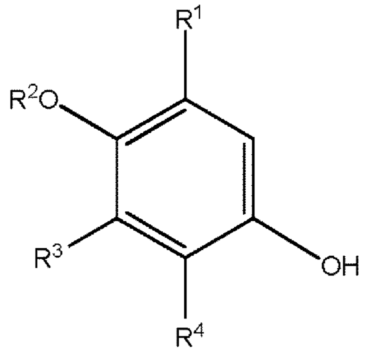

(XI-a).

**15.** Verfahren zur Herstellung einer Verbindung der Formel (XII)

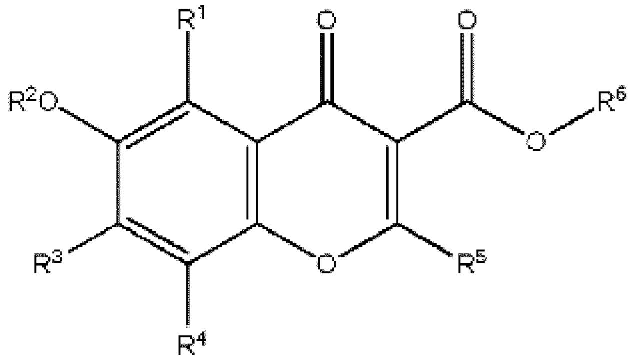

(XII)

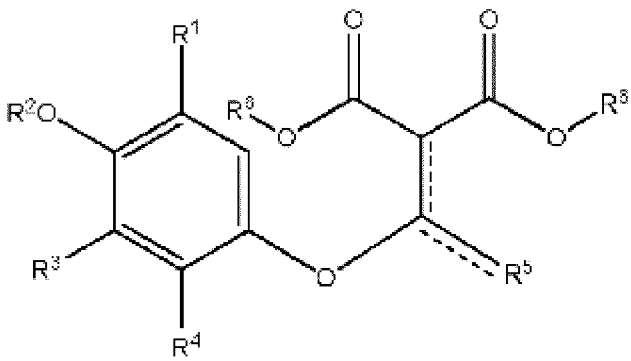

(XI)

wobei $R^5$ entweder für eine lineare oder verzweigte vollständig gesättigte $C_{6-25}$-Alkylgruppe oder für eine lineare oder verzweigte $C_{6-25}$-Alkylgruppe mit mindestens einer Kohlenstoff-Kohlenstoff-Doppelbindung steht;
$R^1$, $R^3$ und $R^4$ unabhängig voneinander für Wasserstoff oder Methylgruppen stehen;
$R^2$ für Wasserstoff oder eine Phenol-Schutzgruppe steht;
und $R^6$ für eine Methyl- oder Ethyl- oder *tert.*-Butylgruppe steht;
und die gestrichelten Linien die beiden alternativen Positionen einer Kohlenstoff-Kohlenstoff-Doppelbindung anzeigen;

umfassend eine intramolekulare Reaktion von Formel (XI) in Gegenwart eines methanolbindenden Mittels in Methansulfonsäure.

**16.** Verfahren zur Herstellung einer Verbindung der Formel (XXI-A) oder (XXI-B)

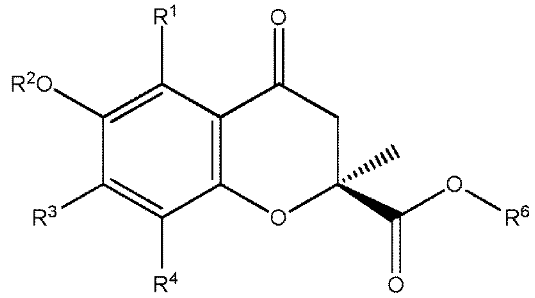

(XXI-A)

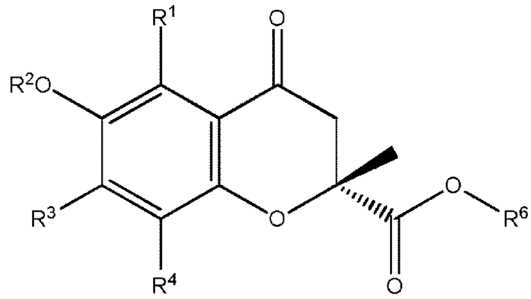

(XXI-B)

umfassend den Schritt des Umsetzens einer Verbindung der Formel (XXII)

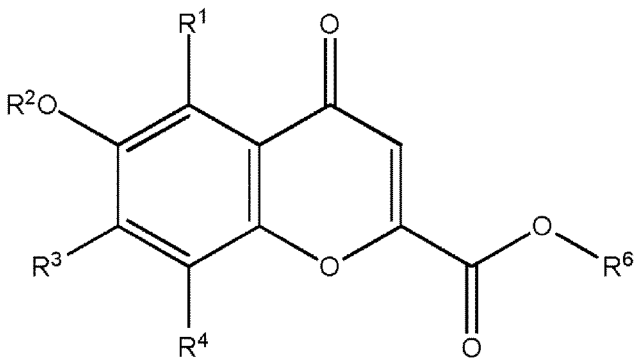

(XXII)

mit $Al(CH_3)_3$ in Gegenwart eines Cu(I)-Salzes und einer chiralen Verbindung der Formel (II-A) oder (II-B)

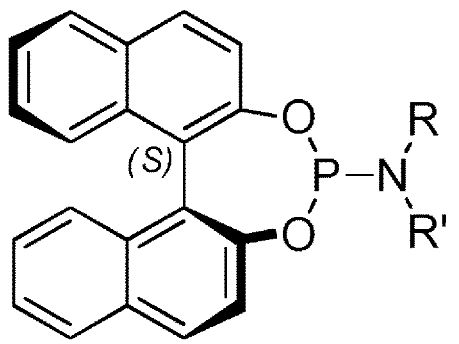

(II-A)

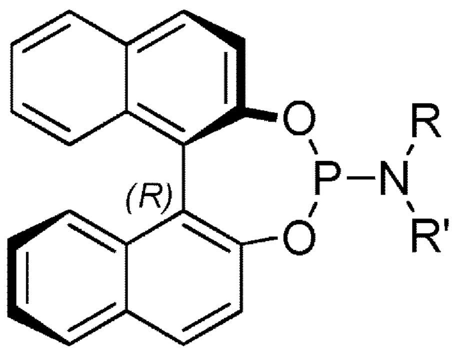

(II-B)

wobei $R^1$, $R^3$ und $R^4$ unabhängig voneinander für Wasserstoff oder Methylgruppen stehen;
$R^2$ für Wasserstoff oder eine Phenol-Schutzgruppe steht;
R' und R unabhängig voneinander für eine Hydrocarbylgruppe mit 1 bis 12 Kohlenstoffatomen, die gegebenenfalls durch $OR^6$ substituiert ist, stehen oder zusammen eine $C_{4-6}$-Alkylengruppe, die gegebenenfalls eine funktionelle Gruppe, die aus der Gruppe bestehend aus

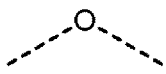, 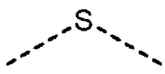, 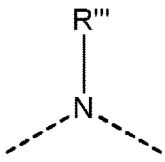 und 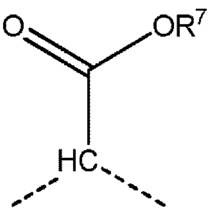

ausgewählt ist, bilden, wobei R''' und $R^7$ unabhängig voneinander für eine $C_{1-4}$-Alkylgruppe stehen und $R^6$ für eine Methyl- oder Ethylgruppe oder *tert.*-Butylgruppe steht;
mit der Maßgabe, dass für die Herstellung von Verbindung (XXI-A) die Verbindung (II-A) verwendet wird und dass für die Herstellung von Verbindung (XXI-B) die Verbindung (II-B) verwendet wird.

## Revendications

1. Procédé pour préparer un composé de formule (I-A) ou (I-B)

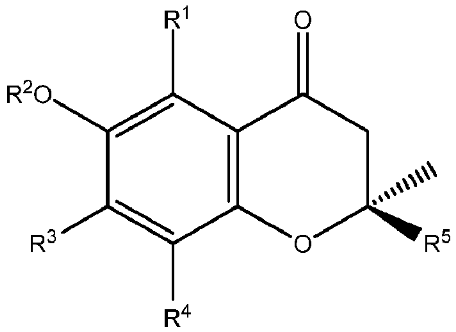

(I-A)

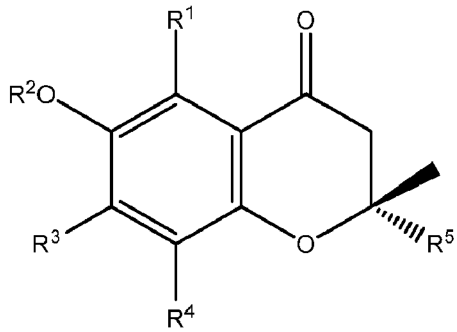

(I-B)

comprenant l'étape consistant à faire réagir un composé de formule (XII)

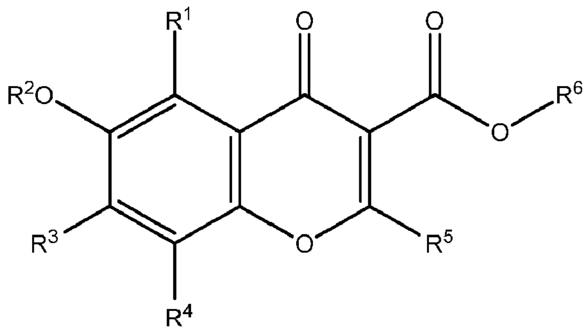

(XII)

avec $Al(CH_3)_3$ en présence d'un sel de Cu(I) et d'un composé chiral de formule (II-A) ou (II-B)

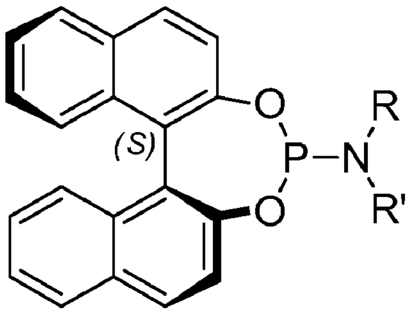

(II-A)

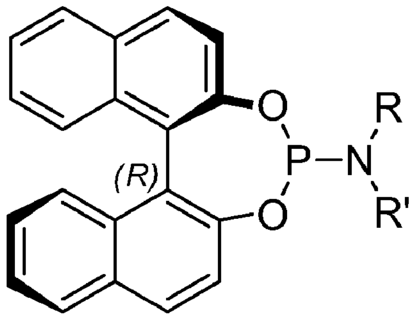

(II-B)

opération suivie d'une hydrolyse d'ester et d'une décarboxylation par chauffage à une température de 100 à 190°C, en particulier de 150 à 190°C,
formules dans lesquelles

$R^5$ représente soit un groupe alkyle en $C_6$ à $C_{25}$ complètement saturé, linéaire ou ramifié, soit un groupe alkyle en $C_6$ à $C_{25}$ linéaire ou ramifié comprenant au moins une double liaison carbone-carbone ;
$R^1$, $R^3$ et $R^4$, indépendamment les uns des autres, sont des atomes d'hydrogène ou des groupes méthyle ;
$R^2$ représente l'hydrogène ou un groupe protecteur de phénol ;
R' et R, indépendamment l'un de l'autre, sont des groupes hydrocarbyle ayant 1 à 12 atomes de carbone qui sont éventuellement substitués par $OR^6$, ou bien forment ensemble un groupe alkylène en $C_4$ à $C_6$ qui comprend éventuellement un groupe fonctionnel choisi dans l'ensemble constitué par

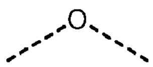 , 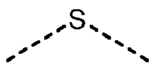 , 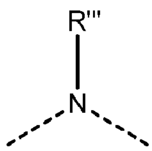 et 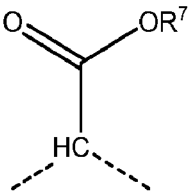

où R''' et $R^7$, indépendamment l'un de l'autre, sont des groupes alkyle en $C_1$ à $C_4$ ;
et $R^6$ est un groupe méthyle ou éthyle ou un groupe tert-butyle ;
sous réserve que le composé (II-A) soit utilisé pour la préparation du composé (I-A) et que le composé (II-B) soit utilisé pour la préparation du composé (I-B).

2. Procédé selon la revendication 1, **caractérisé en ce que** le sel de Cu(I) est un carboxylate de Cu(I) ou un sulfonate de Cu(I), en particulier le thiophène-2-carboxylate de Cu(I) ou le triflate de Cu(I).

3. Procédé selon la revendication 1 ou 2, **caractérisé en ce que** R' et/ou R représentent un groupe alkyle en $C_1$ à $C_6$ ou un groupe aryle ou aralkyle, en particulier un groupe benzyle ou un groupe méthyle ou un groupe 1-phényléthyle ou un groupe 4-méthoxybenzyle ou 1-naphtylméthyle ou 2-naphtylméthyle.

4. Procédé selon l'une quelconque des revendications 1 à 3, **caractérisé en ce que** R = R', en particulier R = R' = un groupe méthyle.

5. Procédé selon l'une quelconque des revendications 1 à 4, **caractérisé en ce que** $R^5$ est de formule (III)

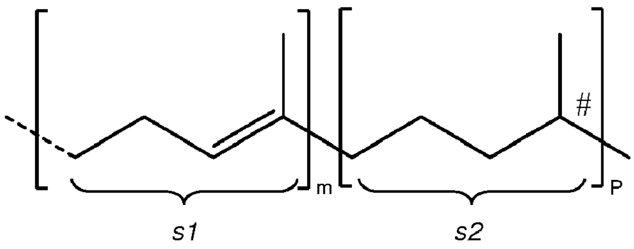

(III)

dans laquelle

m et p, indépendamment l'un de l'autre, valent de 0 à 5, sous réserve que la somme de m et p soit de 1 à 5, et les sous-structures dans la formule (III) représentées par *s1* et *s2* peuvent être dans n'importe quel ordre ;
et la ligne de tirets représente la liaison par laquelle le substituant de formule (III) est lié au reste de la formule (I) ;
et # représente un centre chiral, bien évidemment sauf dans le cas où ledit centre est lié à deux groupes méthyle.

**6.** Procédé pour préparer un composé de formule (IV-A) ou (IV-B)

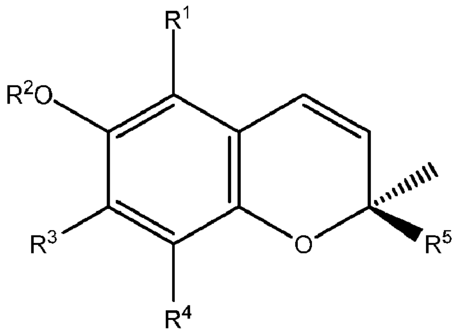

(IV-A)

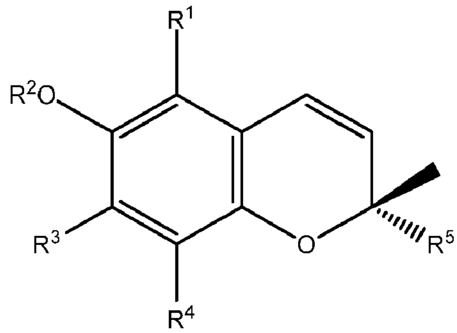

(IV-B)

comprenant les étapes suivantes :

a) préparation d'un composé de formule (I-A) ou (I-B) conformément à un procédé selon l'une quelconque des revendications 1 à 5 ;
b) réduction du composé de formule (I-A) ou (I-B) avec un hydrure, en particulier avec du borohydrure de sodium ;
c) traitement avec un acide ;

sous réserve que le composé de formule (IV-A) soit formé quand le composé de formule (I-A) est utilisé dans l'étape a) et que le composé de formule (IV-B) soit formé quand le composé de formule (I-B) est utilisé dans l'étape a).

**7.** Procédé pour préparer un composé de formule (V-A) ou (V-B)

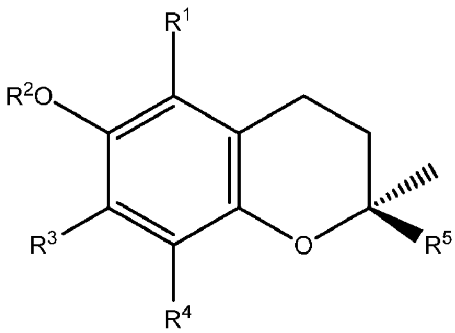

(V-A)

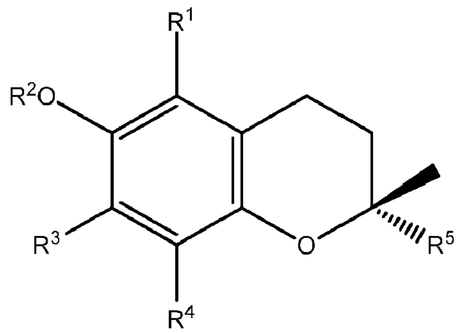

(V-B)

comprenant les étapes suivantes :

a) préparation d'un composé de formule (I-A) ou (I-B) conformément à un procédé selon l'une quelconque des revendications 1 à 5 ;
b) hydrogénation du composé de formule (I-A) ou (I-B), en particulier au moyen d'hydrogène moléculaire en présence d'un catalyseur au Pd/C ou avec du zinc métallique en présence d'un acide ;

sous réserve que le composé de formule (V-A) soit formé quand le composé de formule (I-A) est utilisé dans l'étape a) et que le composé de formule (V-B) soit formé quand le composé de formule (I-B) est utilisé dans l'étape a).

**8.** Composé de formule (X)

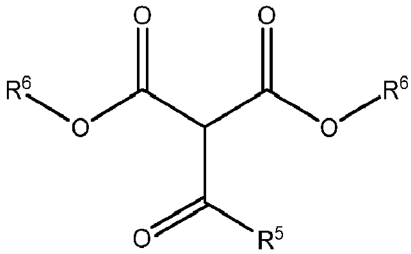

(X)

dans laquelle $R^5$ représente soit un groupe alkyle en $C_6$ à $C_{25}$ complètement saturé, linéaire ou ramifié, soit un groupe alkyle en $C_6$ à $C_{25}$ linéaire ou ramifié comprenant au moins une double liaison carbone-carbone ;
et $R^6$ est un groupe méthyle ou éthyle ou tert-butyle.

**9.** Composé de formule (XI)

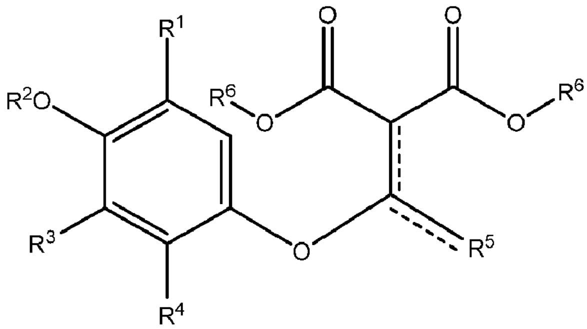

(XI)

dans laquelle $R^5$ représente soit un groupe alkyle en $C_6$ à $C_{25}$ complètement saturé, linéaire ou ramifié, soit un groupe alkyle en $C_6$ à $C_{25}$ linéaire ou ramifié comprenant au moins une double liaison carbone-carbone ;
$R^1$, $R^3$ et $R^4$, indépendamment les uns des autres, sont des atomes d'hydrogène ou des groupes méthyle ;
$R^2$ représente l'hydrogène ou un groupe protecteur de phénol ;
et $R^6$ est un groupe méthyle ou éthyle ou tert-butyle ;

et les lignes de tirets représentent les deux positions alternatives d'une double liaison carbone-carbone.

**10.** Composé de formule (XII)

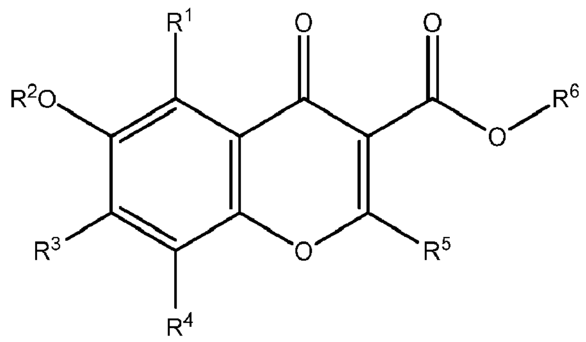

(XII)

dans laquelle $R^5$ représente soit un groupe alkyle en $C_6$ à $C_{25}$ complètement saturé, linéaire ou ramifié, soit un groupe alkyle en $C_6$ à $C_{25}$ linéaire ou ramifié comprenant au moins une double liaison carbone-carbone ;
$R^1$, $R^3$ et $R^4$, indépendamment les uns des autres, sont des atomes d'hydrogène ou des groupes méthyle ;
$R^2$ et $R^{2'}$ représentent l'hydrogène ou un groupe protecteur de phénol ;
et $R^6$ est un groupe méthyle ou éthyle ou tert-butyle.

**11.** Composé de formule (X) ou (XI) ou (XII) selon la revendication 8 ou 9 ou 10, **caractérisé en ce que** $R^5$ est de formule (III)

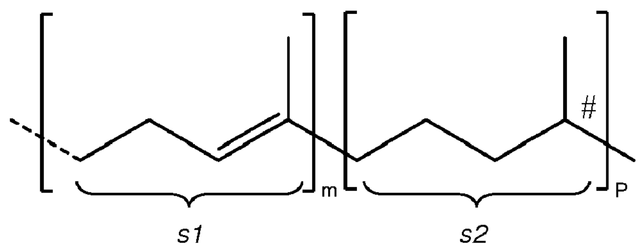

(III)

dans laquelle

m et p, indépendamment l'un de l'autre, valent de 0 à 5, sous réserve que la somme de m et p soit de 1 à 5,
et les sous-structures dans la formule (III) représentées par *s1* et *s2* peuvent être dans n'importe quel ordre ;
et la ligne de tirets représente la liaison par laquelle le substituant de formule (III) est lié au reste de la formule (X), (XI) ou (XII) ;
et # représente un centre chiral, bien évidemment sauf dans le cas où ledit centre est lié à deux groupes méthyle.

**12.** Procédé pour préparer un composé de formule (X)

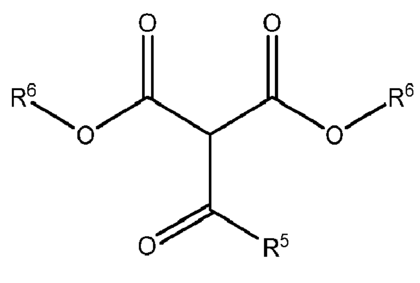

(X)

dans laquelle $R^5$ représente soit un groupe alkyle en $C_6$ à $C_{25}$ complètement saturé, linéaire ou ramifié, soit un groupe alkyle en $C_6$ à $C_{25}$ linéaire ou ramifié comprenant au moins une double liaison carbone-carbone ;
et $R^6$ est un groupe méthyle ou éthyle ou tert-butyle ; comprenant l'étape consistant à faire réagir un composé de formule (X-a) avec un composé de formule (X-b)

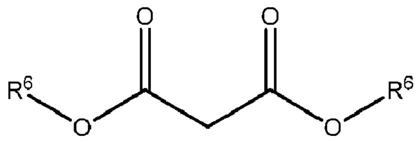

(X-a)

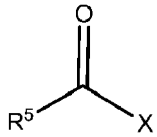

(X-b)

où X = Cl ou Br ou

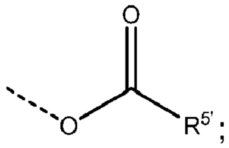

où $R^{5'}$ représente soit un groupe alkyle en $C_1$ à $C_{25}$ complètement saturé, linéaire ou ramifié, soit un groupe alkyle en $C_2$ à $C_{25}$ linéaire ou ramifié comprenant au moins une double liaison carbone-carbone ; en particulier $R^{5'}$;
en présence d'un hydrure de métal alcalin dans un éther, en particulier dans du tétrahydrofurane.

**13.** Procédé pour préparer un composé de formule (X)

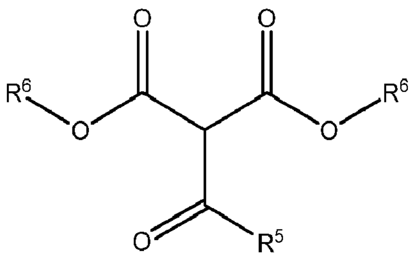

(X)

dans laquelle $R^5$ représente soit un groupe alkyle en $C_6$ à $C_{25}$ complètement saturé, linéaire ou ramifié, soit un groupe alkyle en $C_6$ à $C_{25}$ linéaire ou ramifié comprenant au moins une double liaison carbone-carbone ;
et $R^6$ est un groupe méthyle ou éthyle ou tert-butyle ; comprenant les étapes consistant à

i) faire réagir un composé de formule (X-a') avec un composé de formule (X-b')

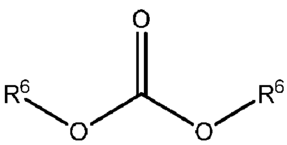

(X-a')

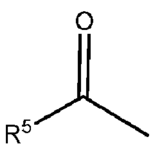

(X-b')

en présence d'un hydrure de métal alcalin dans un éther, en particulier dans du tétrahydrofurane ;
ii) faire réagir avec un composé de formule (X-c')

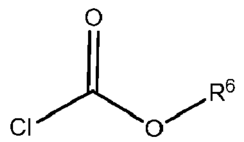

(X-c')

en présence d'une base, en particulier la pyridine, et d'un sel métallique.

**14.** Procédé pour préparer un composé de formule (XI)

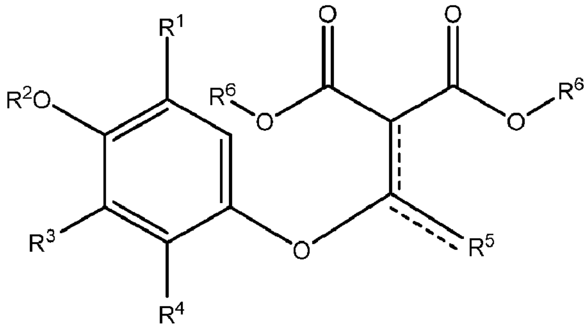

(XI)

dans laquelle $R^5$ représente soit un groupe alkyle en $C_6$ à $C_{25}$ complètement saturé, linéaire ou ramifié, soit un groupe alkyle en $C_6$ à $C_{25}$ linéaire ou ramifié comprenant au moins une double liaison carbone-carbone ;
$R^1$, $R^3$ et $R^4$, indépendamment les uns des autres, sont des atomes d'hydrogène ou des groupes méthyle ;
$R^2$ représente l'hydrogène ou un groupe protecteur de phénol ;
et $R^6$ est un groupe méthyle ou éthyle ou tert-butyle ; et les lignes de tirets représentent les deux positions alternatives d'une double liaison carbone-carbone ; comprenant les étapes consistant à

- faire réagir un composé de formule (X) avec un halogénure de sulfonyle ou un anhydride d'un acide sulfonique organique et en présence d'une base, en particulier en présence d'une amine tertiaire ; pour former un intermédiaire énolsulfonate ;
- faire réagir ledit intermédiaire énolsulfonate avec un composé de formule (XI-a) en présence d'une base, en particulier en présence d'une amine tertiaire

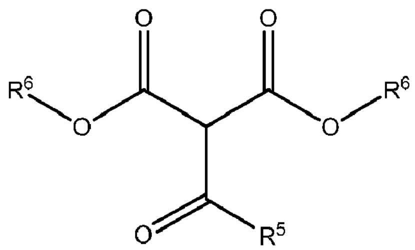

(X)

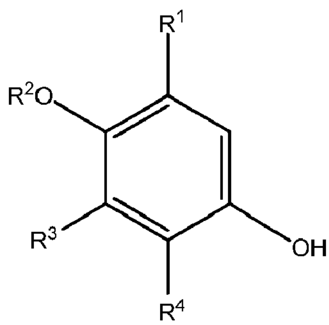

(XI-a).

**15.** Procédé pour préparer un composé de formule (XII)

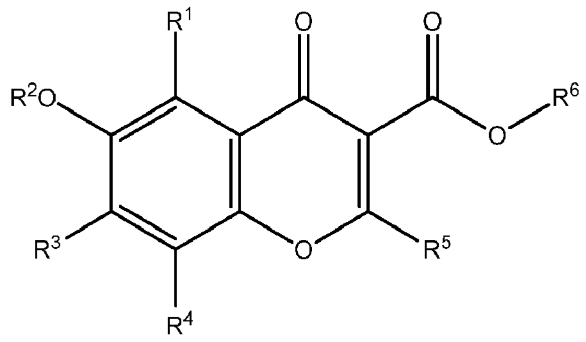

(XII)

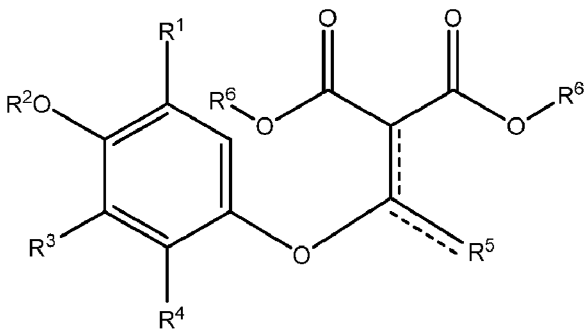

(XI)

où $R^5$ représente soit un groupe alkyle en $C_6$ à $C_{25}$ complètement saturé, linéaire ou ramifié, soit un groupe alkyle en $C_6$ à $C_{25}$ linéaire ou ramifié comprenant au moins une double liaison carbone-carbone ;
$R^1$, $R^3$ et $R^4$, indépendamment les uns des autres, sont des atomes d'hydrogène ou des groupes méthyle ;
$R^2$ représente l'hydrogène ou un groupe protecteur de phénol ;
et $R^6$ est un groupe méthyle ou éthyle ou tert-butyle ; et les lignes de tirets représentent les deux positions alternatives d'une double liaison carbone-carbone ; comprenant une réaction intramoléculaire d'un composé de formule (XI) en présence d'un agent de liaison méthanolique dans de l'acide méthanesulfonique.

**16.** Procédé pour préparer un composé de formule (XXI-A) ou (XXI-B)

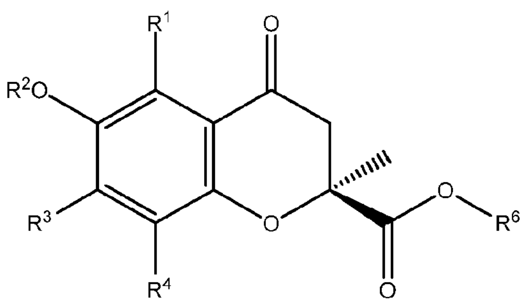

(XXI-A)

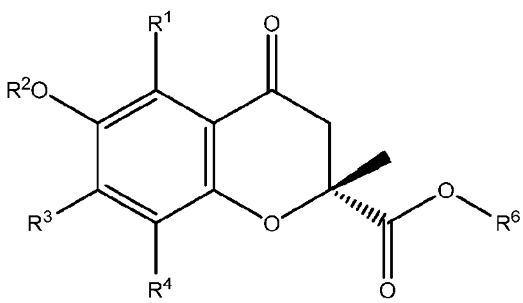

(XXI-B)

comprenant l'étape consistant à faire réagir un composé de formule (XXII)

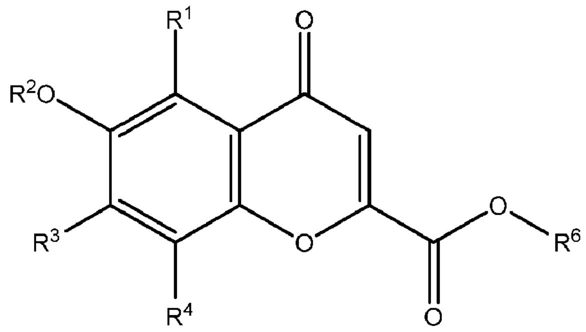

(XXII)

avec de l'$Al(CH_3)_3$ en présence d'un sel de Cu(I) et d'un composé chiral de formule (II-A) ou (II-B)

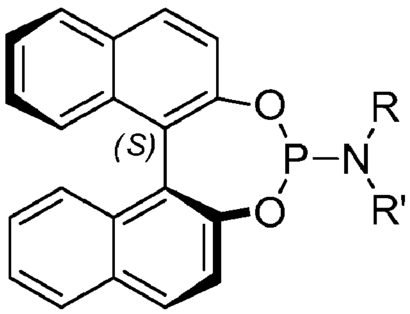

(II-A)

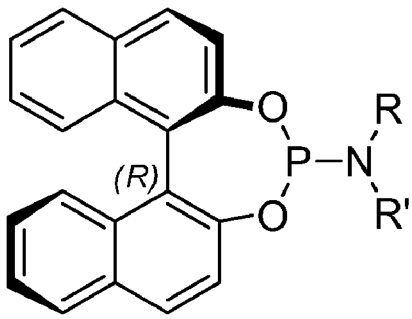

(II-B)

$R^1$, $R^3$ et $R^4$, indépendamment les uns des autres, sont des atomes d'hydrogène ou des groupes méthyle ;
$R^2$ représente l'hydrogène ou un groupe protecteur de phénol ;
R' et R, indépendamment l'un de l'autre, sont des groupes hydrocarbyle ayant 1 à 12 atomes de carbone qui sont éventuellement substitués par $OR^6$, ou bien forment ensemble un groupe alkylène en $C_4$ à $C_6$ qui comprend éventuellement un groupe fonctionnel choisi dans l'ensemble constitué par

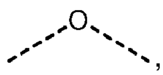 , 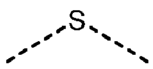 , 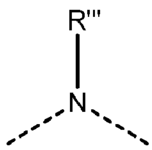 et 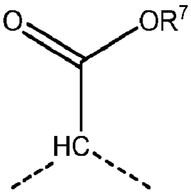

où R''' et $R^7$, indépendamment l'un de l'autre, sont des groupes alkyle en $C_1$ à $C_4$ ;
et $R^6$ est un groupe méthyle ou éthyle ou un groupe tert-butyle ;

sous réserve que le composé (II-A) soit utilisé pour la préparation du composé (XXI-A) et que le composé (II-B) soit utilisé pour la préparation du composé (XXI-B).

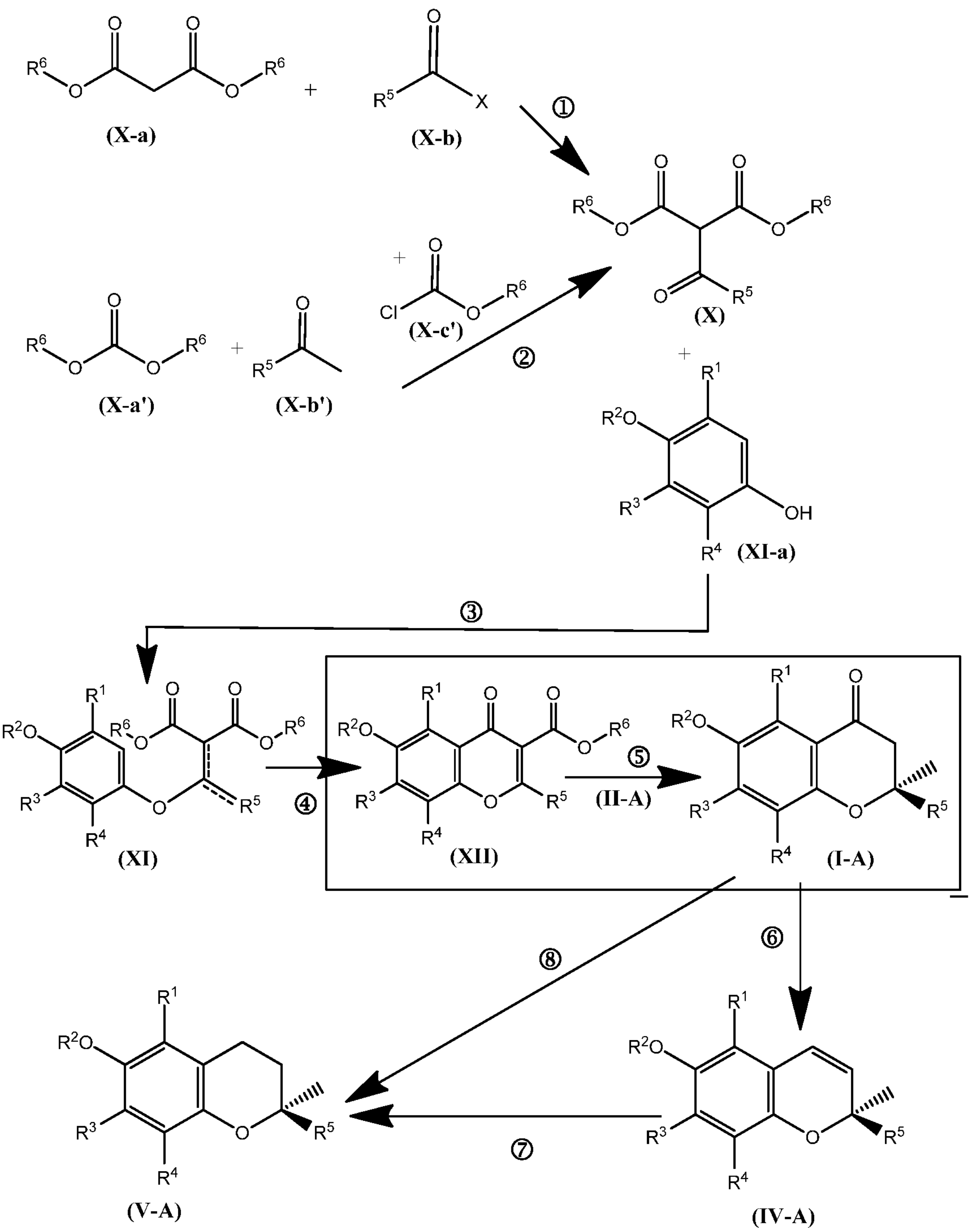

Figure 1

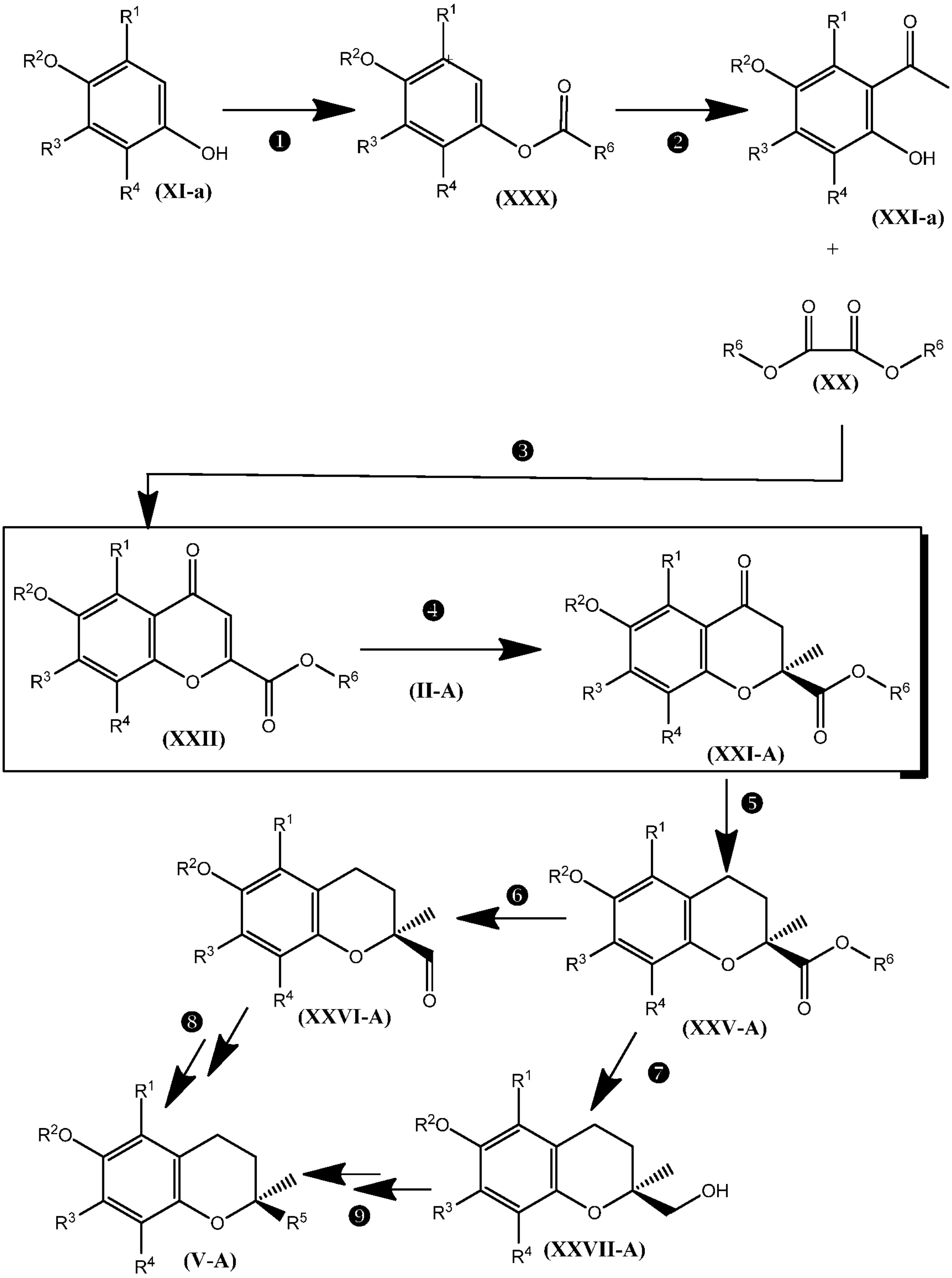


(XI-a) (XXX) (XXI-a) + (XX) (XXII) (XXI-A) (XXV-A) (XXVI-A) (XXVII-A) (V-A)

# Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6096907 A
- WO 2012152779 A1
- WO 2012171969 A1


### Non-patent literature cited in the description


- *Pure & Appl.Chem.,* 1982, vol. 54 (8), 1507-1510
- **H. WEISER et al.** *J. Nutr.,* 1996, vol. 126 (10), 2539-49
- **KABBE ; HEITZER.** *Synthesis,* 1978, 888-889
- **KABBE ; WIDDIG.** *Angew. Chem. Int. Ed. Engl.,* 1982, vol. 21, 247-256
- **D. WOGGON et al.** *Chembiochem,* 2011, vol. 12, 118-124
- *J. Org. Chem.,* 2005, vol. 70, 943-951
- **P. KRAPCHO et al.** *J. Org. Chem.,* 1978, vol. 43, 138-147
- **BRADLEY et al.** *J. Chem. Res., Miniprint,* 1993, 210
- **SCHUDEL et al.** *Helv. Chim. Acta,* 1963, vol. 46, 2517-2526
- **J. CHAPELAT ; A. BUSS ; A. CHOUGNET ; W.-D. WOGGON.** *Org. Lett.,* 2008, vol. 10, 5123-5126